# EUROPEAN PATENT SPECIFICATION

(11) **EP 4 021 537 B1**
(45) Date of publication and mention of the grant of the patent: **04.11.2026**
(21) Application number: 20817513.3
(22) Date of filing: 18.11.2020
(51) Int. Cl.: A61M 5/20, A61M 5/24

(54) **EMERGENCY AUTOMATIC INJECTION DEVICE**
AUTOMATISCHES NOTFALL INJEKTIONSGERÄT
DISPOSITIF D'INJECTION AUTOMATIC POUR CAS D'URGENCE

(30) Priority: 19.11.2019 US 201962937264 P
(43) Date of publication of application: 06.07.2022
(60) Divisional application: 26204370.6
(73) Proprietor: E3D A.C.A.L, 1386000 Merom Hagalil (IL)
(72) Inventor: CARMEL, Ehoud, 5647702 Yehud-Monosson (IL); SHAKED, Tsachi, 1386000 Merom Hagalil (IL); SARKOROV, Dmitri, 5647702 Rosh HaAyin (IL); RADAY, Lior, 7915200 MP Hof Ashkelon (IL); DAILY, David, 4636431 Herzlyia (IL)
(74) Representative: Evens, Paul Jonathan
(86) International application number: PCT/IL2020/051192
(87) International publication number: WO 2021/100039

(56) References cited:
- WO-A1-2017/198383
- GB-A- 2 560 558
- US-A1- 2015 273 160
- US-B2- 10 137 255
- US-B2- 9 022 982

## Description

### REFERENCE TO RELATED APPLICATIONS

Reference is hereby made to U.S. Provisional Patent Application Serial No. 62/937,264, filed November 19, 2019 and entitled "EMERGENCY AUTOMATIC INJECTION DEVICE".

Reference is also hereby made to U.S. Patent No. US8376998, issued February 19, 2013 and entitled "Automatic Injection Device" and to U.S. Patent No. US8708968, issued April 29, 2014 and entitled "Removal of needle shields from syringes and automatic injection devices'.

### FIELD OF THE INVENTION

The present invention generally relates to an auto injector, and more specifically to an auto-injector adapted for parenteral administration of substances (e.g., a medication) to a living organism (human or animal) by means of pressing the auto injector against an injection site.

### BACKGROUND OF THE INVENTION

Various emergency automatic injectors are known, such that are activatable by means of pressing the automatic injector against an injection site on the skin of a patient. It is important to ensure the needle is protected at all times before, during and after injection of the medicament. It is also required to ensure that the automatic injector is not inadvertently actuated.

### SUMMARY OF THE INVENTION

The present invention seeks to provide an emergency automatic injection device. The invention is defined in claim 1, with further embodiments defined in the dependent claims.

There is thus provided in accordance with an embodiment of the present invention an automatic injection device for use with a syringe including at least one syringe piston and a needle coupled to a forward end thereof, comprising: a housing element arranged along a longitudinal axis and having a forward end and a rearward end; at least one resilient element arranged to be located within the housing element; a needle shield selectably positionable with respect to the housing element; and a control unit adapted, when actuated, to be driven by the at least one resilient element for initially displacing the syringe relative to the housing element from a non-penetration position to a penetration position and thereafter displacing the at least one syringe piston in the syringe to effect drug delivery, and wherein said control unit is configured to be actuated upon axial rearward displacement of the needle shield with respect to the housing element.

Preferably, the automatic injection device also comprising a locking element operative for selectable displacement relative to the housing element and being operatively engageable with the needle shield and wherein upon axial rearward displacement of the needle shield with respect to the housing element, the locking element is permitted to rotate about the longitudinal axis under the urge of the at least one resilient element. Further preferably, the locking element is selectably operatively engaged with the control unit and wherein the control unit is operative for causing rotation of the locking element under the urge of the at least one resilient element upon axial rearward displacement of the needle shield with respect to the housing element.

Still further preferably, the automatic injection device also comprising a plunger rod, operative to selectably drive the at least one syringe piston in axial motion relative to the housing element; the plunger rod is operative to be displaced together with the control unit from the actuation of the control unit up to the penetration position of the syringe.

Yet further preferably, the at least one resilient element includes a single spring. Alternatively, the at least one resilient element includes a first spring and a second spring. Preferably, the second spring is at least partially disposed within the plunger rod and is operative for biasing the plunger rod to be displaced forwardly along the longitudinal axis.

In accordance with an embodiment of the present invention, the automatic injection device also comprising a needle shield remover or needle cover remover configured to be removably attached to the housing element, operative for protecting the needle, and wherein the needle shield is prevented from axial rearward displacement with respect to the housing element when the needle shield remover is attached to the housing element.

Preferably, the needle cover remover includes at least one fixating element, operative to engage a corresponding fixating counter-element formed on the needle shield to prevent inadvertent rearward displacement of the needle shield with respect to the housing element.

Further preferably, the locking element is permitted to be rotated in a single rotational direction. Still further preferably, the locking element is selectably positioned in one of a locked orientation and an unlocked orientation relative to the control unit; and wherein when the locking element is positioned in the unlocked orientation, the at least one resilient element is permitted to drive the control unit axially forwardly relative to the housing element.

Yet further preferably, the locking element has a rotation enabling element and the control unit has a counter rotation enabling element, which engages the rotation enabling element when the locking element is disposed in the locked orientation.

In accordance with an embodiment of the present invention, the needle shield is prevented from axial forward longitudinal displacement relative to the housing when the locking element is disposed in the locked orientation. Preferably, the locking element has a protrusion formed on an outer surface thereof for assuring actuation of the control unit upon rearward displacement of the needle shield relative to the housing element. Further preferably, the automatic injection device also comprising a syringe sleeve, which is fixedly attached to or integrally made with the housing element and includes a dampening element, adapted to dampen the impact on the syringe upon forward displacement thereof and during penetration of the needle.

Still further preferably, in a post-injection operative state, the plunger rod is prevented from rearward axial displacement relative to the housing element. Yet further preferably, the automatic injection device also comprising a locking element, which is prevented from displacement relative to the housing element due to engagement with the needle shield in a prior to injection operative state and wherein the control unit is prevented from displacement relative to the housing element due to engagement with the locking element in the prior to injection operative state. Still further preferably, the needle shield comprises at least one stopping rib which engages a protrusion formed on the locking element for restricting rotation of the locking element in the prior to injection operative state.

In accordance with an embodiment of the present invention, an automatic injection device for use with a syringe including at least one syringe piston and a needle coupled to a forward end thereof, comprising a housing element arranged along a longitudinal axis and having a forward end and a rearward end; at least one resilient element arranged to be located within the housing element; a needle shield selectably positionable with respect to the housing element; a locking element operative for selectable displacement relative to the housing element and being operatively engageable with the needle shield; and a control unit adapted, when actuated, to be driven by the at least one resilient element for initially displacing the syringe relative to the housing element from a non-penetration position to a penetration position and thereafter displacing the at least one syringe piston in the syringe to effect drug delivery, and wherein when the locking element engages the needle shield, displacement of the locking element relative the housing is prevented, thereby preventing the at least one resilient element from driving the control unit.

Preferably, upon axial rearward displacement of the needle shield with respect to the housing element, the locking element is permitted to rotate about the longitudinal axis under the urge of the at least one resilient element. Further preferably, the locking element is selectably operatively engaged with the control unit and wherein the control unit is operative for causing rotation of the locking element under the urge of the at least one resilient element upon axial rearward displacement of the needle shield with respect to the housing element.

In accordance with an embodiment of the present invention, the automatic injection device also comprising a plunger rod, operative to selectably drive the at least one syringe piston in axial motion relative to the housing element; the plunger rod is operative to be displaced together with the control unit from the actuation of the control unit up to the penetration position of the syringe.

Preferably, the at least one resilient element includes a single spring. Alternatively, the at least one resilient element includes a first spring and a second spring. Preferably, the second spring is at least partially disposed within the plunger rod and is operative for biasing the plunger rod to be displaced forwardly along the longitudinal axis.

Preferably, the automatic injection device also comprising a needle cover remover configured to be removably attached to the housing element, operative for protecting the needle, and wherein the needle shield is prevented from axial rearward displacement with respect to the housing element when the needle shield remover is attached to the housing element. Further preferably, the needle cover remover includes at least one fixating element, operative to engage a corresponding fixating counter-element formed on the needle shield to prevent inadvertent rearward displacement of the needle shield with respect to the housing element.

Still further preferably, the locking element is permitted to be rotated in a single rotational direction. Yet further preferably, the locking element is selectably positioned in one of a locked orientation and an unlocked orientation relative to the control unit; and wherein when the locking element is positioned in the unlocked orientation, the at least one resilient element is permitted to drive the control unit axially forwardly relative to the housing element.

In accordance with an embodiment of the present invention the locking element has a rotation enabling element and the control unit has a counter rotation enabling element, which engages the rotation enabling element when the locking element is disposed in the locked orientation.

Preferably, the needle shield is prevented from axial forward longitudinal displacement relative to the housing when the locking element is disposed in the locked orientation. Further preferably, the locking element has a protrusion formed on an outer surface thereof for assuring actuation of the control unit upon rearward displacement of the needle shield relative to the housing element.

Still further preferably, the automatic injection device also comprising a syringe sleeve, which is fixedly attached to or integrally made with the housing element and includes a dampening element, adapted to dampen the impact on the syringe upon forward displacement thereof and during penetration of the needle.

In accordance with an embodiment of the present invention, in a post-injection operative state, the plunger rod is prevented from rearward axial displacement relative to said housing element.

Preferably, the locking element is prevented from displacement relative to the housing element due to engagement with the needle shield in a prior to injection operative state and wherein the control unit is prevented from displacement relative to the housing element due to engagement with the locking element in the prior to injection operative state. Further preferably, the needle shield comprises at least one stopping rib which engages a protrusion formed on the locking element for restricting rotation of the locking element in the prior to injection operative state.

In accordance with an embodiment of the present invention, an automatic injection device for use with a syringe including at least one syringe piston and a needle coupled to a forward end thereof, comprising a housing element arranged along a longitudinal axis and having a forward end and a rearward end; at least one resilient element arranged to be located within the housing element; a needle shield selectably positionable with respect to the housing element; a locking element operative for selectable displacement relative to the housing element and being operatively engageable with the needle shield; and a control unit adapted, when actuated, to be driven by the at least one resilient element for initially displacing the syringe relative to the housing element from a non-penetration position to a penetration position and thereafter displacing the at least one syringe piston in the syringe to effect drug delivery, and wherein the locking element is permitted to be displaced relative to the housing upon axial rearward displacement of the needle shield with respect to the housing element.

Preferably, the locking element is selectably operatively engaged with the control unit and wherein the control unit is operative for causing rotation of the locking element under the urge of the at least one resilient element upon axial rearward displacement of the needle shield with respect to the housing element.

Further preferably, the automatic injection device also comprising a plunger rod, operative to selectably drive the at least one syringe piston in axial motion relative to the housing element; the plunger rod is operative to be displaced together with the control unit from the actuation of the control unit up to the penetration position of the syringe.

Still further preferably, the at least one resilient element includes a single spring. Alternatively, the at least one resilient element includes a first spring and a second spring. Preferably, the second spring is at least partially disposed within the plunger rod and is operative for biasing the plunger rod to be displaced forwardly along the longitudinal axis.

In accordance with an embodiment of the present invention, the automatic injection device also comprising a needle cover remover configured to be removably attached to the housing element, operative for protecting the needle, and wherein the needle shield is prevented from axial rearward displacement with respect to the housing element when the needle shield remover is attached to the housing element.

Preferably, the needle cover remover includes at least one fixating element, operative to engage a corresponding fixating counter-element formed on the needle shield to prevent inadvertent rearward displacement of the needle shield with respect to the housing element. Further preferably, the locking element is permitted to be rotated in a single rotational direction. Still further preferably, the locking element is selectably positioned in one of a locked orientation and an unlocked orientation relative to the control unit; and wherein when the locking element is positioned in the unlocked orientation, the at least one resilient element is permitted to drive the control unit axially forwardly relative to the housing element.

In an embodiment of the present invention, the locking element has a rotation enabling element and the control unit has a counter rotation enabling element, which engages the rotation enabling element when the locking element is disposed in the locked orientation.

Preferably, the needle shield is prevented from axial forward longitudinal displacement relative to the housing when the locking element is disposed in the locked orientation. Further preferably, the locking element has a protrusion formed on an outer surface thereof for assuring actuation of the control unit upon rearward displacement of the needle shield relative to the housing element. Still further preferably, the automatic injection device also comprising a syringe sleeve, which is fixedly attached to or integrally made with the housing element and includes a dampening element, adapted to dampen the impact on the syringe upon forward displacement thereof and during penetration of the needle.

Preferably, in a post-injection operative state, the plunger rod is prevented from rearward axial displacement relative to the housing element. Further preferably, the locking element is prevented from displacement relative to the housing element due to engagement with the needle shield in a prior to injection operative state and wherein the control unit is prevented from displacement relative to the housing element due to engagement with the locking element in the prior to injection operative state. Still further preferably, the needle shield comprises at least one stopping rib which engages a protrusion formed on the locking element for restricting rotation of the locking element in the prior to injection operative state.

In accordance with an embodiment of the present invention, an automatic injection device for use with a syringe including at least one syringe piston and a needle coupled to a forward end thereof, comprising a housing element arranged along a longitudinal axis and having a forward end and a rearward end; at least one resilient element arranged to be located within the housing element; a control unit adapted, when actuated, to be driven by the at least one resilient element for initially displacing the syringe relative to the housing element from a non-penetration position to a penetration position and thereafter displacing the at least one syringe piston in the syringe to effect drug delivery, and a locking element selectably positioned in one of a locked orientation and an unlocked orientation relative to the control unit; and wherein when the locking element is positioned in the unlocked orientation, the at least one resilient element is permitted to drive the control unit axially forwardly relative to the housing element.

Preferably, the automatic injection device also comprising a needle shield selectably positionable with respect to the housing element. Further preferably, the locking element is selectably operatively engaged with the control unit and wherein the control unit is operative for causing rotation of the locking element under the urge of the at least one resilient element upon axial rearward displacement of the needle shield with respect to the housing element. Still further preferably, the automatic injection device also comprising a plunger rod, operative to selectably drive the at least one syringe piston in axial motion relative to the housing element; the plunger rod is operative to be displaced together with the control unit from the actuation of the control unit up to the penetration position of the syringe.

In accordance with an embodiment of the present invention, the at least one resilient element includes a single spring. Alternatively, the at least one resilient element includes a first spring and a second spring. Preferably, the second spring is at least partially disposed within the plunger rod and is operative for biasing the plunger rod to be displaced forwardly along the longitudinal axis.

Further preferably, the automatic injection device also comprising a needle cover remover configured to be removably attached to the housing element, operative for protecting the needle, and wherein the needle shield is prevented from axial rearward displacement with respect to the housing element when the needle shield remover is attached to the housing element. Still further preferably, the needle cover remover includes at least one fixating element, operative to engage a corresponding fixating counter-element formed on the needle shield to prevent inadvertent rearward displacement of the needle shield with respect to the housing element. Yet further preferably, the locking element is permitted to be rotated in a single rotational direction.

In accordance with an embodiment of the present invention, the locking element has a rotation enabling element and the control unit has a counter rotation enabling element, which engages the rotation enabling element when the locking element is disposed in the locked orientation.

Preferably, the needle shield is prevented from axial forward longitudinal displacement relative to the housing when the locking element is disposed in the locked orientation. Further preferably, in a post-injection operative state, the plunger rod is prevented from rearward axial displacement relative to the housing element. Still further preferably, the locking element is prevented from displacement relative to the housing element due to engagement with the needle shield in a prior to injection operative state and wherein the control unit is prevented from displacement relative to the housing element due to engagement with the locking element in the prior to injection operative state. Yet further preferably, the needle shield comprises at least one stopping rib which engages a protrusion formed on the locking element for restricting rotation of the locking element in the prior to injection operative state.

In accordance with an embodiment of the present invention, an automatic injection device for use with a syringe including at least one syringe piston and a needle coupled to a forward end thereof, comprising a housing element arranged along a longitudinal axis and having a forward end and a rearward end; at least one resilient element arranged to be located within the housing element; a control unit adapted, when actuated, to be driven by the at least one resilient element for initially displacing the syringe relative to the housing element from a non-penetration position to a penetration position and thereafter displacing the at least one syringe piston in the syringe to effect drug delivery, a plunger rod operative to selectably drive the at least one syringe piston in axial motion relative to the housing element; and a locking element selectably positioned in one of a locked orientation and an unlocked orientation relative to the control unit; and wherein when the locking element is positioned in the locked orientation, the plunger rod engages a portion of the locking element and when the locking element is positioned in the unlocked orientation, the plunger rod engages a portion of the control unit.

Preferably, the automatic injection device also comprising a needle shield selectably positionable with respect to the housing element. Further preferably, the locking element is selectably operatively engaged with the control unit and wherein the control unit is operative for causing rotation of the locking element under the urge of the at least one resilient element upon axial rearward displacement of the needle shield with respect to the housing element. Still further preferably, the plunger rod is operative to be displaced together with the control unit from the actuation of the control unit up to the penetration position of the syringe.

Yet further preferably, the at least one resilient element includes a single spring. Alternatively, the at least one resilient element includes a first spring and a second spring. Preferably, the second spring is at least partially disposed within the plunger rod and is operative for biasing the plunger rod to be displaced forwardly along the longitudinal axis.

In accordance with an embodiment of the present invention, the automatic injection device also comprising a needle cover remover configured to be removably attached to the housing element, operative for protecting the needle, and wherein said needle shield is prevented from axial rearward displacement with respect to the housing element when the needle shield remover is attached to the housing element.

Preferably, the needle cover remover includes at least one fixating element, operative to engage a corresponding fixating counter-element formed on the needle shield to prevent inadvertent rearward displacement of the needle shield with respect to the housing element. Further preferably, the locking element is permitted to be rotated in a single rotational direction. Still further preferably, the locking element has a rotation enabling element and the control unit has a counter rotation enabling element, which engages the rotation enabling element when the locking element is disposed in the locked orientation. Yet further preferably, the needle shield is prevented from axial forward longitudinal displacement relative to the housing when the locking element is disposed in the locked orientation.

In accordance with an embodiment of the present invention, in a post-injection operative state, the plunger rod is prevented from rearward axial displacement relative to the housing element. Preferably, the locking element is prevented from displacement relative to the housing element due to engagement with the needle shield in a prior to injection operative state and wherein the control unit is prevented from displacement relative to the housing element due to engagement with the locking element in the prior to injection operative state. Further preferably, the needle shield comprises at least one stopping rib which engages a protrusion formed on the locking element for restricting rotation of the locking element in the prior to injection operative state.

In accordance with an embodiment of the present invention, an automatic injection device for use with a syringe including at least one syringe piston and a needle coupled to a forward end thereof, comprising a housing element arranged along a longitudinal axis and having a forward end and a rearward end; at least one resilient element arranged to be located within the housing element; a needle shield selectably positionable with respect to the housing element; a needle cover remover configured to be removably attached to the housing element; and a control unit adapted, when actuated, to be driven by the at least one resilient element for initially displacing the syringe relative to the housing element from a non-penetration position to a penetration position and thereafter displacing the at least one syringe piston in the syringe to effect drug delivery, and wherein the needle shield is prevented from axial rearward displacement with respect to the housing element when the needle shield remover is attached to the housing element.

Preferably, the automatic injection device also comprising a locking element operative for selectable displacement relative to the housing element and being operatively engageable with the needle shield and wherein upon axial rearward displacement of the needle shield with respect to the housing element, the locking element is permitted to rotate about the longitudinal axis under the urge of the at least one resilient element.

Further preferably, the locking element is selectably operatively engaged with the control unit and wherein the control unit is operative for causing rotation of the locking element under the urge of the at least one resilient element upon axial rearward displacement of the needle shield with respect to the housing element. Still further preferably, the automatic injection device also comprising a plunger rod, operative to selectably drive the at least one syringe piston in axial motion relative to the housing element; the plunger rod is operative to be displaced together with the control unit from the actuation of the control unit up to the penetration position of the syringe.

In accordance with an embodiment of the present invention, the at least one resilient element includes a single spring. Alternatively, the at least one resilient element includes a first spring and a second spring. Preferably, the second spring is at least partially disposed within the plunger rod and is operative for biasing the plunger rod to be displaced forwardly along the longitudinal axis.

Preferably, the needle cover remover includes at least one fixating element, operative to engage a corresponding fixating counter-element formed on the needle shield to prevent inadvertent rearward displacement of the needle shield with respect to the housing element. Further preferably, the locking element is selectably positioned in one of a locked orientation and an unlocked orientation relative to the control unit; and wherein when the locking element is positioned in the unlocked orientation, the at least one resilient element is permitted to drive the control unit axially forwardly relative to the housing element. Still further preferably, the locking element has a rotation enabling element and the control unit has a counter rotation enabling element, which engages the rotation enabling element when the locking element is disposed in the locked orientation.

In accordance with an embodiment of the present invention, the needle shield is prevented from axial forward longitudinal displacement relative to the housing when the locking element is disposed in the locked orientation.

Preferably, in a post-injection operative state, the plunger rod is prevented from rearward axial displacement relative to the housing element. Further preferably, the automatic injection device also comprising a locking element, which is prevented from displacement relative to the housing element due to engagement with the needle shield in a prior to injection operative state and wherein the control unit is prevented from displacement relative to the housing element due to engagement with the locking element in the prior to injection operative state. Still further preferably, the needle shield comprises at least one stopping rib which engages a protrusion formed on the locking element for restricting rotation of the locking element in the prior to injection operative state.

GB 2 560 558 A discloses a prior art automatic injection device.

### BRIEF DESCRIPTION OF THE DRAWINGS

The present invention will be understood and appreciated more fully from the following detailed description, taken in conjunction with the drawings in which:

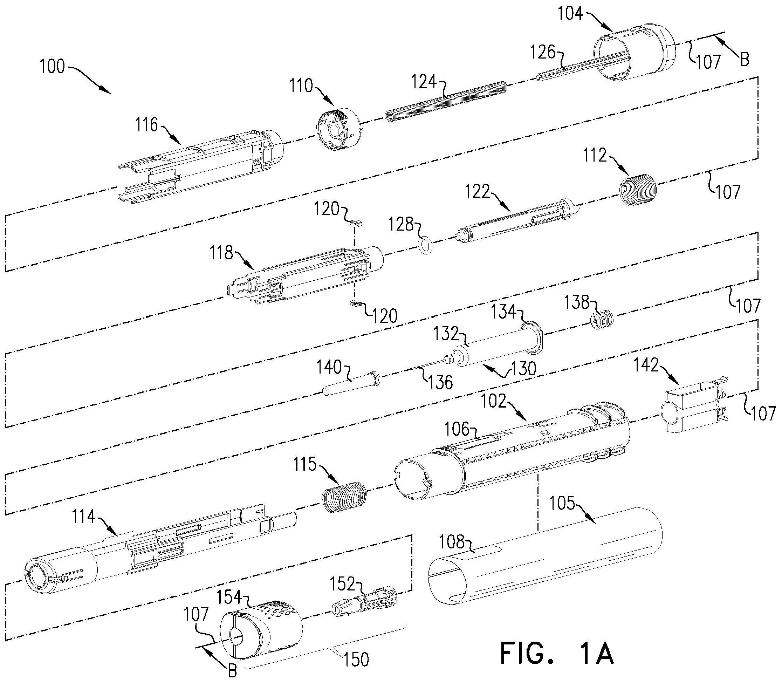

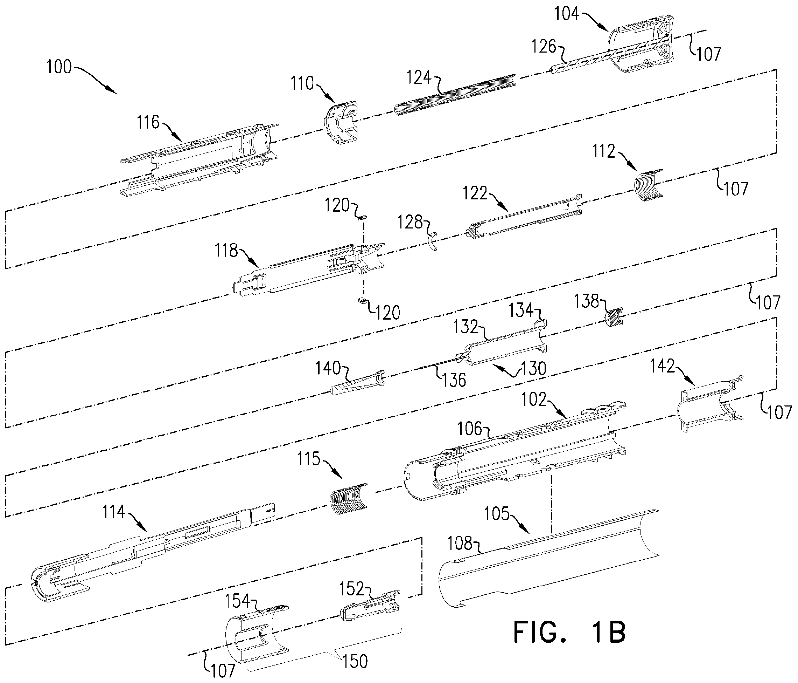

Figs. 1A and 1B are respectively simplified exploded view and sectional exploded view of an emergency automatic injection assembly constructed and operative in accordance with an embodiment of the present invention, the sectional view taken along lines B - B in Fig. 1A;

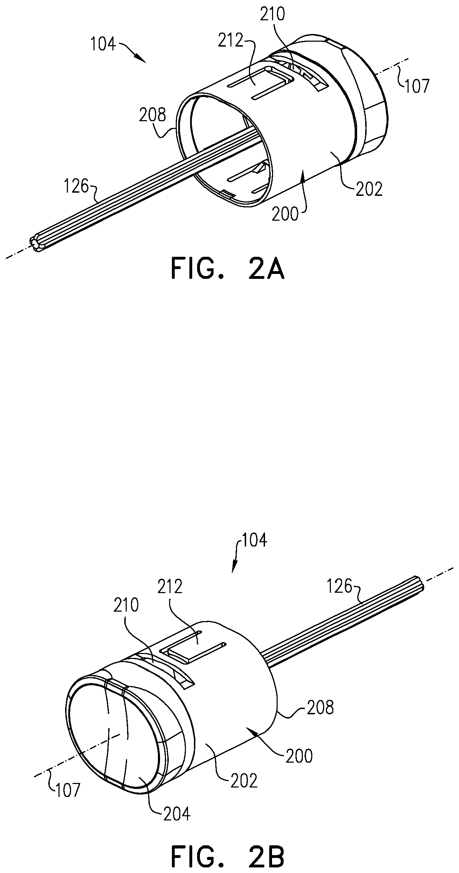

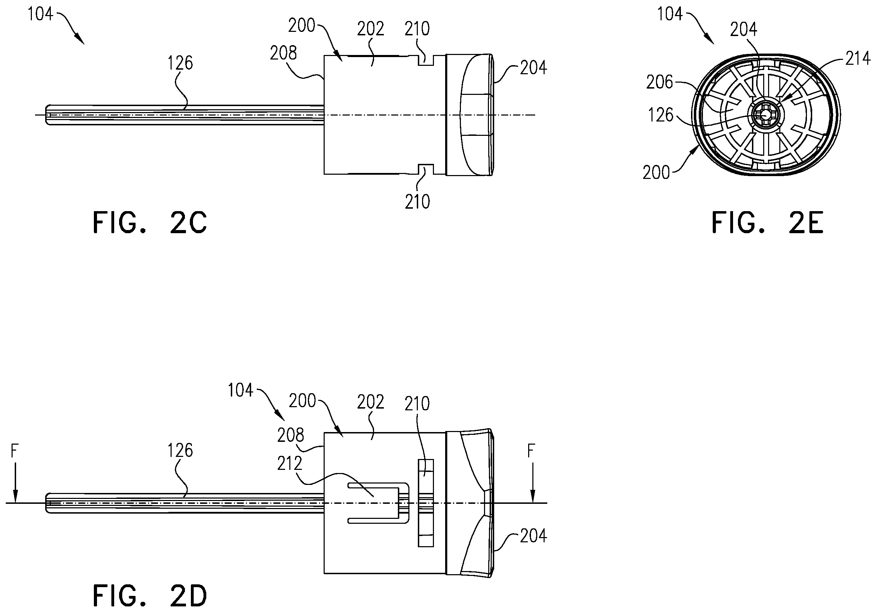

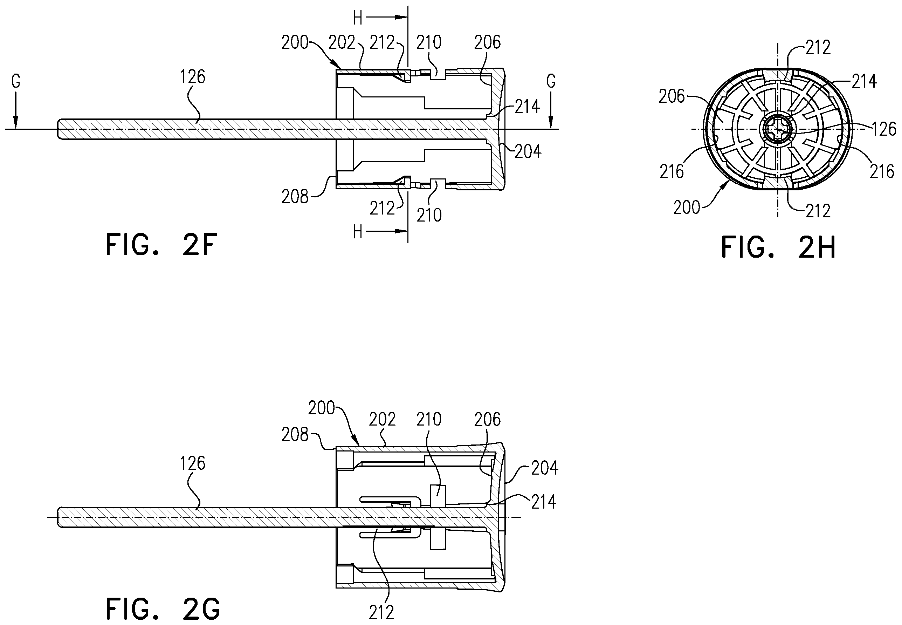

Figs. 2A, 2B, 2C, 2D, 2E, 2F, 2G & 2H are respectively a simplified perspective view of a forward-facing portion, a simplified perspective view of a rearward-facing portion, two simplified side plan views, a simplified top plan view, three simplified sectional views taken along lines F - F in Fig. 2D, lines G - G in Fig. 2F and lines H - H in Fig. 2F respectively of a rear end element forming part of the emergency automatic injection assembly of Figs. 1A & 1B;

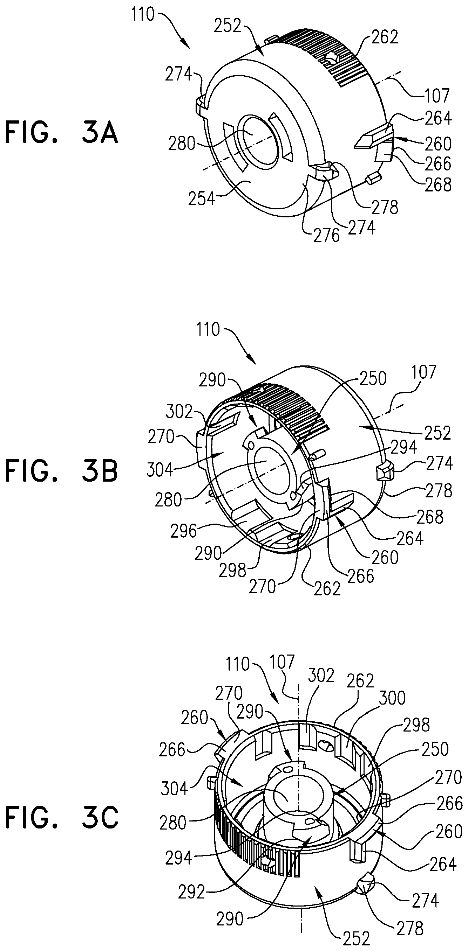

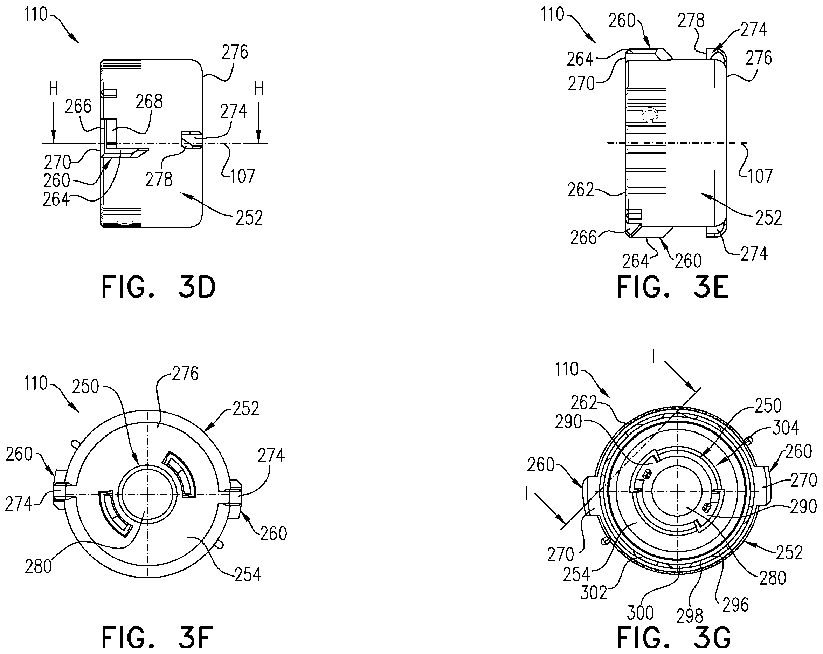

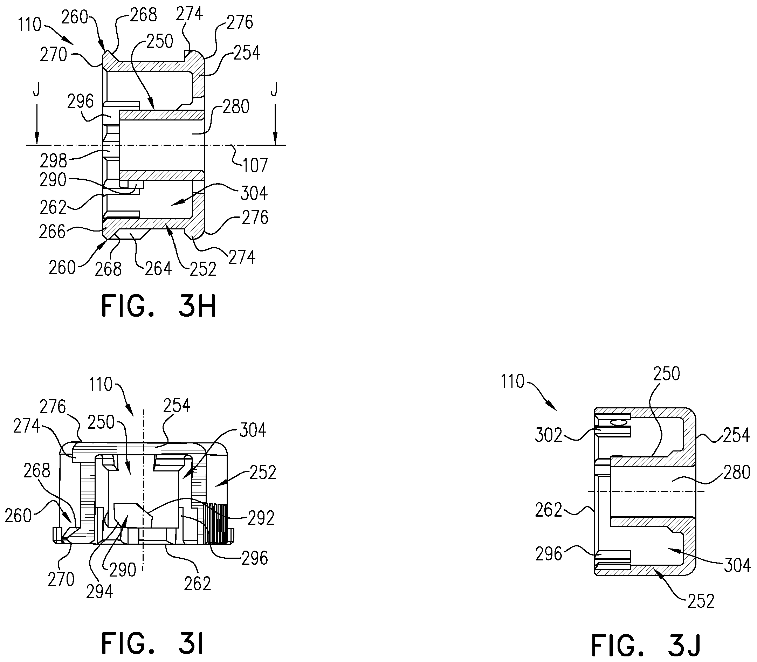

Figs. 3A, 3B, 3C, 3D, 3E, 3F, 3G, 3H, 3I & 3J are respectively three simplified perspective views, two simplified side plan views, a simplified top plan view, a simplified bottom plan view and three simplified sectional views taken along lines H - H in Fig. 3D, lines I - I in Fig. 3G and lines J - J in Fig. 3H respectively of a locking ring element forming part of the emergency automatic injection assembly of Figs. 1A & 1B;

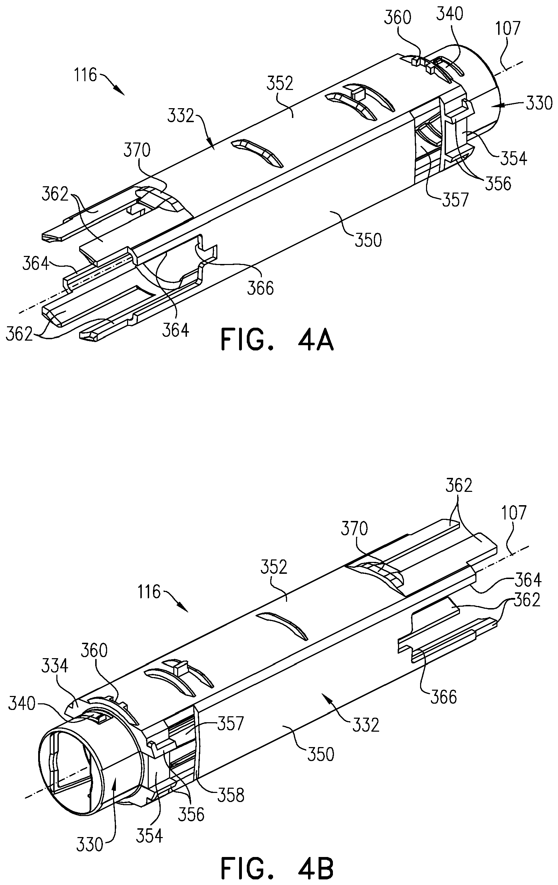

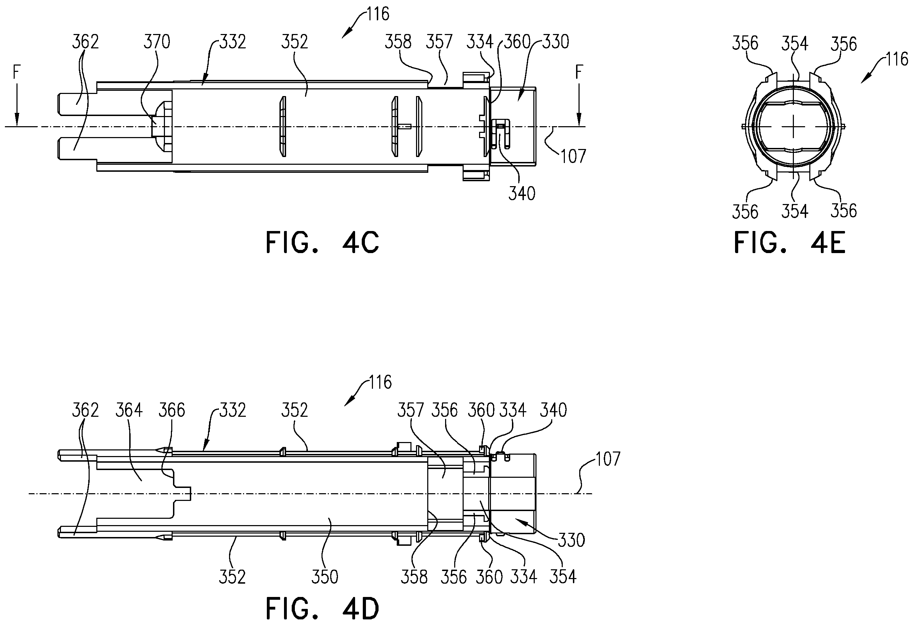

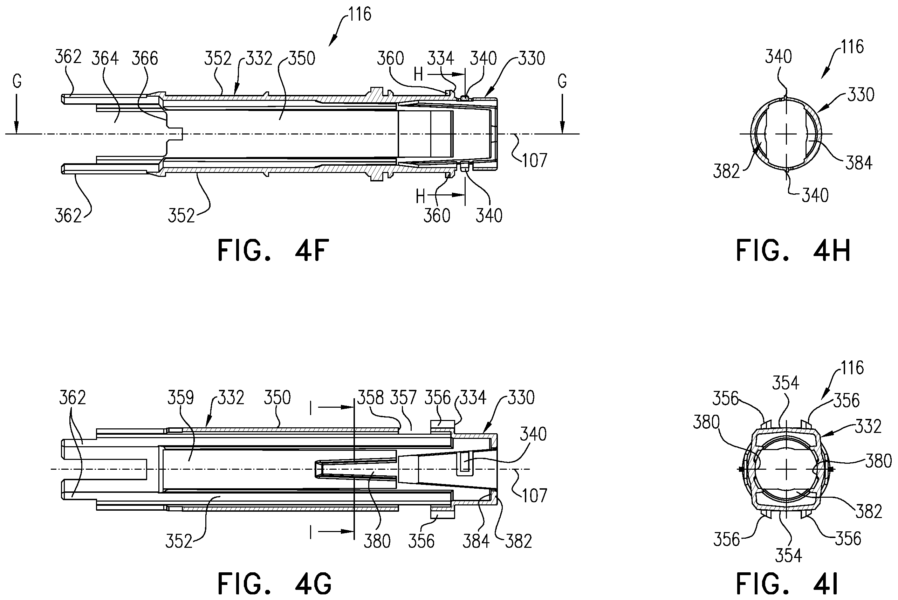

Figs. 4A, 4B, 4C, 4D, 4E, 4F, 4G, 4H & 4I are respectively two simplified perspective views, two simplified side plan views, a simplified top plan view, and four simplified sectional views taken along lines F - F in Fig. 4C, lines G - G in Fig. 4F, lines H - H in Fig. 4F and lines I - I in Fig. 4G respectively of the rear housing element forming part of the emergency automatic injection assembly of Figs. 1A & 1B;

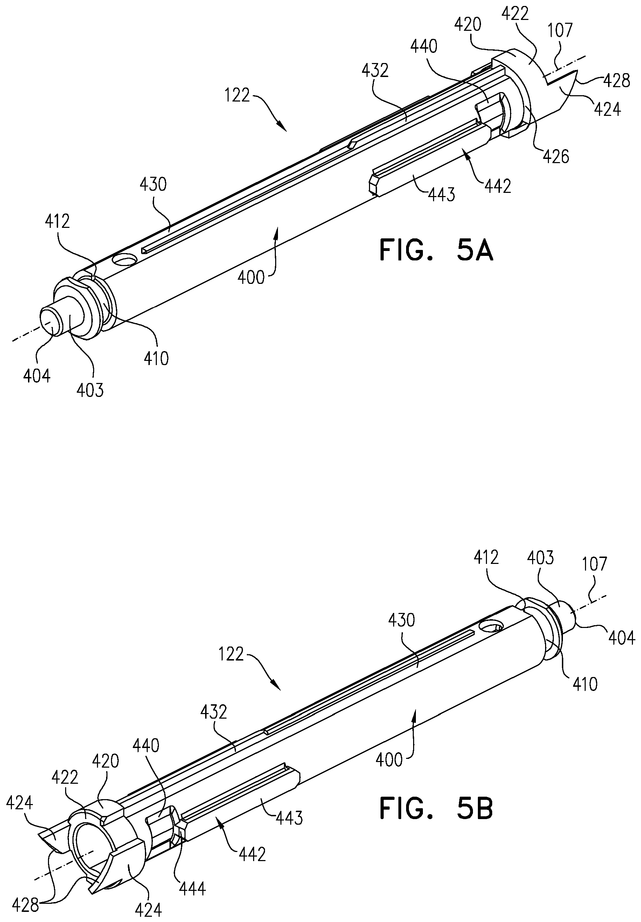

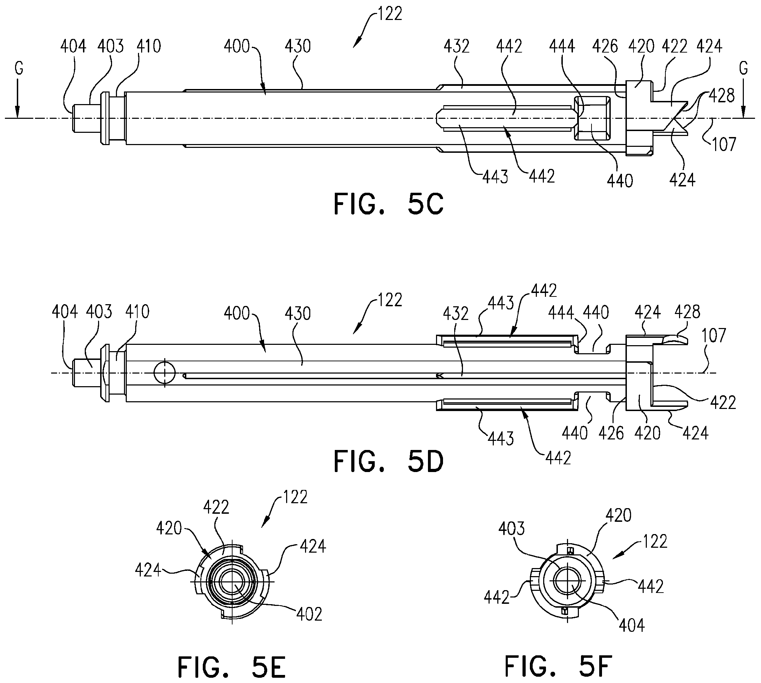

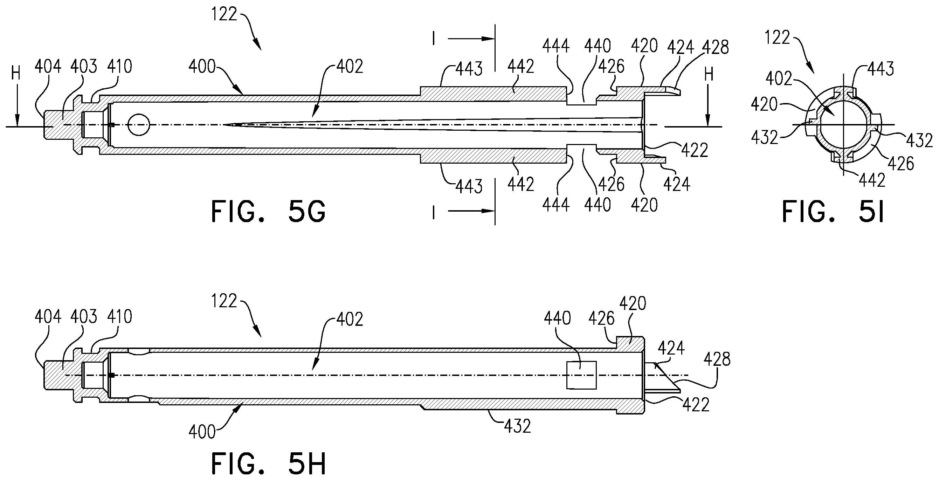

Figs. 5A, 5B, 5C, 5D, 5E, 5F, 5G, 5H & 5I are respectively two simplified perspective views, two simplified side plan views, a simplified top plan view, a simplified bottom plan view and three simplified sectional views taken along lines G - G in Fig. 5C, lines H - H and lines I - I in Fig. 5G of a plunger rod element forming part of the emergency automatic injection assembly of Figs. 1A & 1B;

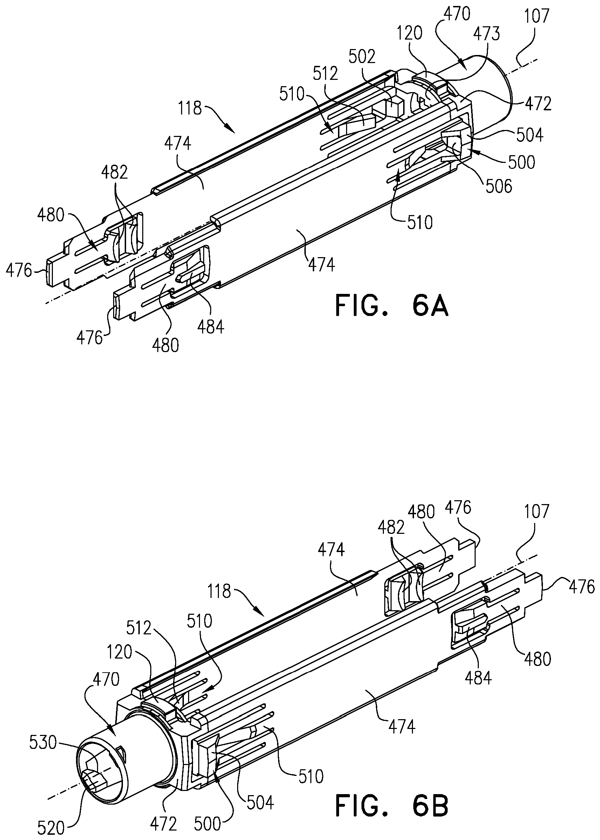

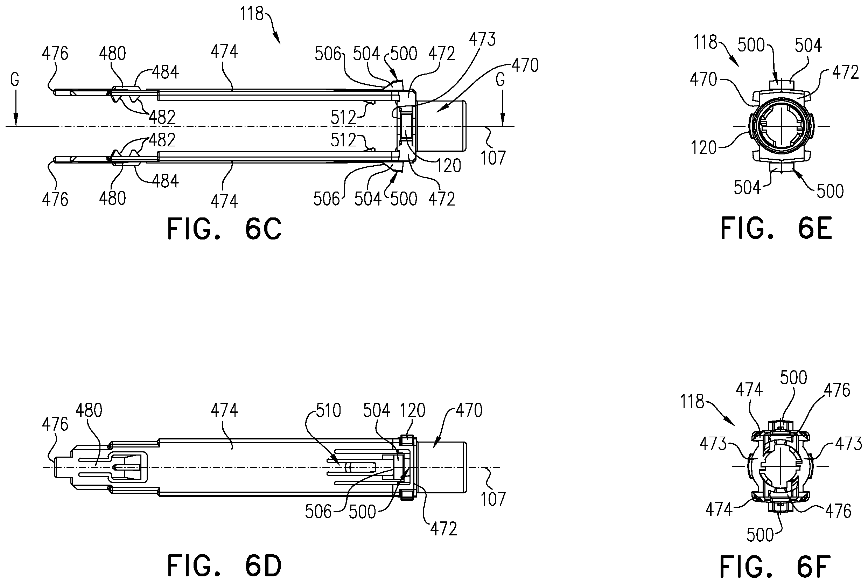

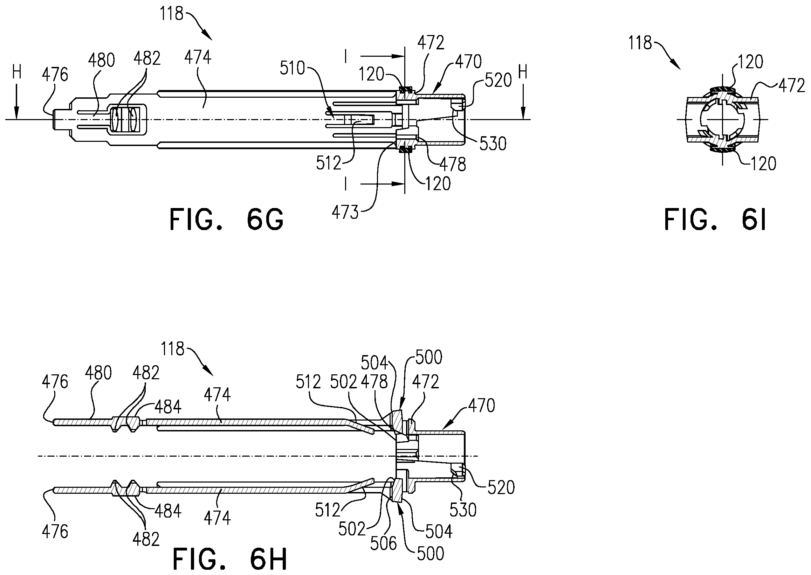

Figs. 6A, 6B, 6C, 6D, 6E, 6F, 6G, 6H and 6I are respectively two simplified perspective views, two simplified side plan views, a simplified top plan view, a simplified bottom plan view and three simplified sectional views taken along lines G - G in Fig. 6C, lines H - H and lines I - I in Fig. 6G of a control unit element forming part of the emergency automatic injection assembly of Figs. 1A & 1B;

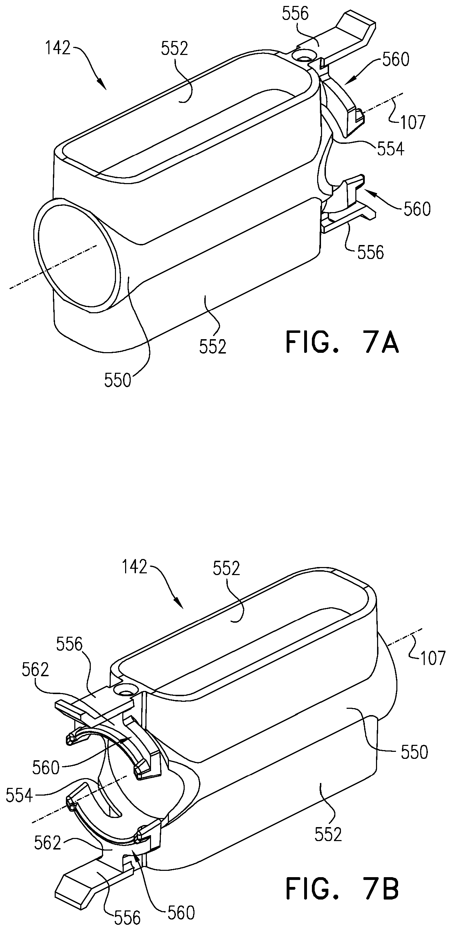

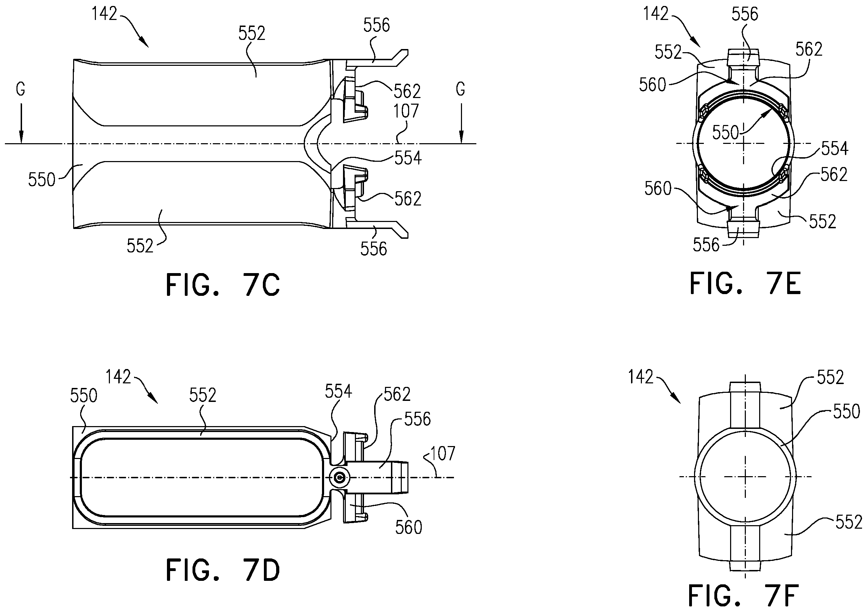

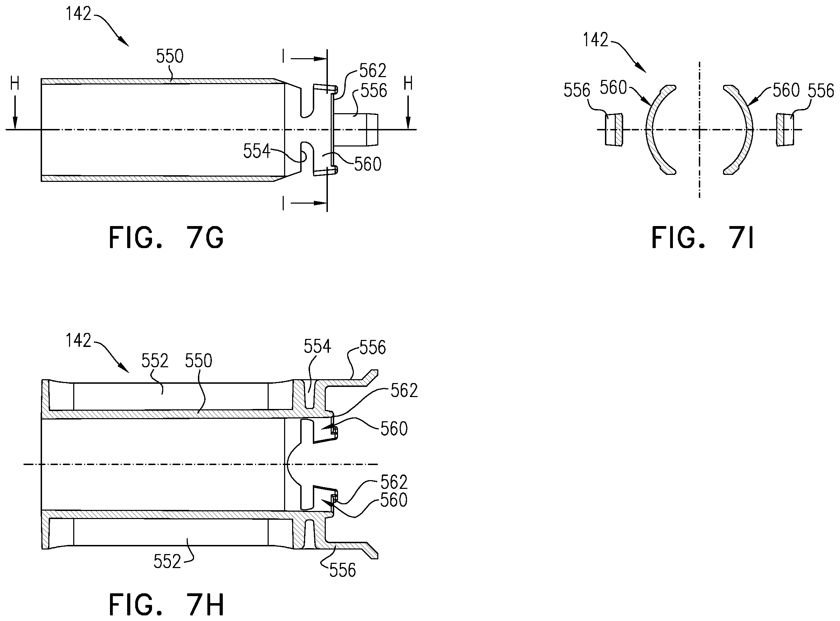

Figs. 7A, 7B, 7C, 7D, 7E, 7F, 7G, 7H and 7I are respectively two simplified perspective views, two simplified side plan views, a simplified top plan view, a simplified bottom plan view and three simplified sectional views taken along lines G - G in Fig. 7C, lines H - H and lines I - I in Fig. 7G of a syringe sleeve element forming part of the emergency automatic injection assembly of Figs. 1A & 1B;

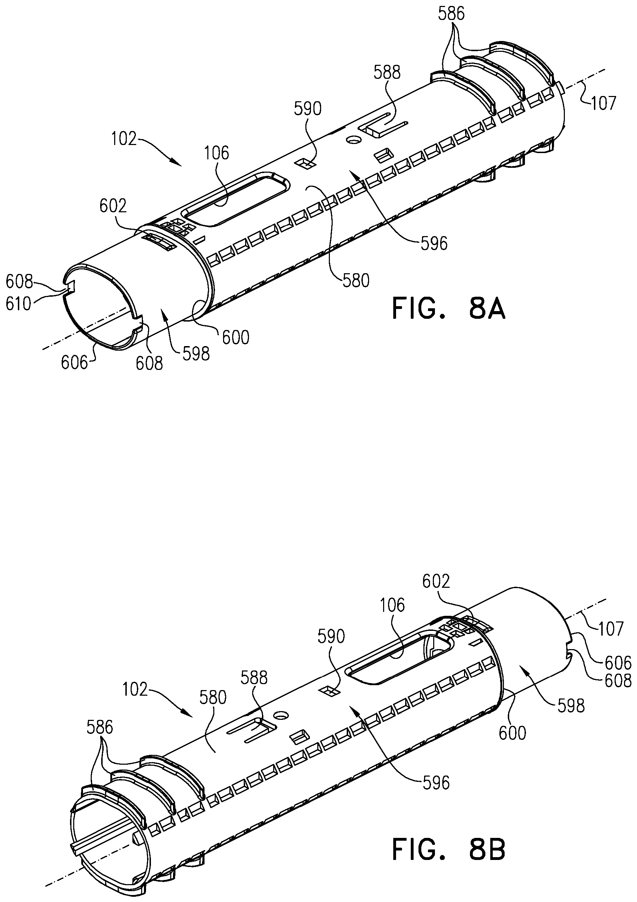

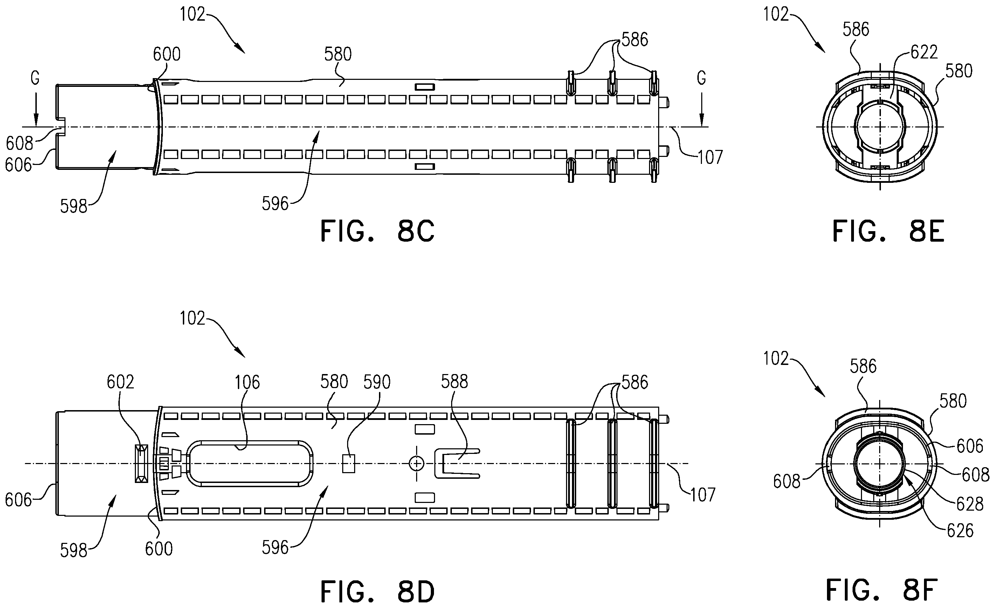

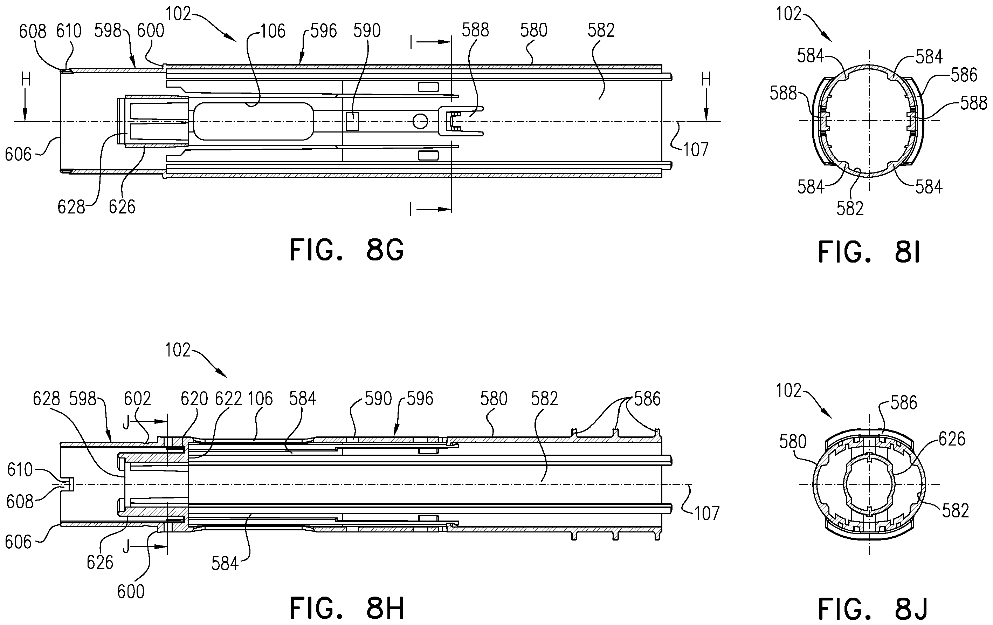

Figs. 8A, 8B, 8C, 8D, 8E, 8F, 8G, 8H, 8I and 8J are respectively two simplified perspective views, two simplified side plan views, a simplified top plan view, a simplified bottom plan view and four simplified sectional views taken along lines G - G in Fig. 8C, lines H - H and lines I - I in Fig. 8G and lines J - J in Fig. 8H of the front housing element forming part of the emergency automatic injection assembly of Figs. 1A & 1B;

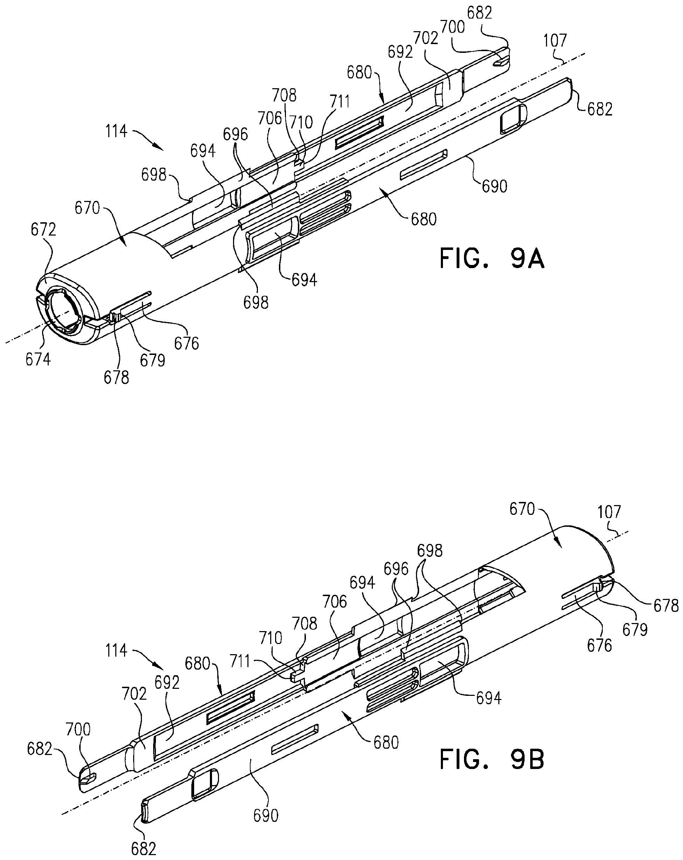

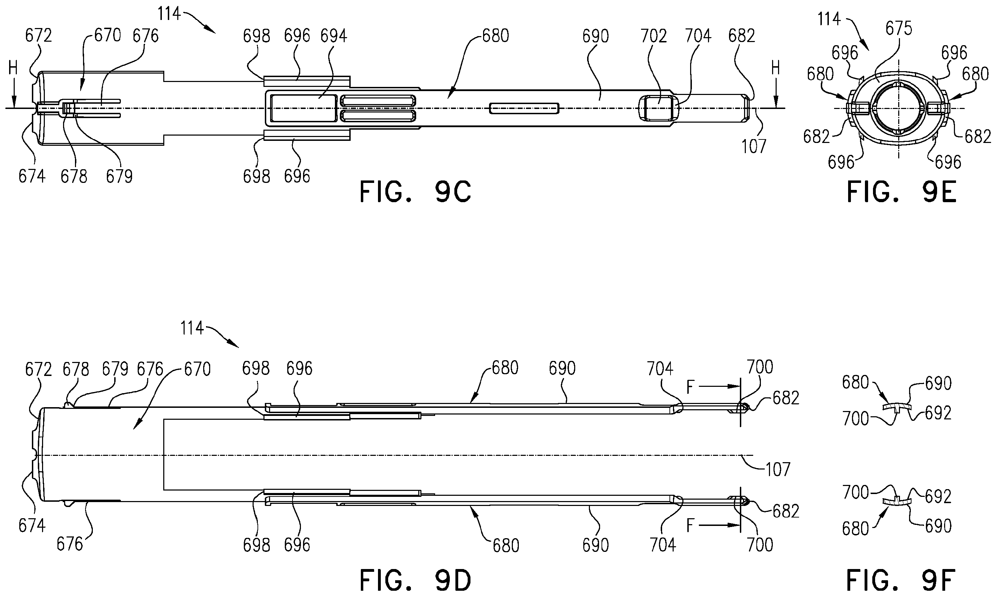

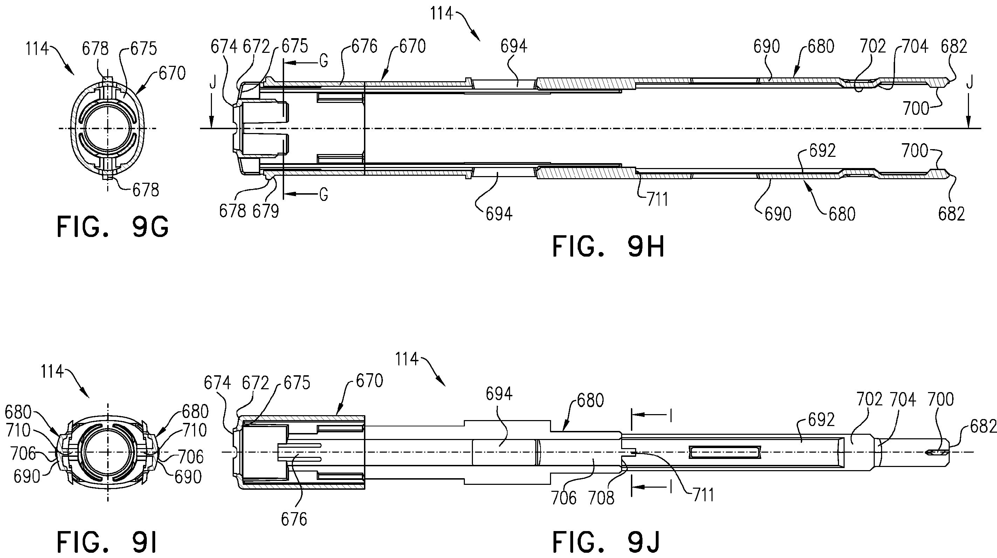

Figs. 9A, 9B, 9C, 9D, 9E, 9F, 9G, 9H, 9I and 9J are respectively two simplified perspective views, two simplified side plan views, a simplified top plan view, and five simplified sectional views taken along lines H - H in Fig. 9C, lines F - F in Fig. 9D, lines G - G and lines J - J in Fig. 9H and lines I - I in Fig. 9J of the needle shield element forming part of the emergency automatic injection assembly of Figs. 1A & 1B;

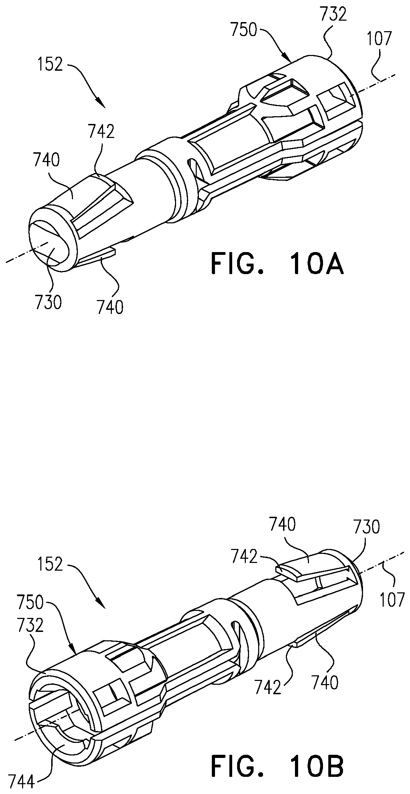

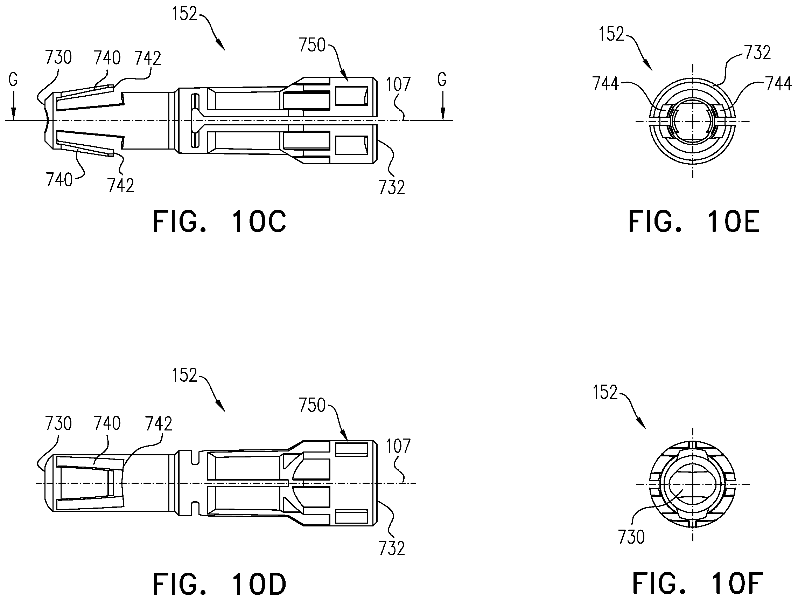

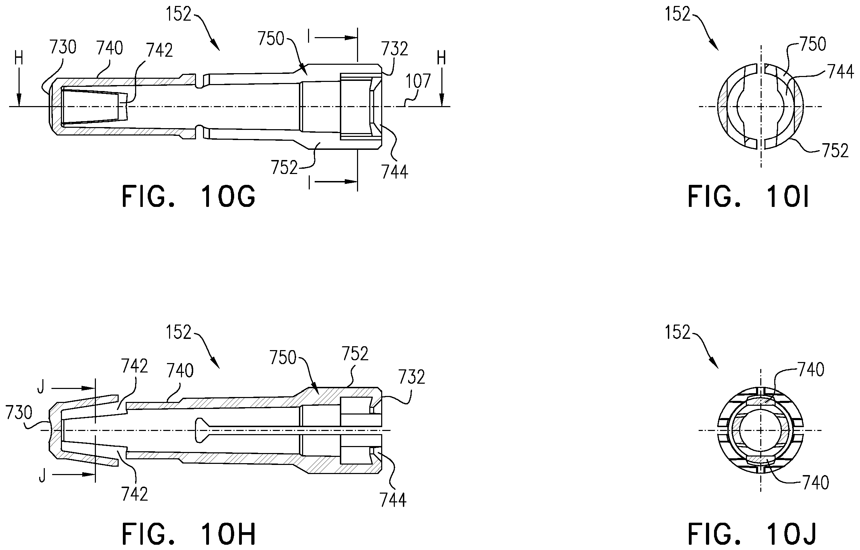

Figs. 10A, 10B, 10C, 10D, 10E, 10F, 10G, 10H, 10I and 10J are respectively two simplified perspective views, two simplified side plan views, a simplified top plan view, a simplified bottom plan view and four simplified sectional views taken along lines G - G in Fig. 10C, lines H - H and lines I - I in Fig. 10G, and lines J - J in Fig. 10H of the floating cylinder element forming part of the emergency automatic injection assembly of Figs. 1A & 1B;

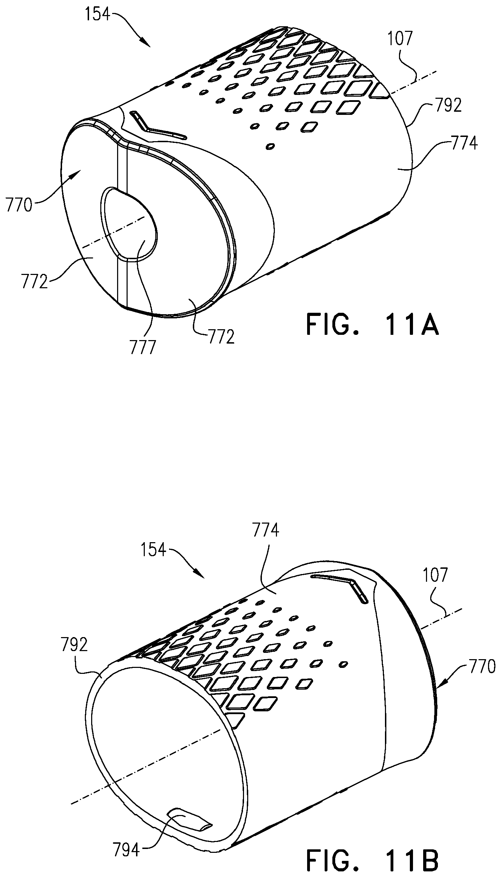

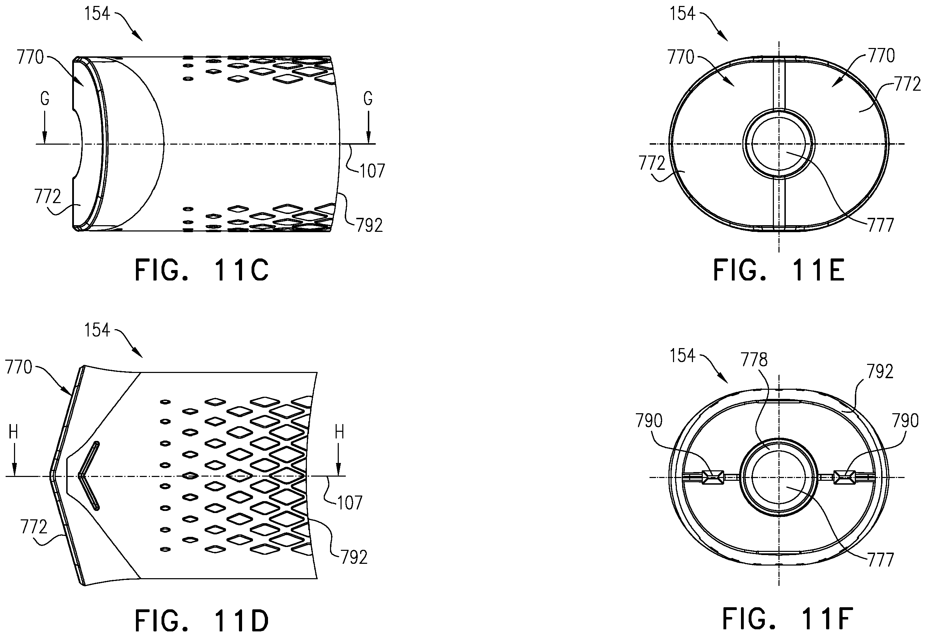

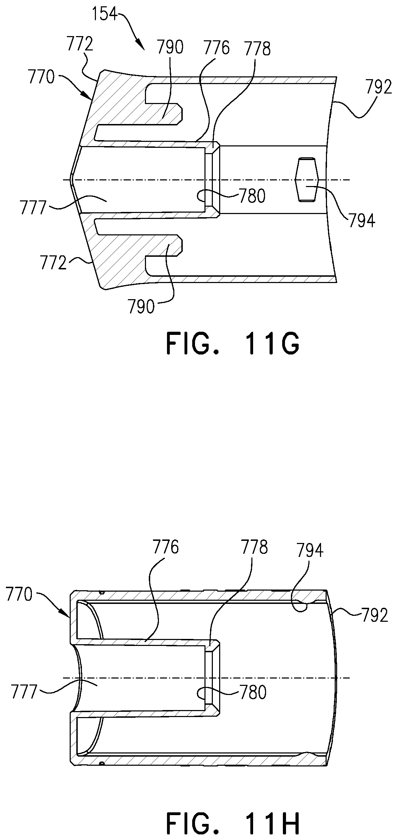

Figs. 11A, 11B, 11C, 11D, 11E, 11F, 11G and 11H are respectively two simplified perspective views, two simplified side plan views, a simplified top plan view, a simplified bottom plan view and two simplified sectional views taken along lines G - G in Fig. 11C and lines H - H in Fig. 11D of a safety cap element forming part of the emergency automatic injection assembly of Figs. 1A & 1B;

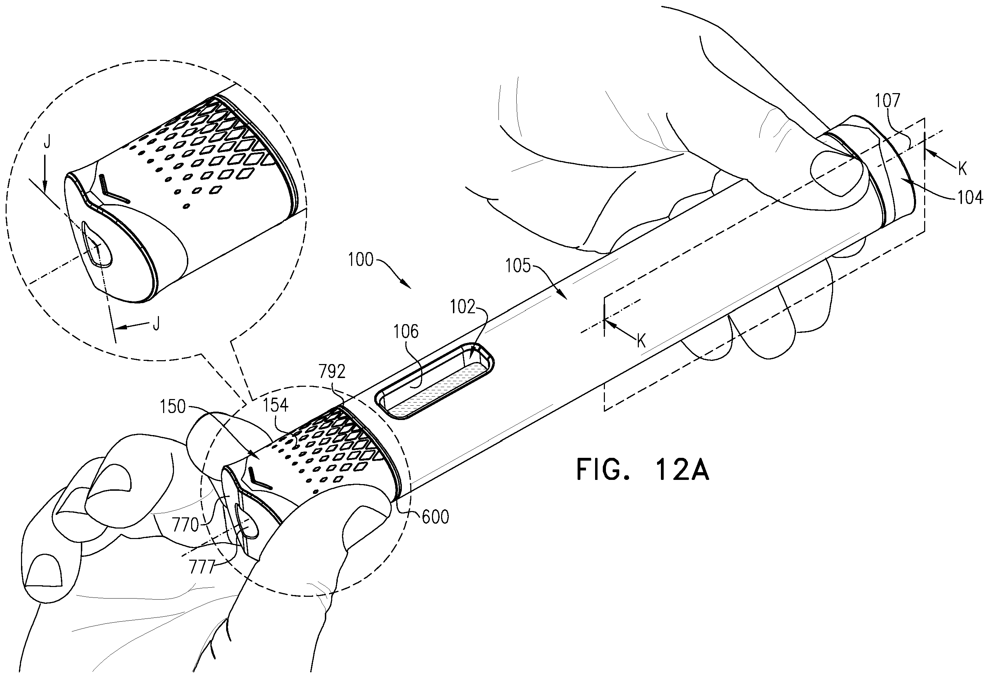

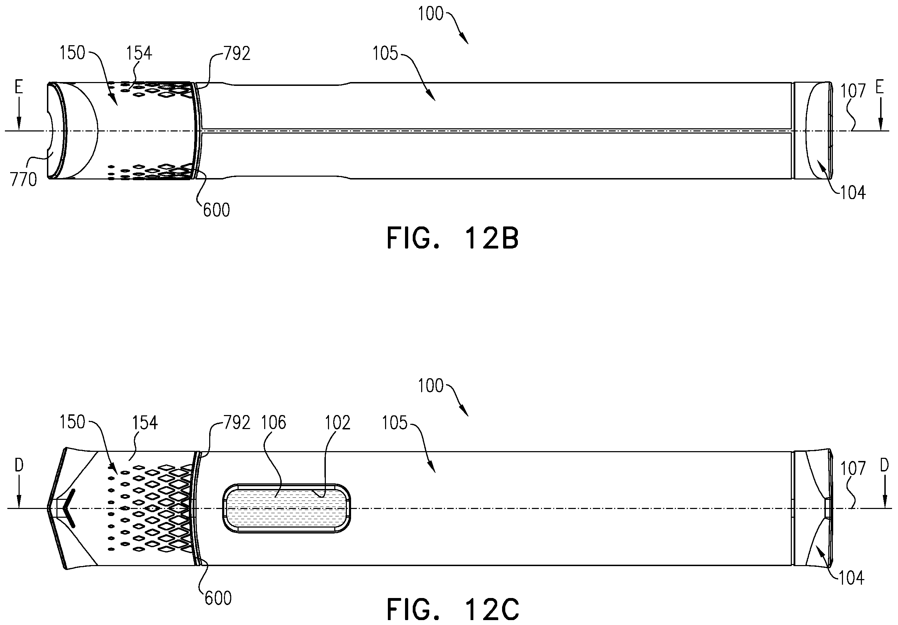

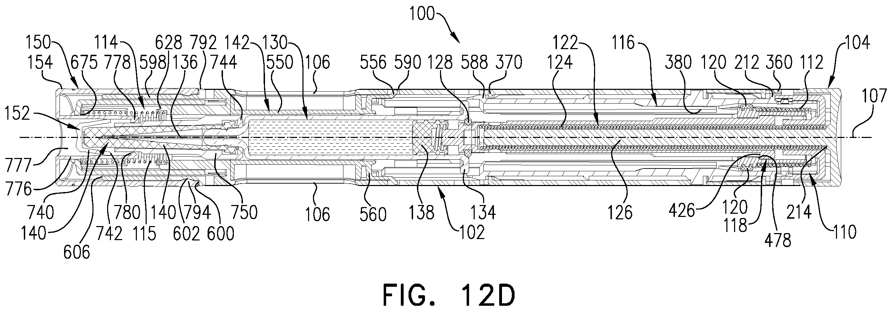

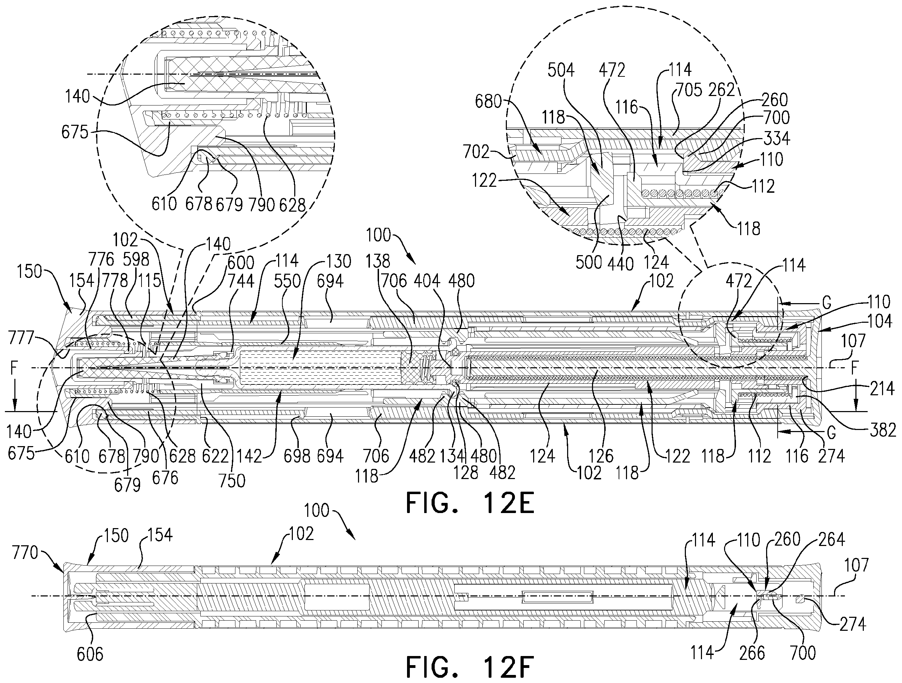

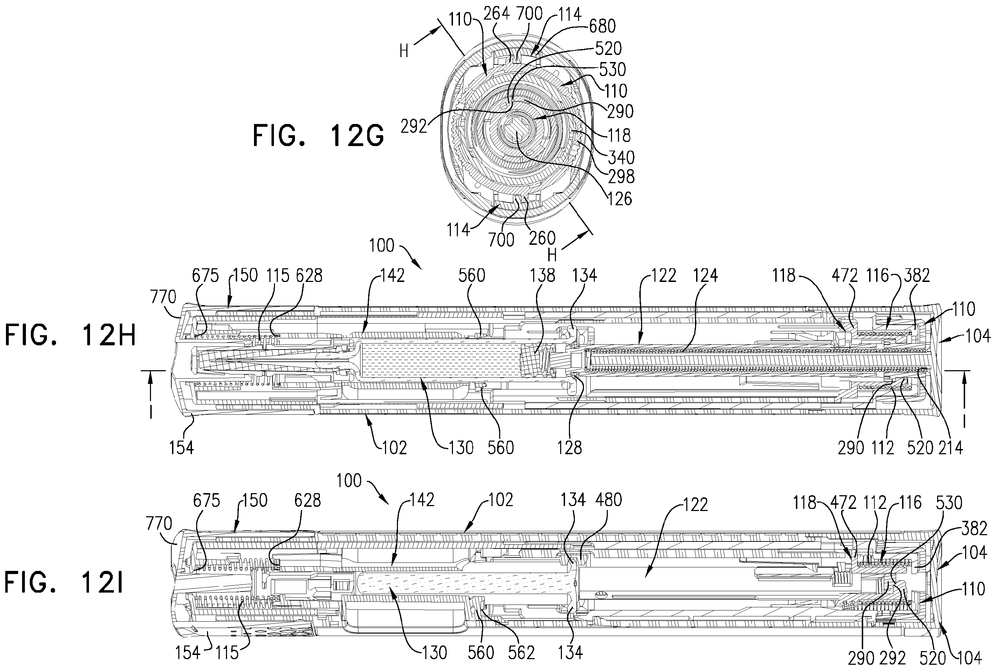

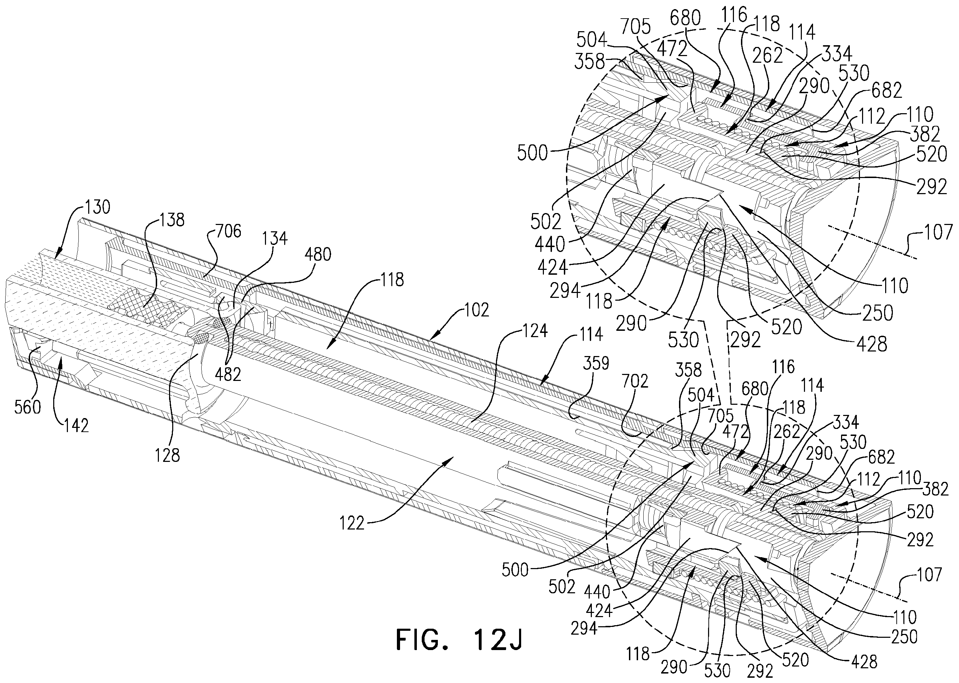

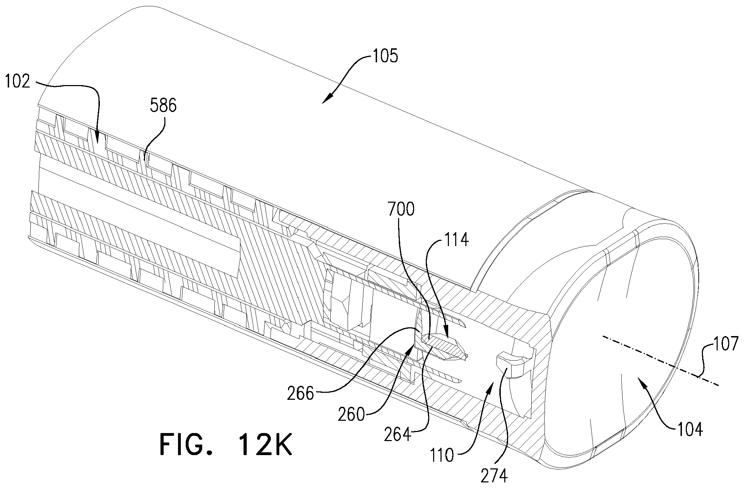

Figs. 12A, 12B, 12C, 12D, 12E, 12F, 12G, 12H, 12I, 12J and 12K are simplified drawings of the emergency automatic injection assembly of Figs. 1A - 11H in a "storage" operative orientation, including a simplified perspective view, two simplified side plan views, seven simplified sectional views taken along lines D - D in Fig. 12C, lines E - E in Fig. 12B, lines F - F and G - G in Fig. 12E, lines H - H in Fig. 12G and lines I - I in Fig. 12H and two partial cut-out views taken along lines J - J and K - K in Fig. 12A, shown without the forward portion of the emergency automatic injection assembly;

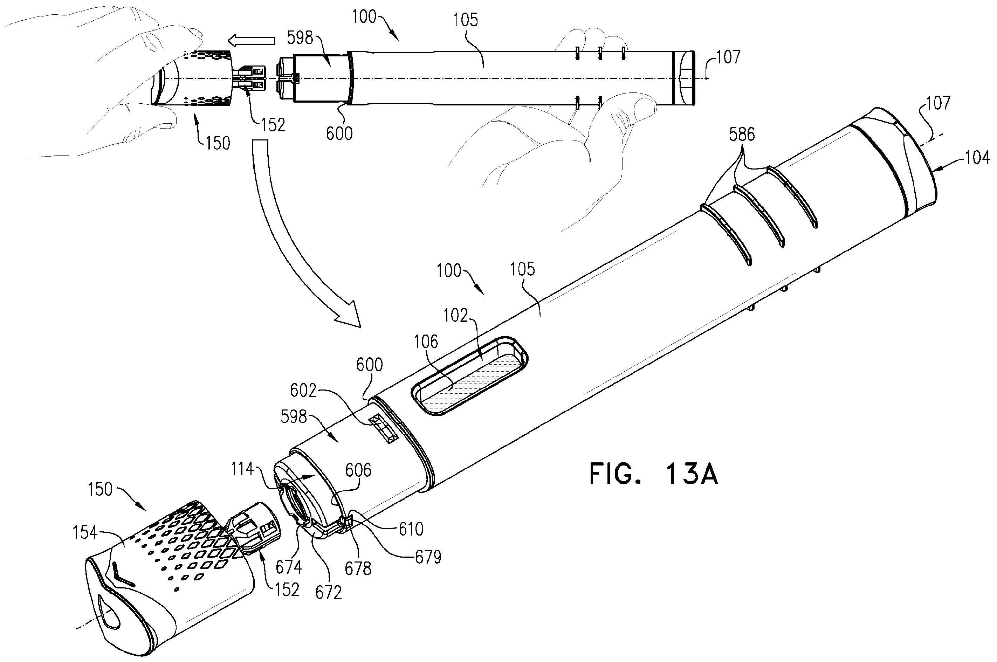

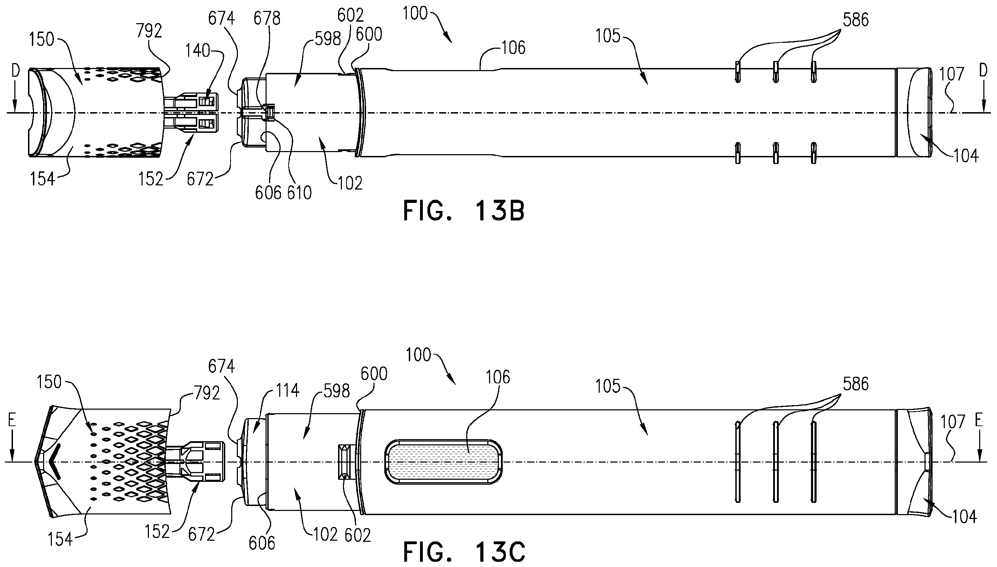

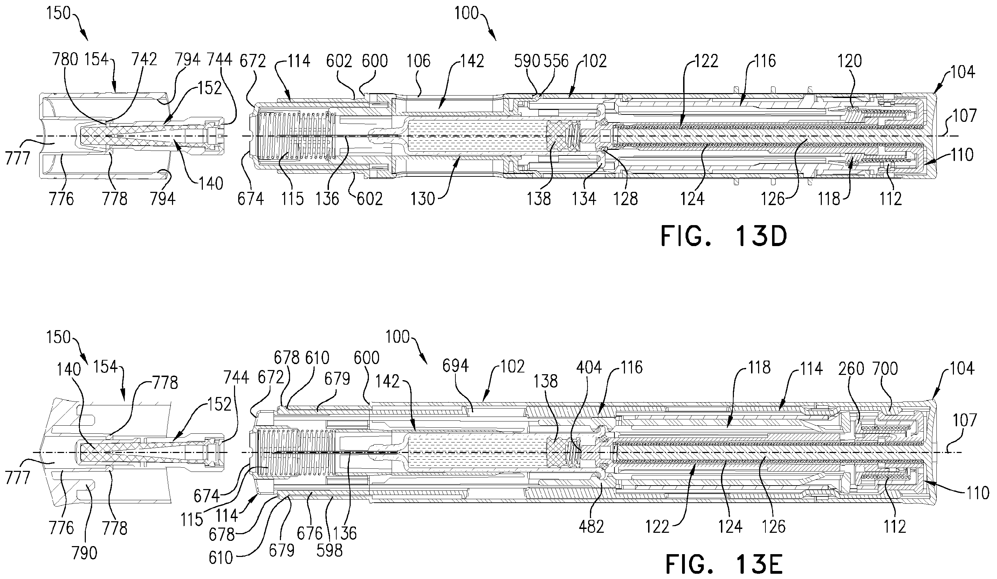

Figs. 13A, 13B, 13C, 13D and 13E are simplified drawings of the emergency automatic injection assembly of Figs. 1A - 11H in a cover removal operative orientation, including a simplified perspective view, two simplified side plan views, and two simplified sectional views taken along lines D - D in Fig. 13B and lines E - E in Fig. 13C;

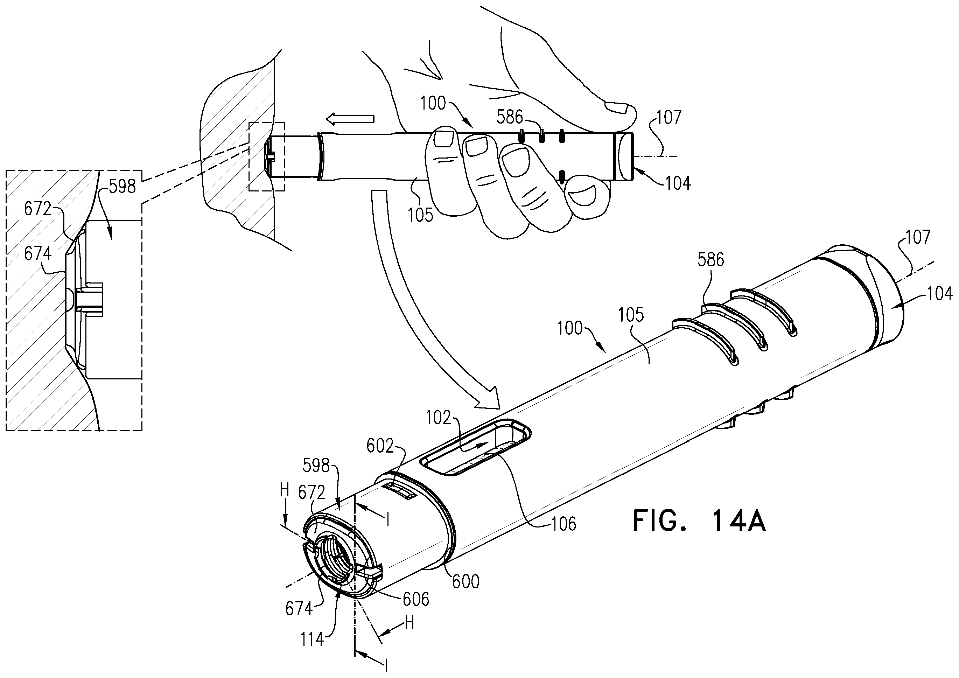

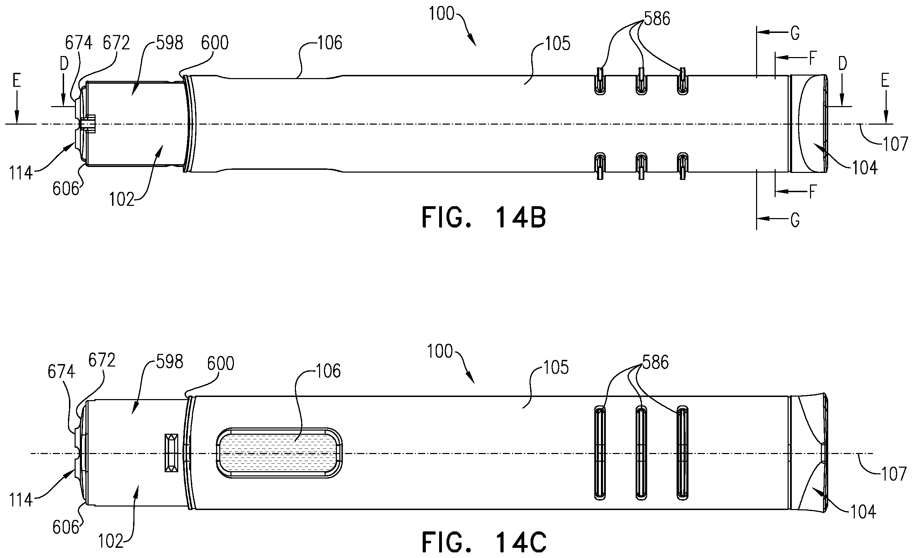

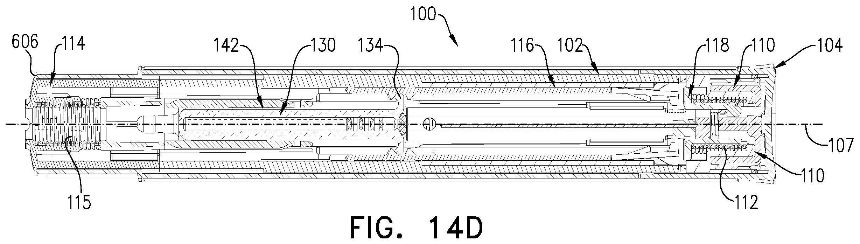

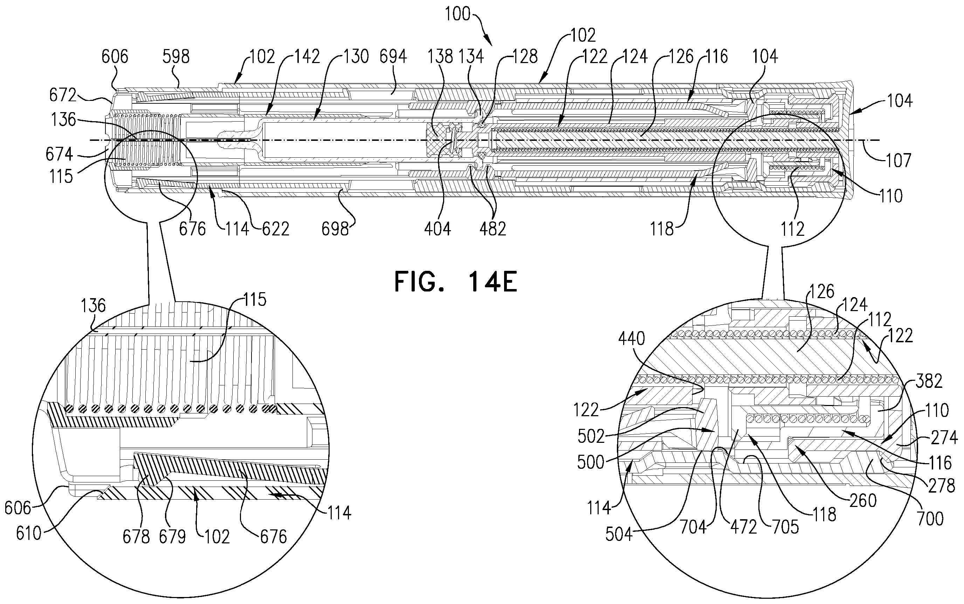

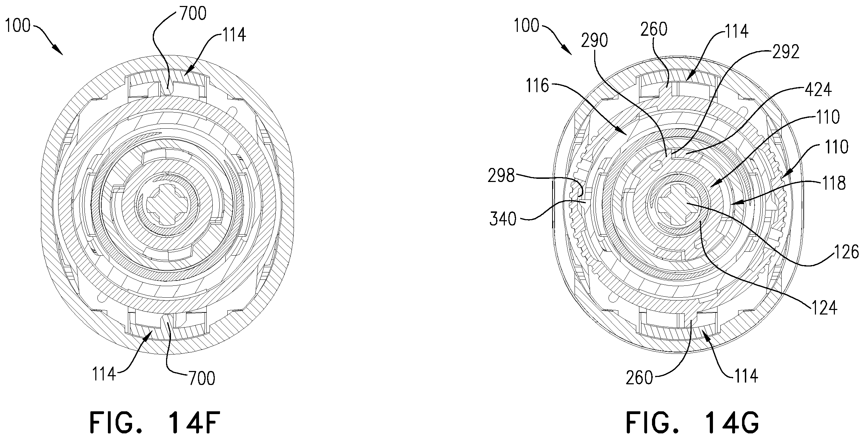

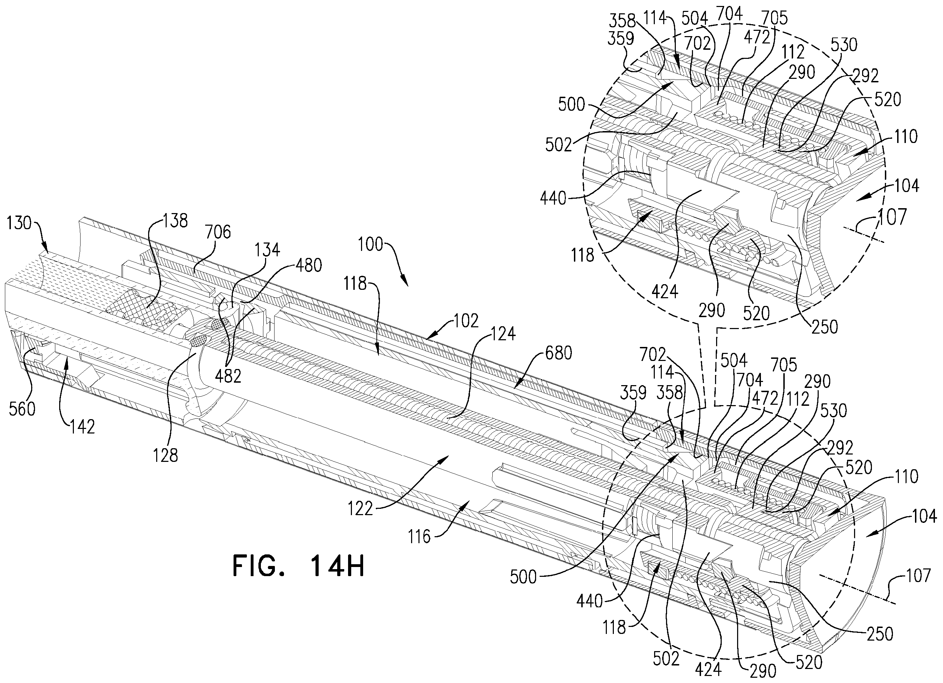

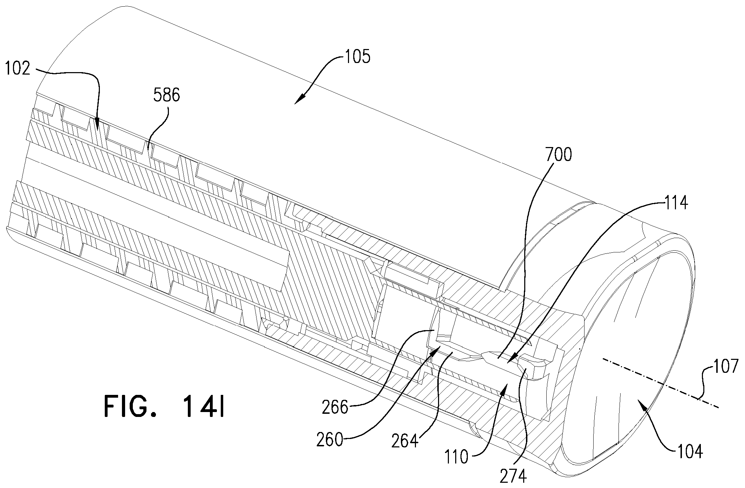

Figs. 14A, 14B, 14C, 14D, 14E, 14F, 14G, 14H and 14I are simplified drawings of the emergency automatic injection assembly of Figs. 1A - 11H in a first activation stage operative orientation, including a simplified perspective view, two simplified side plan views, four simplified sectional views taken along lines D - D, E - E, F - F and lines G - G in Fig. 14B, and lines H - H and lines I - I in Fig. 14A and two partial cut-out views taken along lines H - H and I - I in Fig. 14A, shown without the forward portion of the emergency automatic injection assembly;

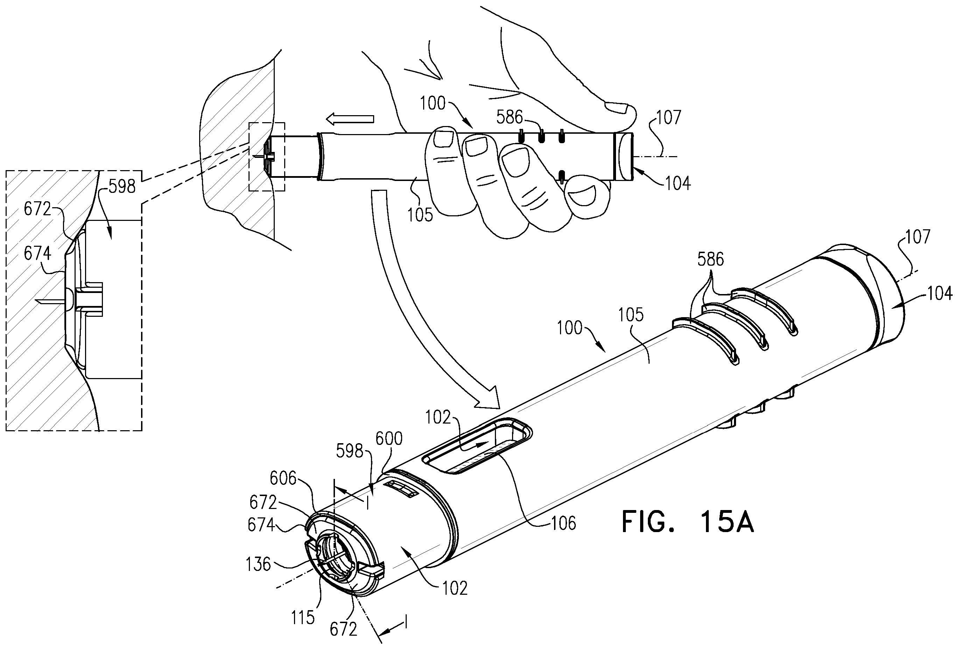

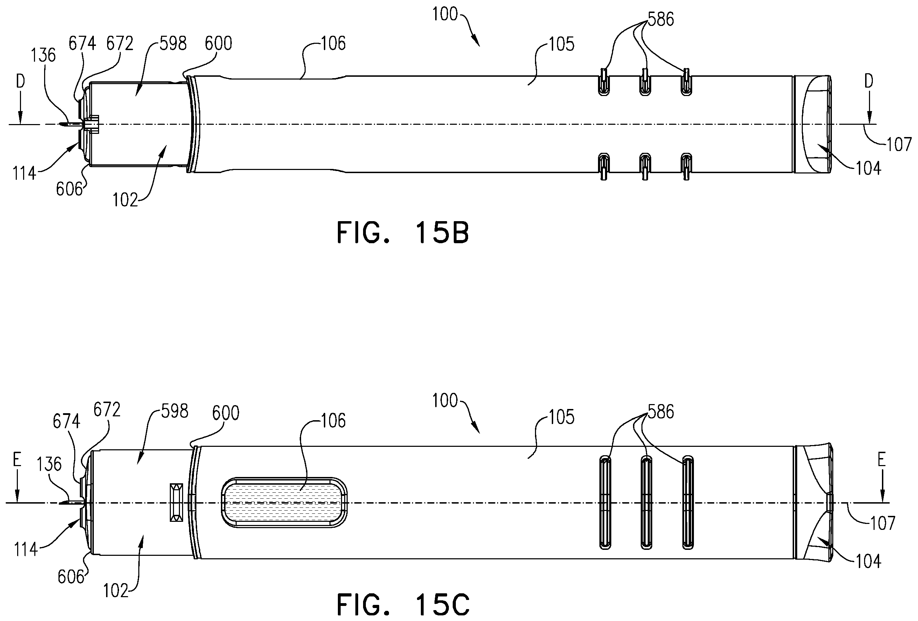

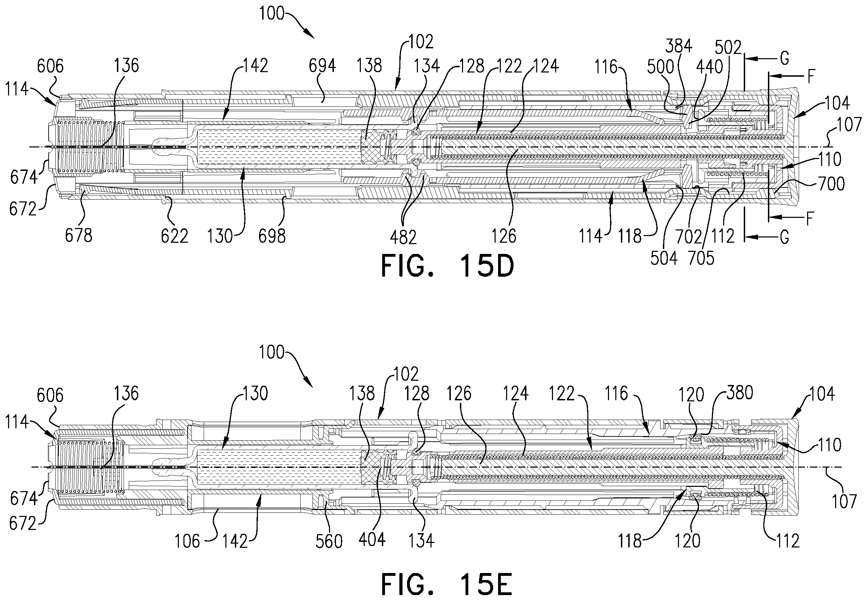

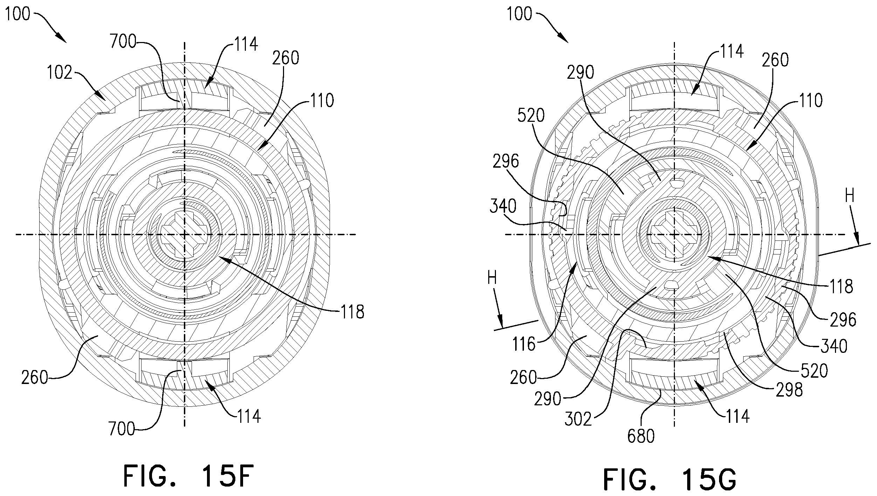

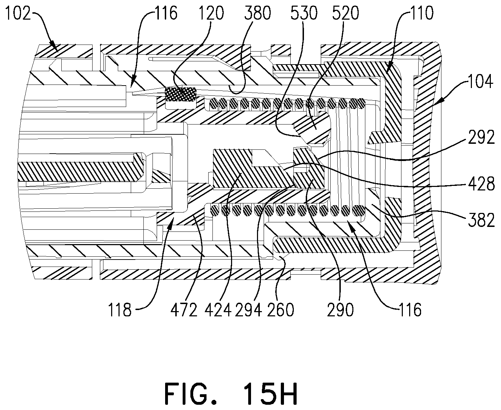

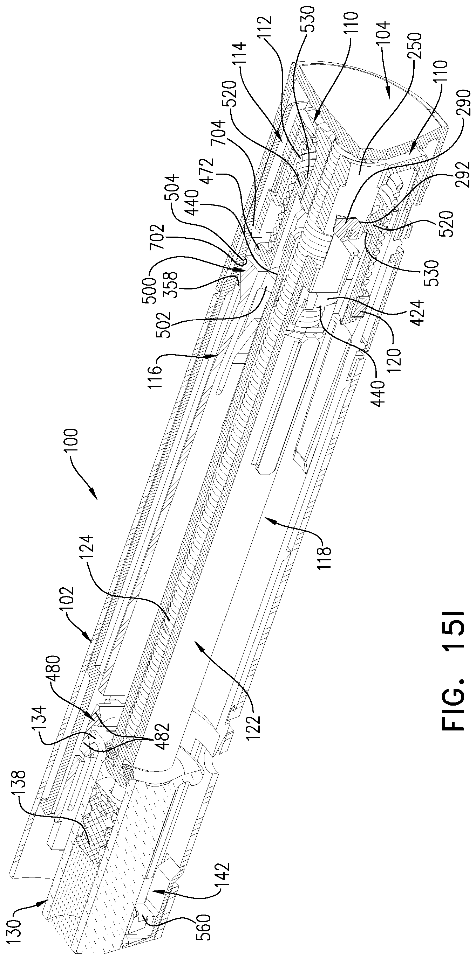

Figs. 15A, 15B, 15C, 15D, 15E, 15F, 15G, 15H and 15I are simplified drawings of the emergency automatic injection assembly of Figs. 1A - 11H in a second activation stage operative orientation, including a simplified perspective view, two simplified side plan views, five simplified sectional views taken along lines D - D in Fig. 15B, lines E - E in Fig. 15C, lines F - F and lines G - G in Fig. 15D, lines H - H in Fig. 15G and a partial section view taken along lines I - I in Fig. 15A, shown without the forward portion of the emergency automatic injection assembly;

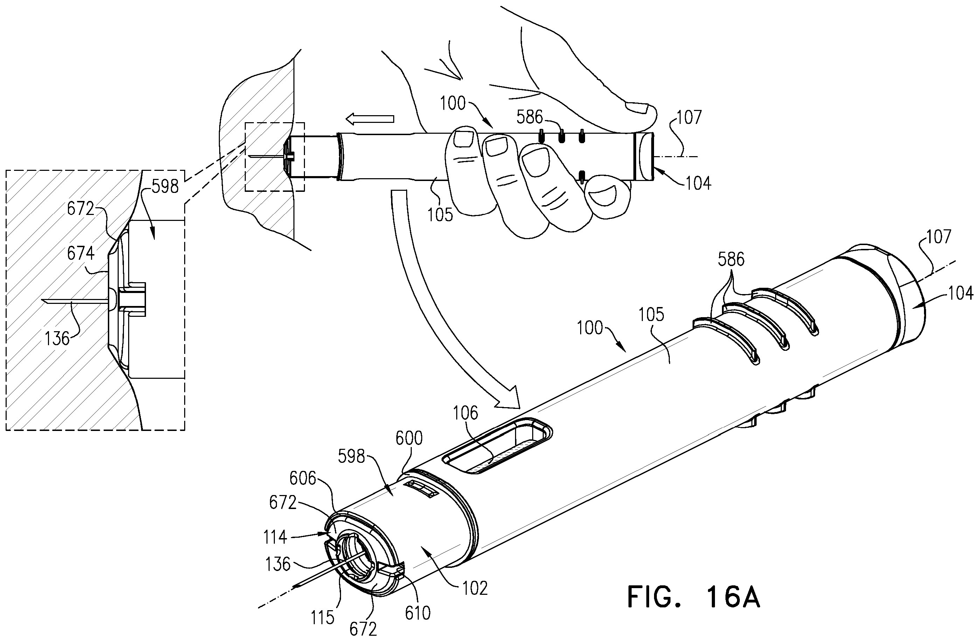

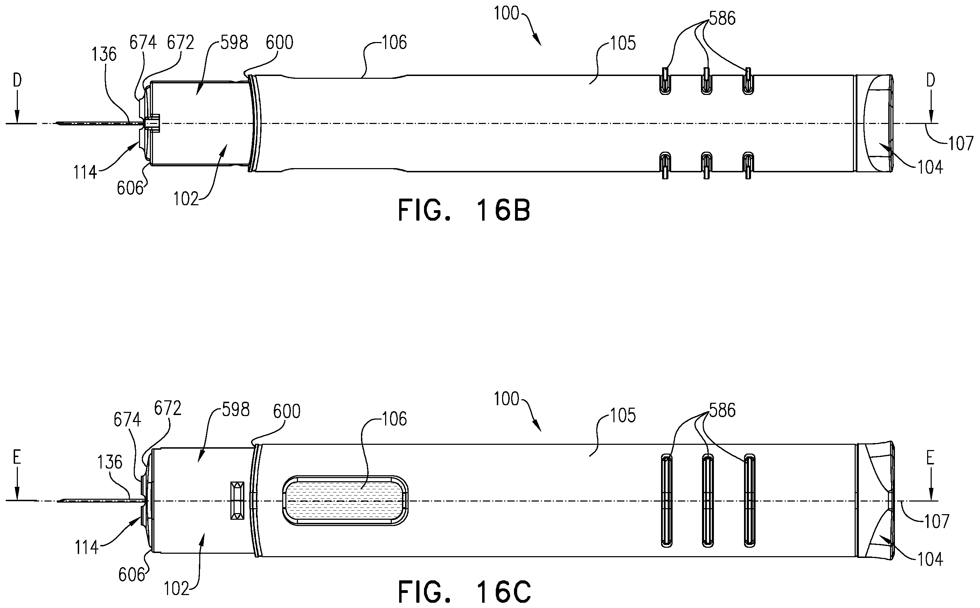

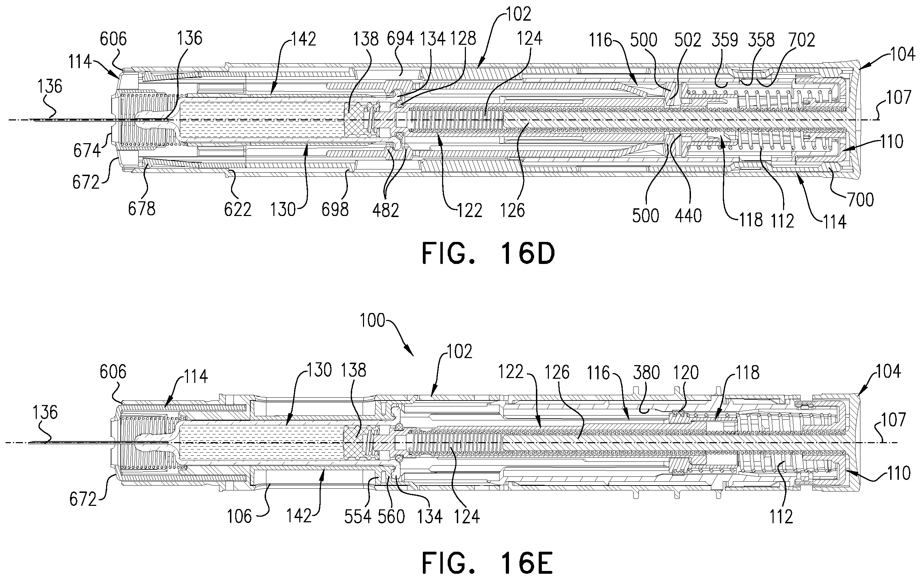

Figs. 16A, 16B, 16C, 16D and 16E are simplified drawings of the emergency automatic injection assembly of Figs. 1A - 11H in a first needle insertion stage operative orientation, including a simplified perspective view, two simplified side plan views, and two simplified sectional views taken along lines D - D in Fig. 16B and lines E - E in Fig. 16C;

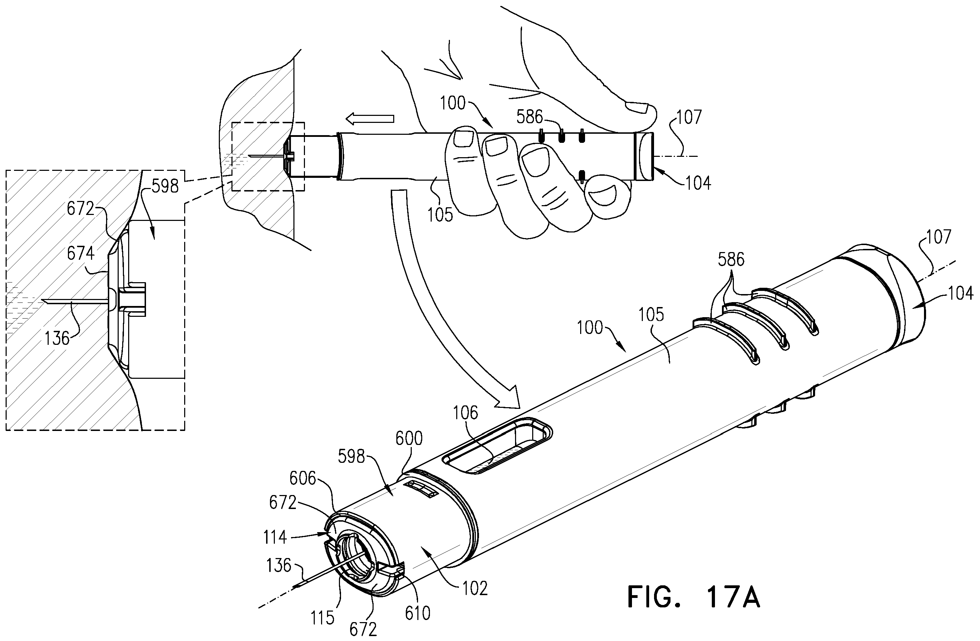

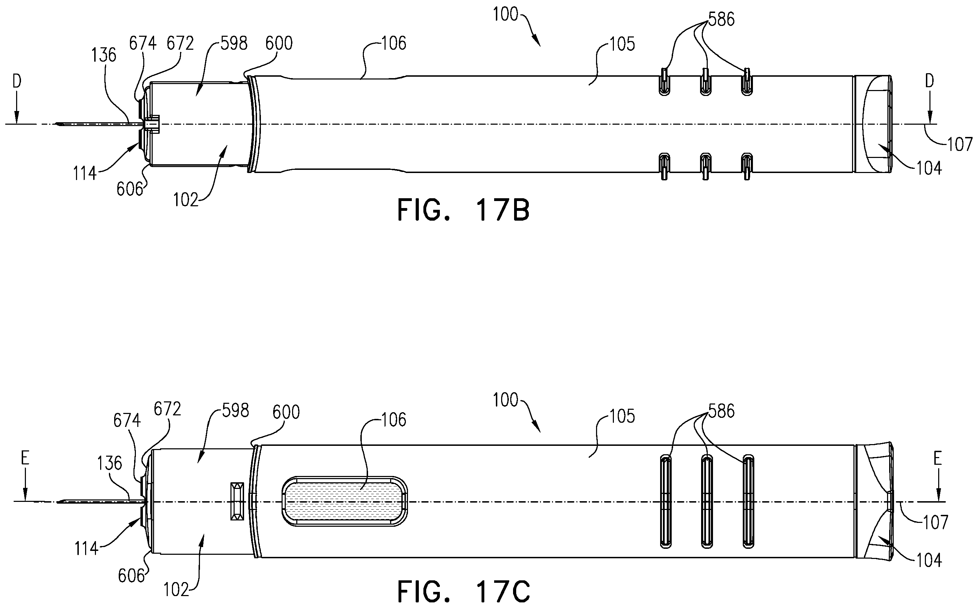

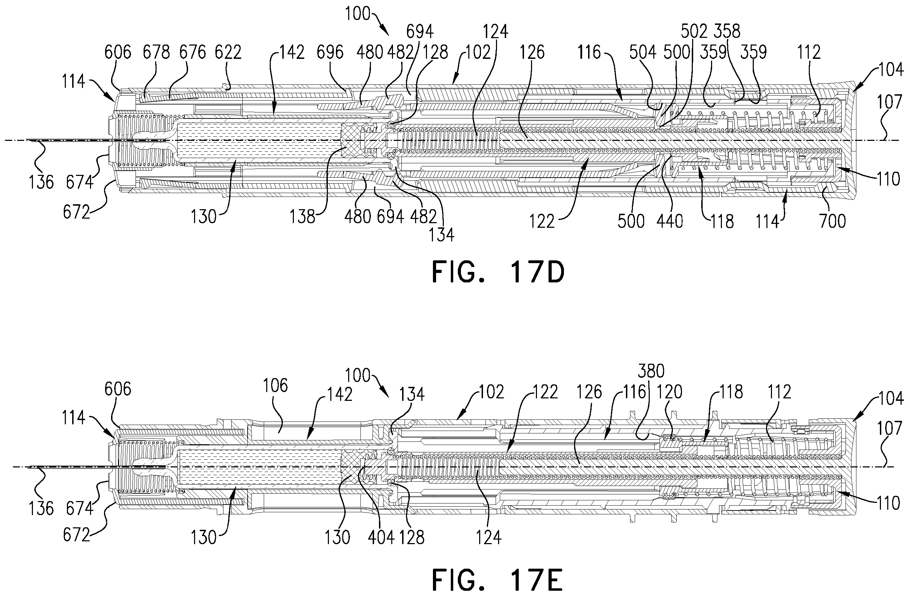

Figs. 17A, 17B, 17C, 17D and 17E are simplified drawings of the emergency automatic injection assembly of Figs. 1A - 11H in a second needle insertion stage operative orientation, including a simplified perspective view, two simplified side plan views, and two simplified sectional views taken along lines D - D in Fig. 17B and lines E - E in Fig. 17C;

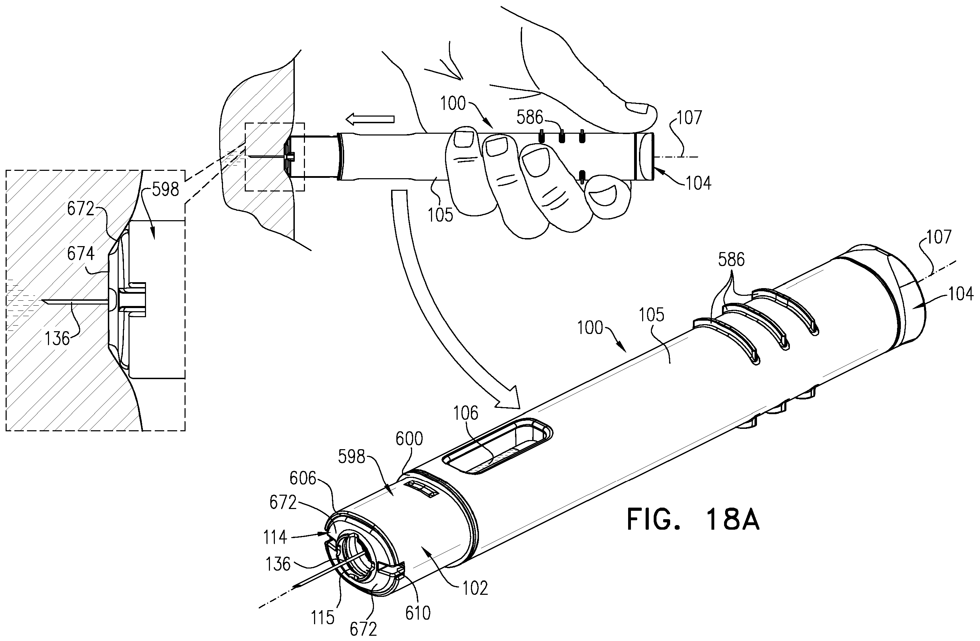

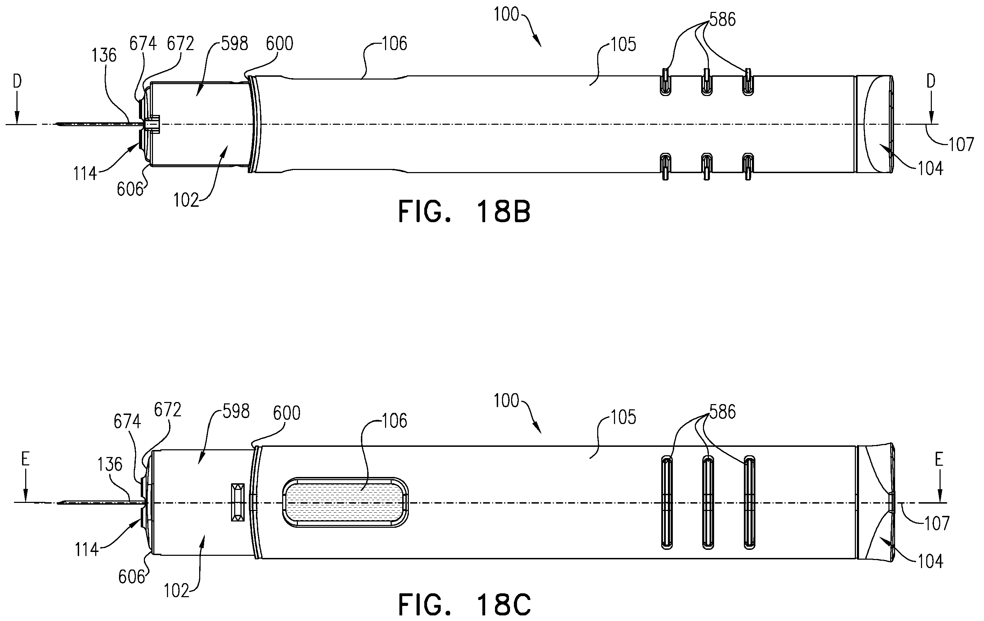

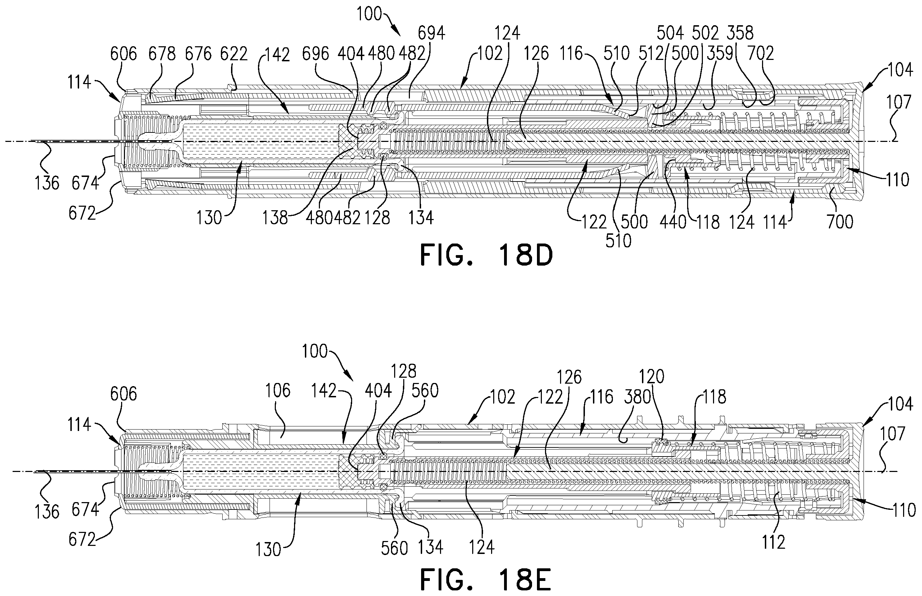

Figs. 18A, 18B, 18C, 18D and 18E are simplified drawings of the emergency automatic injection assembly of Figs. 1A - 11H in a third needle insertion stage operative orientation, including a simplified perspective view, two simplified side plan views, and two simplified sectional views taken along lines D - D in Fig. 18B and lines E - E in Fig. 18C;

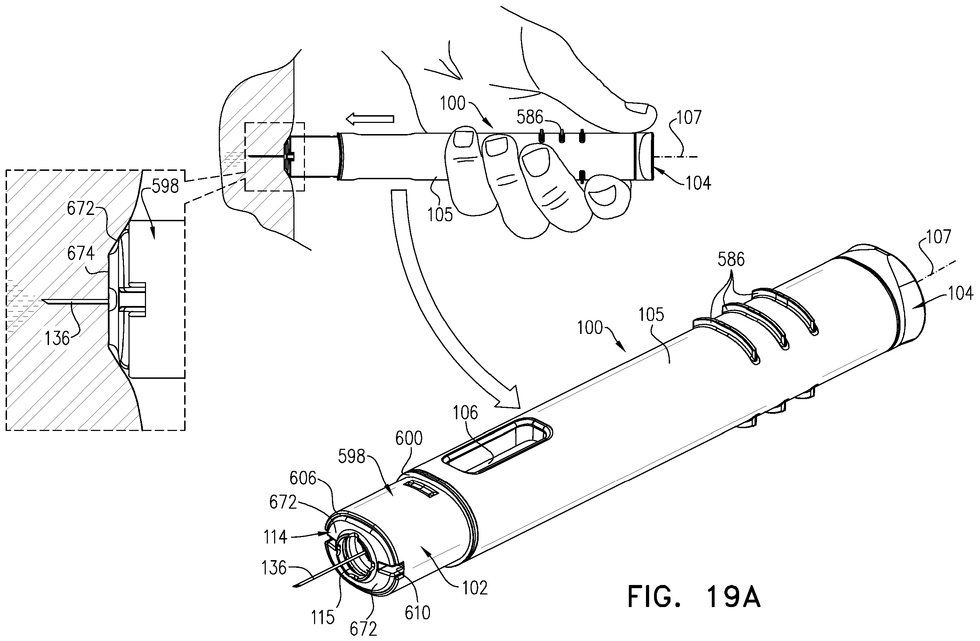

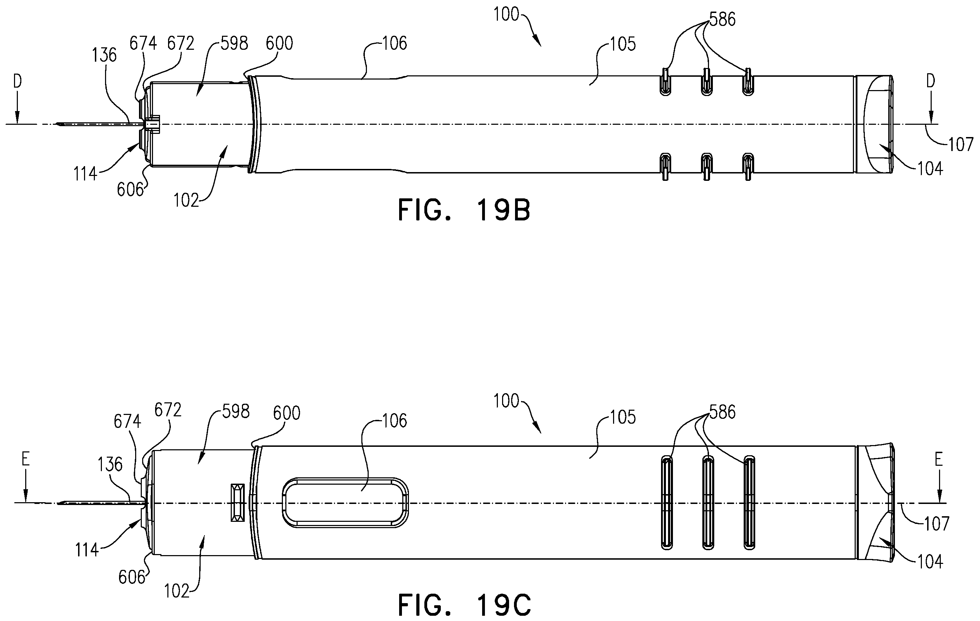

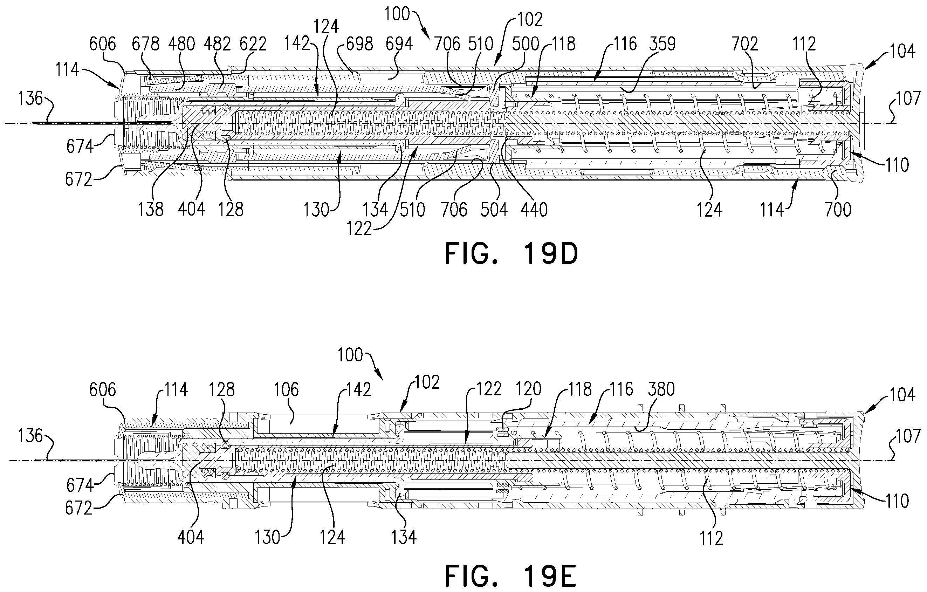

Figs. 19A, 19B, 19C, 19D and 19E are simplified drawings of the emergency automatic injection assembly of Figs. 1A - 11H in an end of delivery operative orientation, including a simplified perspective view, two simplified side plan views, and two simplified sectional views taken along lines D - D in Fig. 19B and lines E - E in Fig. 19C;

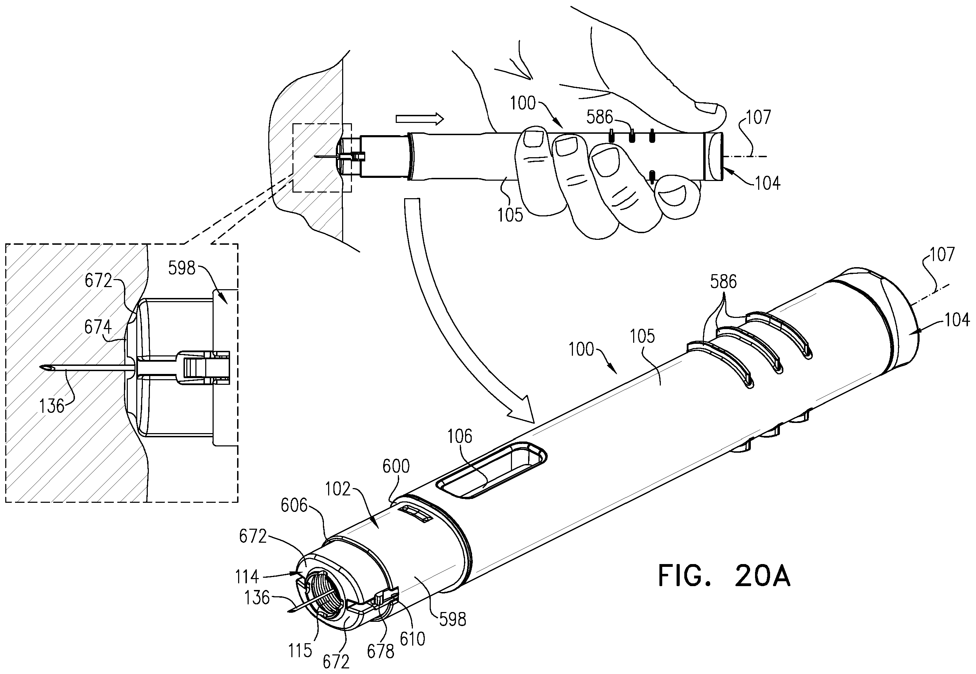

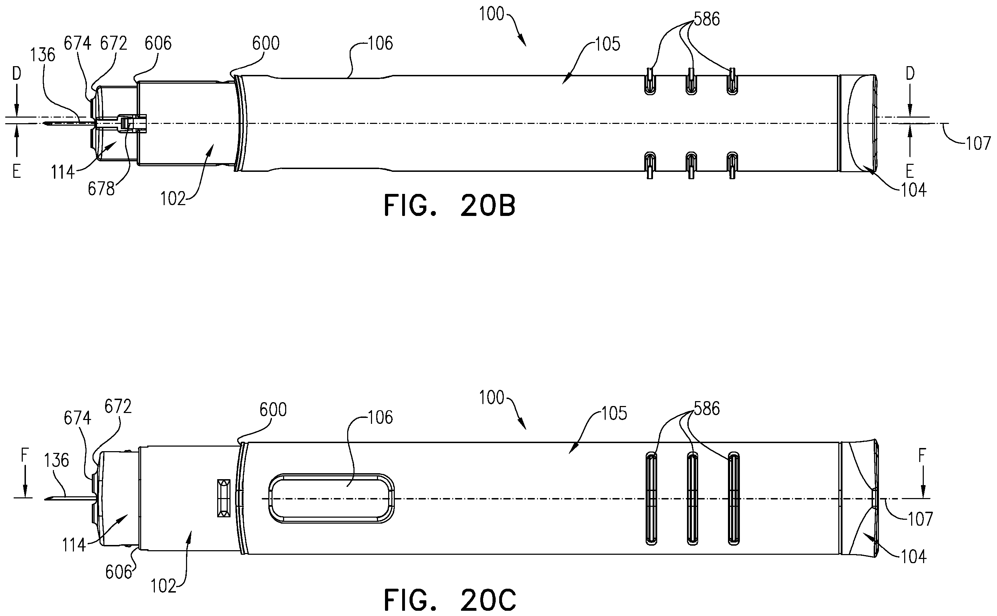

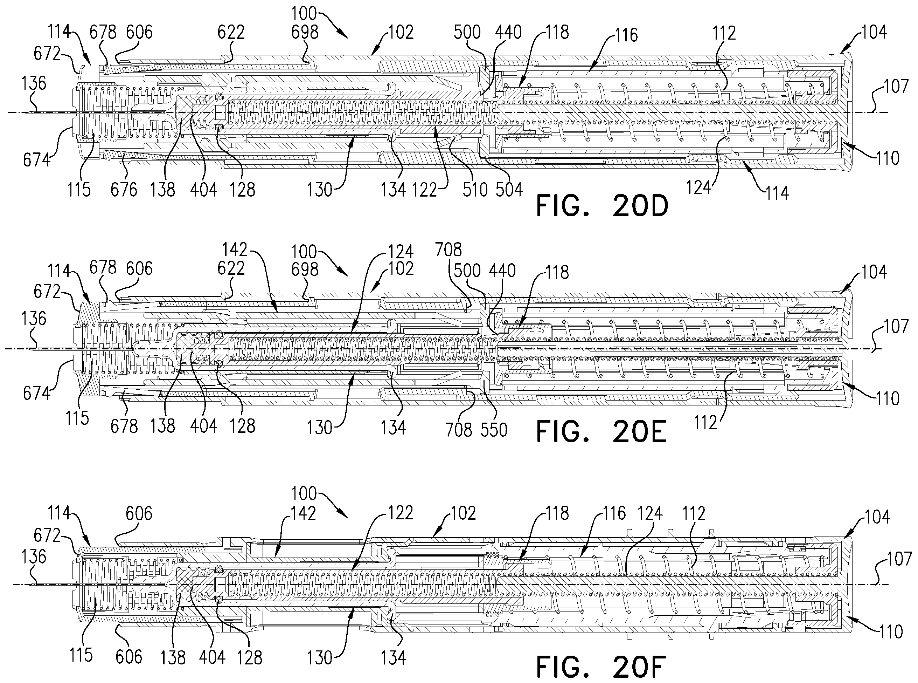

Figs. 20A, 20B, 20C, 20D, 20E and 20F are simplified drawings of the emergency automatic injection assembly of Figs. 1A - 11H in a removal from injection site operative orientation, including a simplified perspective view, two simplified side plan views, and three simplified sectional views taken along lines D - D and lines E - E in Fig. 20B and lines F - F in Fig. 20C;

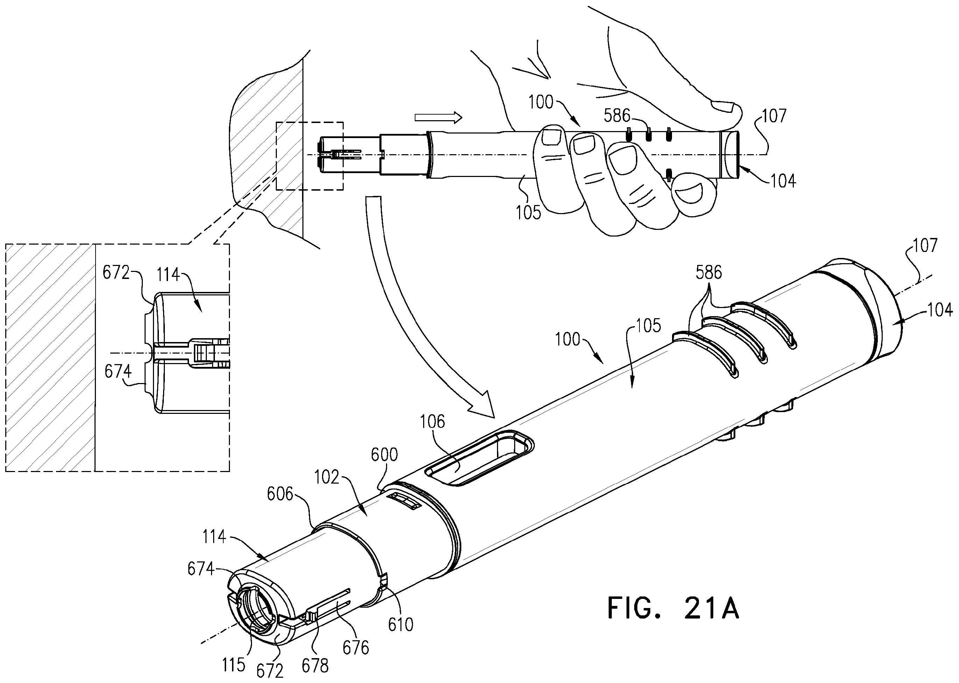

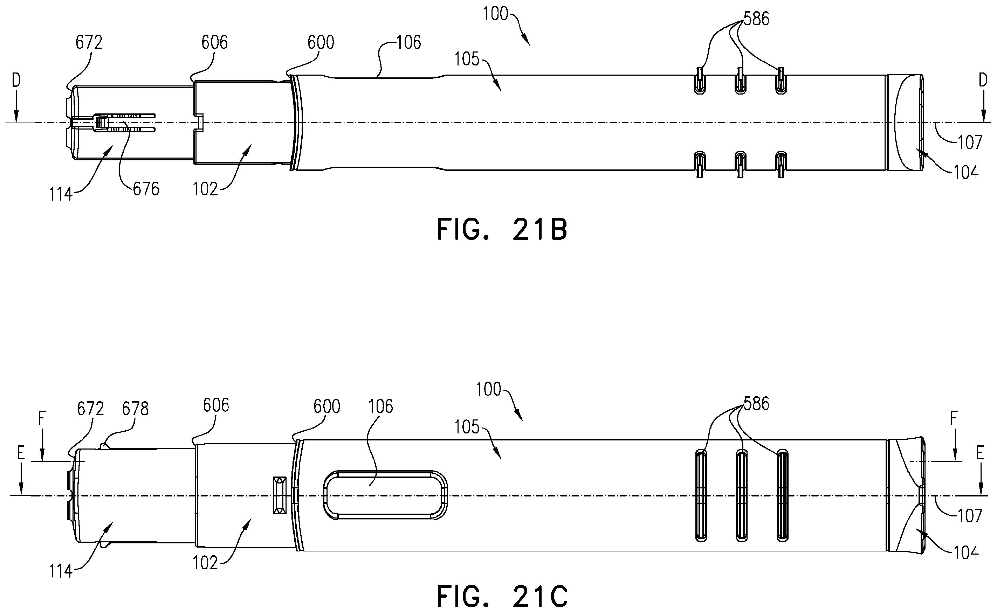

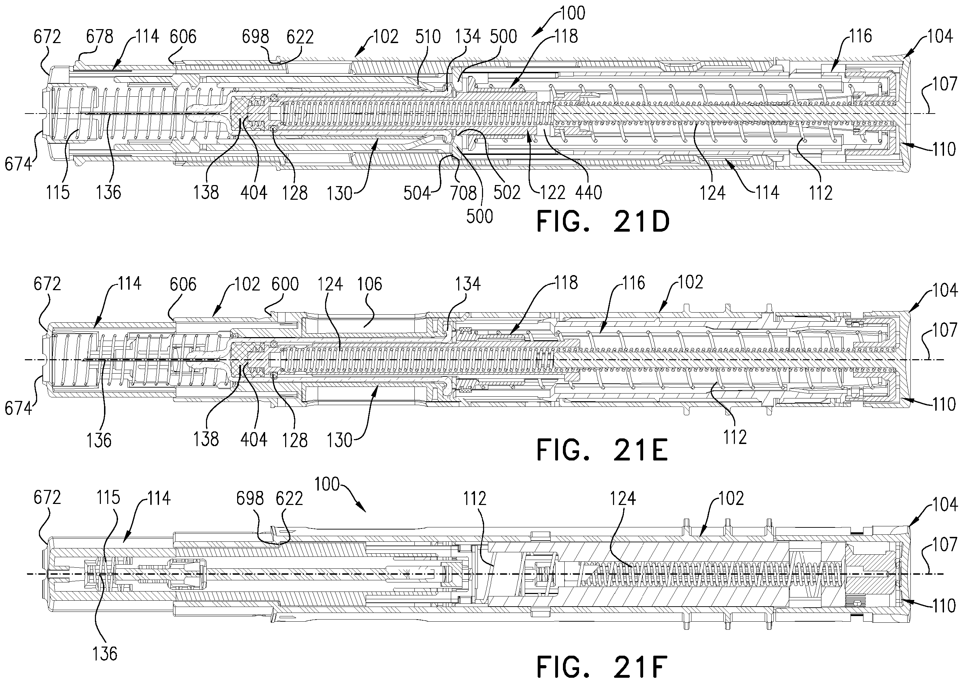

Figs. 21A, 21B, 21C, 21D, 21E and 21F are simplified drawings of the emergency automatic injection assembly of Figs. 1A - 11H in a first discard stage operative orientation, including a simplified perspective view, two simplified side plan views, and three simplified sectional views taken along lines D - D in Fig. 21B, lines E - E in Fig. 21C and lines F - F in Fig. 21D;

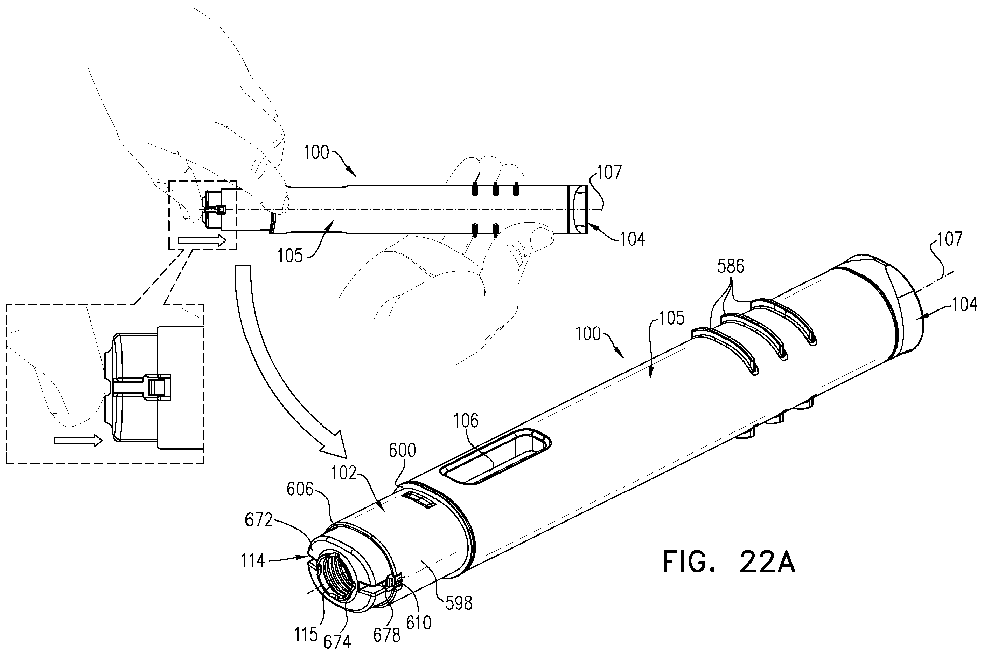

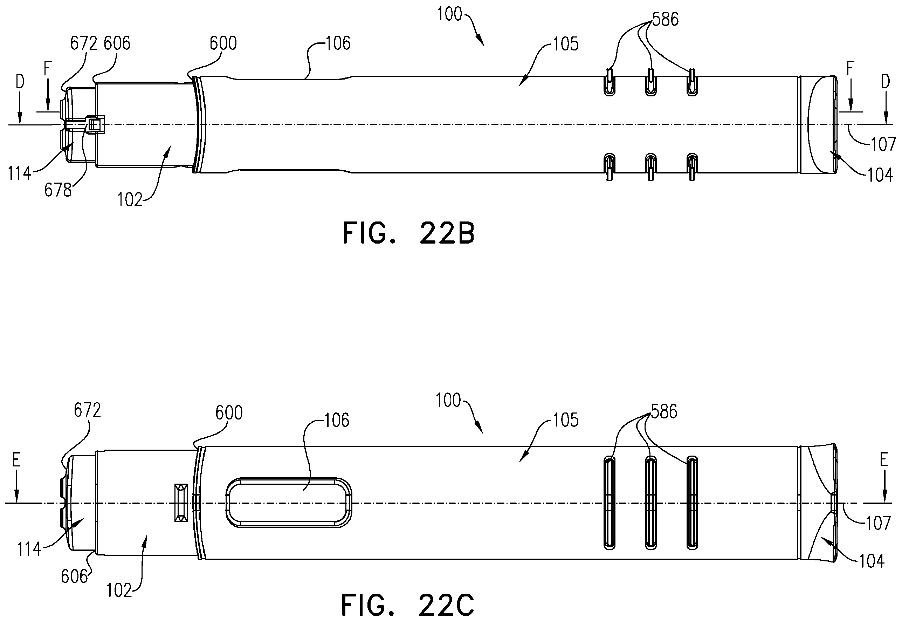

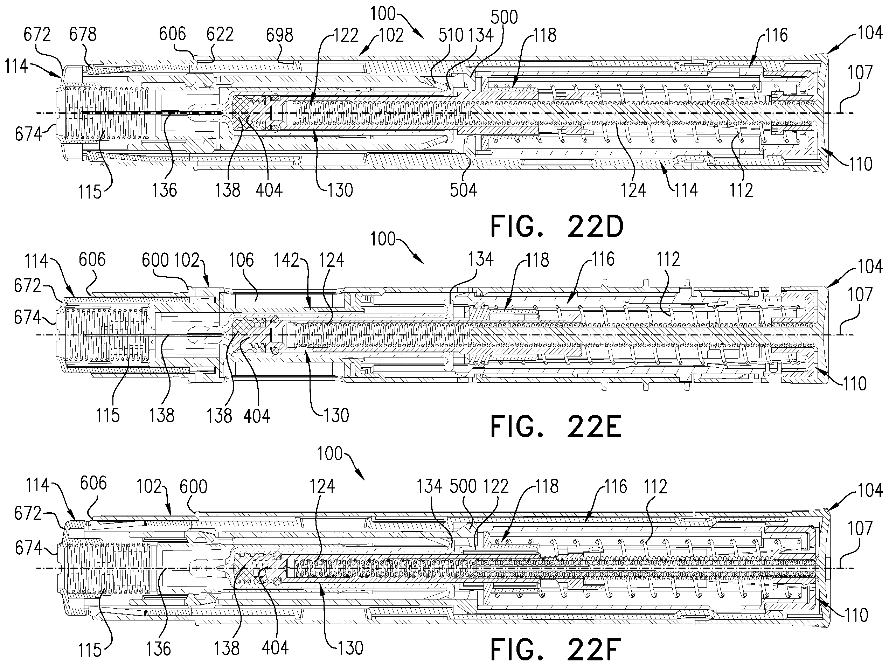

Figs. 22A, 22B, 22C, 22D, 22E and 22F are simplified drawings of the emergency automatic injection assembly of Figs. 1A - 11H in a second discard stage operative orientation, including a simplified perspective view, two simplified side plan views, and three simplified sectional views taken along lines D - D and lines F - F in Fig. 22B and lines E - E in Fig. 22C;

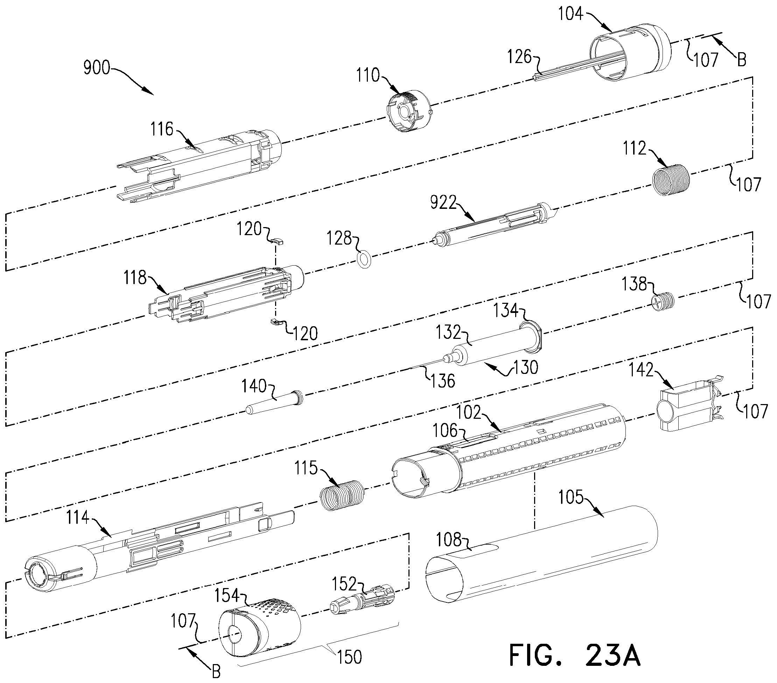

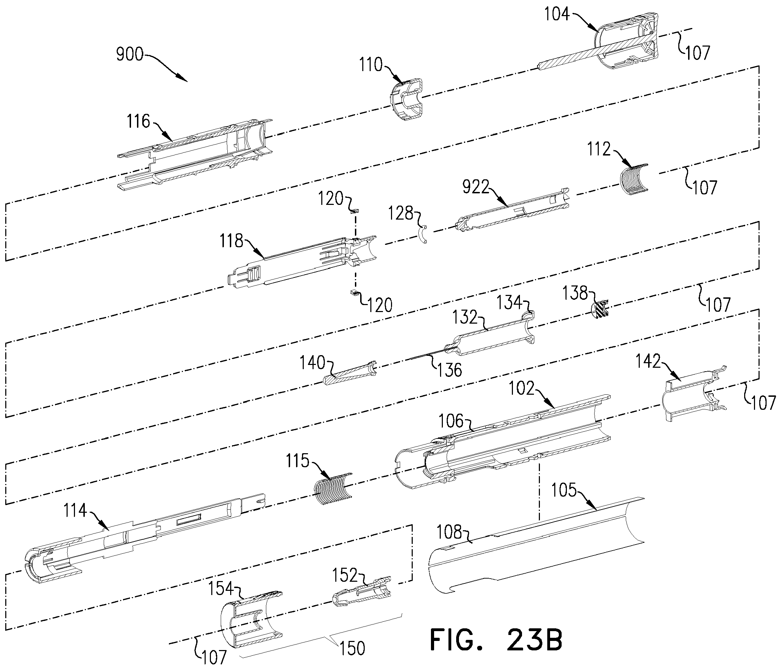

Figs. 23A and 23B are respectively simplified exploded view and sectional exploded view of an emergency automatic injection assembly constructed and operative in accordance with another embodiment of the present invention, the sectional view taken along lines B - B in Fig. 23A;

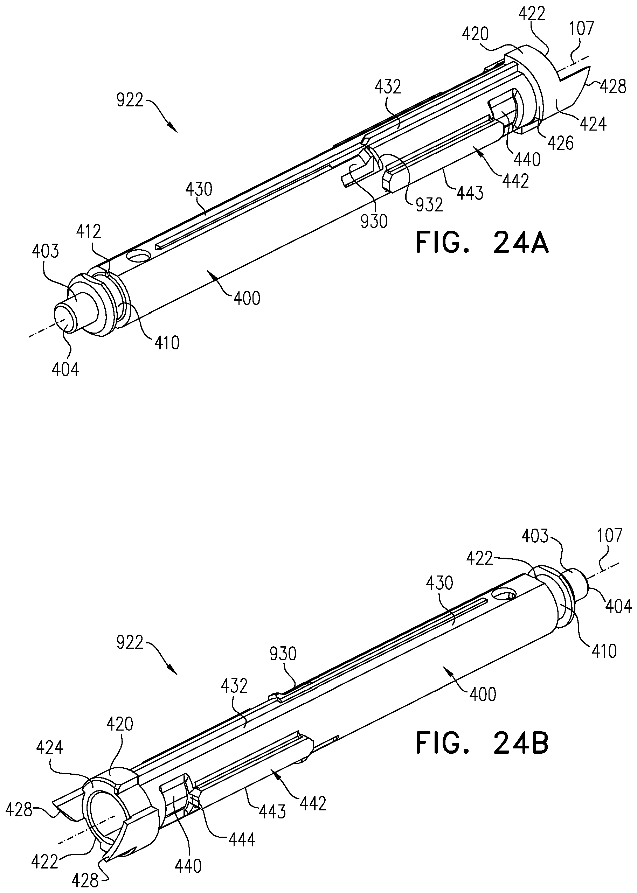

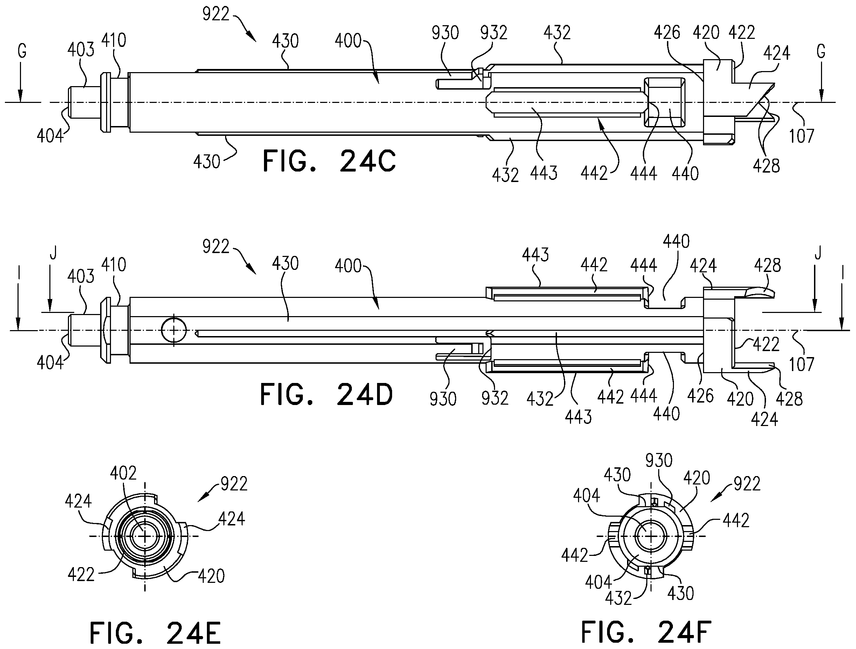

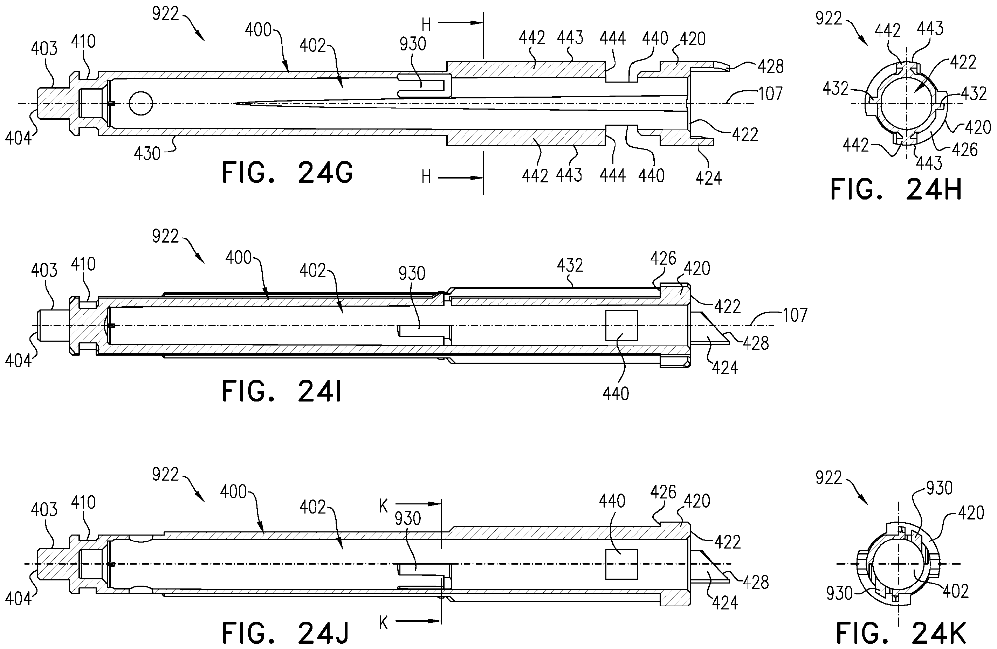

Figs. 24A, 24B, 24C, 24D, 24E, 24F, 24G, 24H, 24I & 24K are respectively two simplified perspective views, two simplified side plan views, a simplified top plan view, a simplified bottom plan view and five simplified sectional views taken along lines G - G in Fig. 24C, lines I - I in Fig. 24D, lines H - H and J - J in Fig. 24G and lines K - K in Fig. 24J of the plunger rod element forming part of the emergency automatic injection assembly of Figs. 23A & 23B;

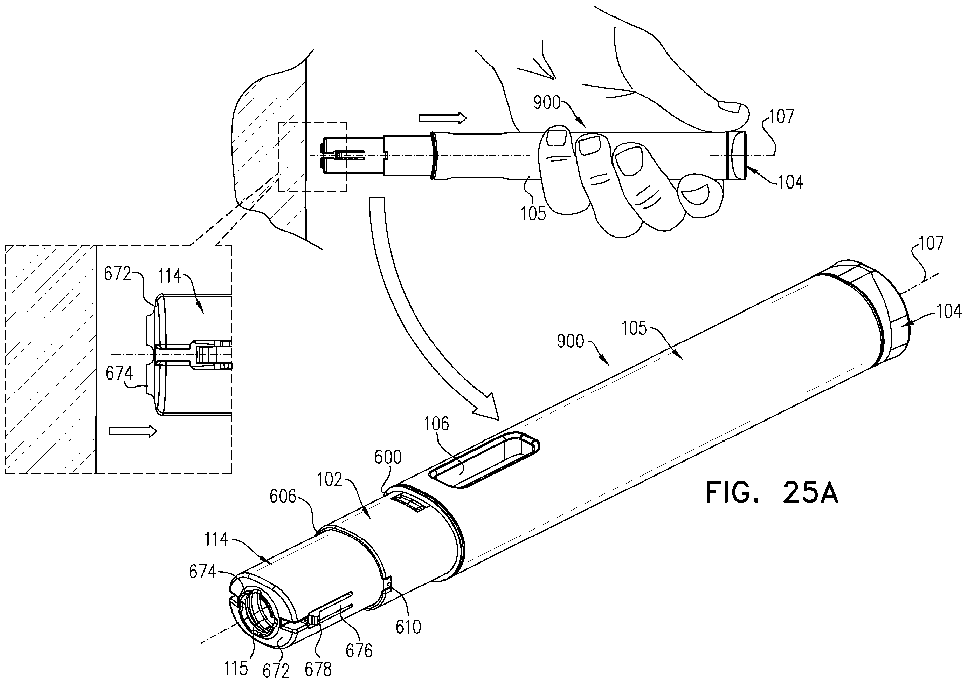

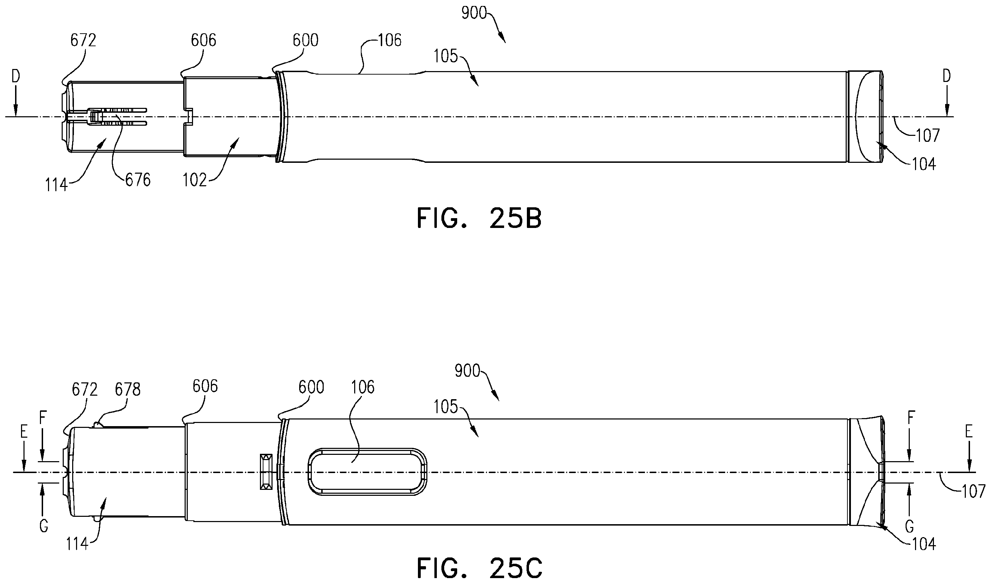

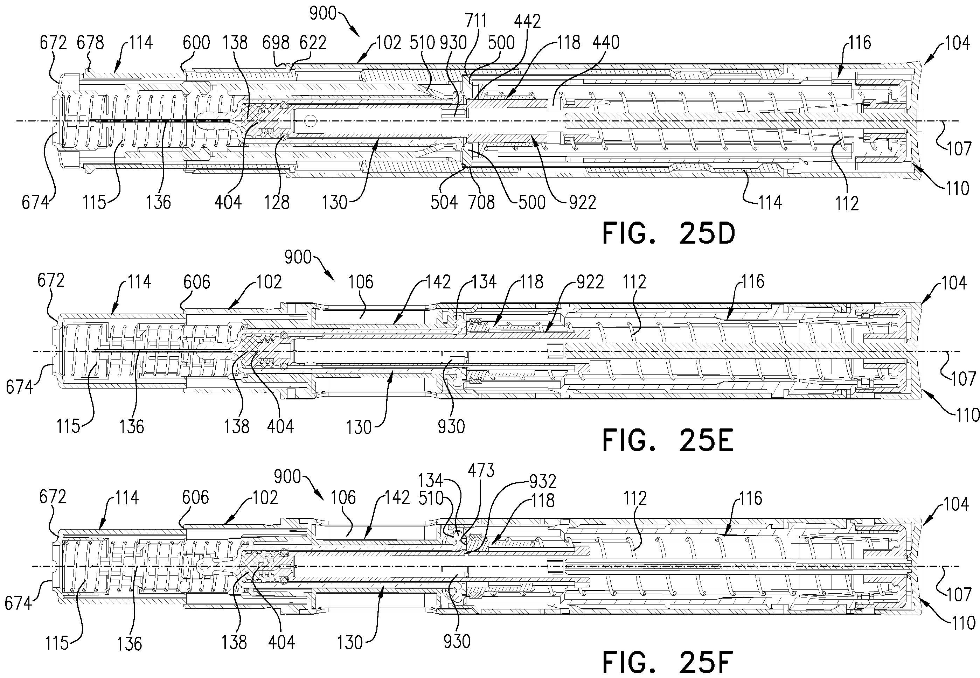

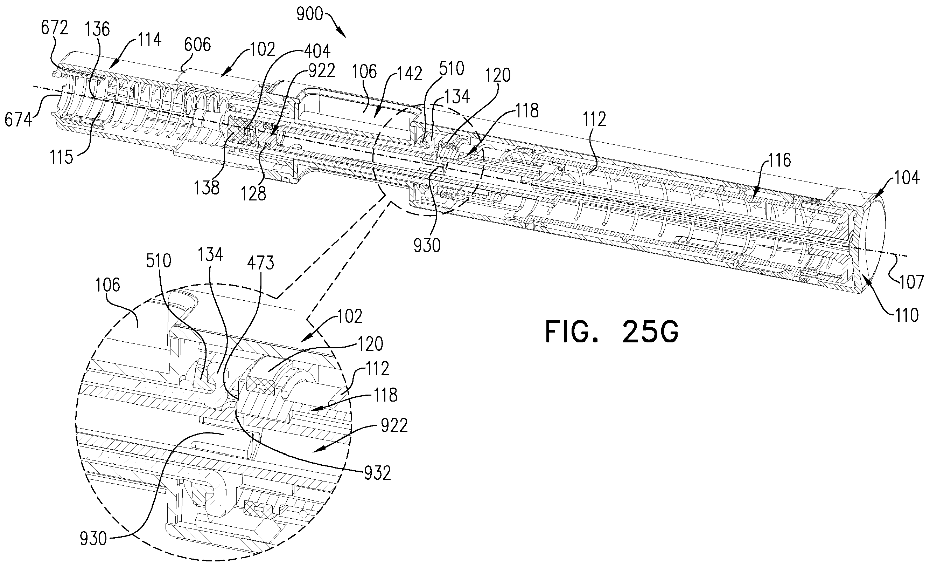

Figs. 25A, 25B, 25C, 25D, 25E, 25F and 25G are simplified drawings of the emergency automatic injection assembly of Figs. 23A and 23B in a first discard stage operative orientation, including a simplified perspective view, two simplified side plan views, and four simplified sectional views taken along lines D - D in Fig. 25B, lines E - E, lines F - F and lines G - G in Fig. 25C.

### DESCRIPTION OF EMBODIMENTS

The principles, uses and implementations of the teachings herein may be better understood with reference to the accompanying description and figures. Upon perusal of the description and figures present herein, one skilled in the art is able to implement the invention without undue effort or experimentation.

Before explaining at least one embodiment of the invention in detail, it is to be understood that the invention is not limited in its applications to the details of construction and the arrangement of the components set forth in the following description and/or illustrated in the drawings and/or the Examples. The invention can be implemented with other embodiments and can be practiced or carried out in various ways. It is also understood that the phraseology and terminology employed herein is for descriptive purpose and should not be regarded as limiting.

Some embodiments of the invention are described herein with reference to the accompanying figures. The description, together with the figures, makes apparent to a person having ordinary skill in the art how some embodiments of the invention may be practiced. The figures are for the purpose of illustrative discussion and no attempt is made to show structural details of an embodiment in more detail than is necessary for a fundamental understanding of the invention. For the sake of clarity, some objects depicted in the figures are not to scale.

Reference is now made to Figs. 1A and 1B, which are respectively simplified exploded view and sectional exploded view of an emergency automatic injection assembly 100 constructed and operative in accordance with an embodiment of the present invention, the sectional view taken along lines B - B in Fig. 1A.

As seen in Figs. 1A and 1B, the emergency automatic injection assembly 100 comprises a front housing element 102 and a rear end element 104, which are preferably fixedly attached, such as by snap-fit engagement. A label 105 is adapted to be mounted over the front housing element 102. It is noted that the front housing element 102 is formed with a window 106 and the label 105 has an opening 108, which is adapted to be aligned with the window 106 when the label is mounted onto the front housing element 102 to permit viewing of a portion of the contents of the emergency automatic injection assembly 100 therethrough. It is noted that the front housing element 102 and the rear end 104 are arranged along a mutual longitudinal axis 107.

Disposed within the enclosure formed by the rear end 104 and the front housing element 102 there is provided a locking element in the form of a locking ring 110, which is configured to be biased to rotate about longitudinal axis 107 under the force of a first injection spring 112, but is operatively blocked from rotation in certain operative orientations of the emergency automatic injection assembly 100 by engagement with a rear portion of a needle shield 114. The needle shield 114 is arranged along longitudinal axis 107 and is partially received into the front housing element 102 and extends forwardly so as to protrude forwardly from the front housing element 102. The needle shield 114 is operative to be biased forwardly under the force of a needle shield spring 115.

A rear housing element 116 is also arranged along the longitudinal axis 107 and a rear portion thereof is at least partially received within the locking ring 110. The rear housing element 116 is preferably contained within the needle shield 114.

A control unit 118 is arranged along the longitudinal axis 107 and a rear portion thereof is at least partially received within the rear portion of the rear housing element 116. The first injection spring 112 is disposed generally between the rear portion of the control unit 118 and the rear portion of the rear housing 116 and is adapted to act on the control unit 118 when released. It is noted that typically two resilient dampening elements 120 are mounted onto the rear portion of the control unit 118 for frictional engagement with an inner surface of the rear housing element 116.

It is further seen in Figs. 1A & 1B that a plunger rod 122 is generally enclosed within the control unit 118 and is configured to be restrained by the control unit 118 in a retracted rearward position. The plunger rod 122 is arranged along longitudinal axis 107 and a second injection spring 124, which is arranged in coaxial relationship with the first injection spring 112, is inserted into the interior volume defined by the plunger rod 122. The second injection spring 124 is supported and guided by a guiding shaft 126, which forms part of the rear end element 104. It is noted that the second injection spring 124 is configured to provide additional force for displacement of the plunger rod 122 along longitudinal axis 107. A forward dampening element 128 is preferably mounted onto a forward portion of the plunger rod 122.

A syringe 130 is configured to be held by the control unit 118 in certain operative orientations of the emergency automatic injection assembly 100. The pre-filled syringe 130 has a syringe barrel 132 having a flange 134 formed at its rearward end and a needle 136 fixedly attached to its forward end. A piston 138 is contained within the syringe barrel 132, which confines the medicament within the syringe barrel 132. A cover 140, suitable for single use, is adapted to seal and protect the needle 136. It is appreciated that syringe 130 can be any type of medicament container, such as pre-filled syringe or cartridge.

It is also noted that at least a portion of the syringe 130 is configured to reside within a syringe sleeve 142, which is preferably fixedly attached to the front housing element 102.

A needle shield remover in the form of a cover remover assembly 150 is adapted to be mounted over the forward portion of the front housing element 102 and of the needle shield 114 to protect the needle 136 in storage and permit removal of the cover 140 before injection.

It is seen in Figs. 1A & 1B that the cover remover assembly 150 comprises a floating cylinder 152 and a safety cap 154 at least partially disposed around it, both the floating cylinder 152 and the safety cap 154 being arranged along longitudinal axis 107. It is noted that the floating cylinder 152 is axially displaceable relative to the safety cap 154 along the longitudinal axis 107 to compensate for manufacturing tolerances of the various elements of the emergency automatic injection assembly 100.

Reference is now made to Figs. 2A, 2B, 2C, 2D, 2E, 2F, 2G & 2H are respectively a simplified perspective view of a forward-facing portion, a simplified perspective view of a rearward-facing portion, two simplified side plan views, a simplified top plan view, three simplified sectional views taken along lines F - F in Fig. 2D, lines G - G in Fig. 2F and lines H - H in Fig. 2F respectively of the rear end element 104 forming part of the emergency automatic injection assembly 100 of Figs. 1A & 1B.

The rear end element 104 preferably is an integrally formed element, preferably injection molded of plastic and is arranged along longitudinal axis of symmetry 107.

The rear end element 104 preferably includes a generally cylindrical base portion 200 defining a circumferential wall 202 and a rearwardly-facing base wall 204 having a forwardly-facing surface 206 from which extends the above-mentioned guiding shaft 126. The circumferential wall 202 extends forwardly to a forwardly-facing circumferential edge 208. The guiding shaft 126 extends forwardly of edge 208 along longitudinal axis 107.

Several openings 210 are provided on the circumferential wall 202. Typically, two snap portions 212 are provided on the circumferential wall 202 for attachment of the rear end element 104 with the rear housing element 116.

It is particularly seen in Figs. 2F & 2G that an annular protrusion 214 is provided around the rearward portion of the guiding shaft 126, which preferably serves as a spring seat for the second injection spring 124.

It is additionally seen in Fig. 2H that generally two diametrically opposed recesses 216 are formed on the inner surface of the circumferential wall 202 of the rear end 104. The recesses 216 permit passage of a portion of the needle shield 114 therethrough.

Reference is now made to Figs. 3A, 3B, 3C, 3D, 3E, 3F, 3G, 3H, 3I & 3J, which are respectively three simplified perspective views, two simplified side plan views, a simplified top plan view, a simplified bottom plan view and three simplified sectional views taken along lines H - H in Fig. 3D, lines I - I in Fig. 3G and lines J - J in Fig. 3H respectively of the locking ring element 110 forming part of the emergency automatic injection assembly 100 of Figs. 1A & 1B.

The locking ring 110 preferably is an integrally formed element, preferably injection molded of plastic and is arranged along longitudinal axis of symmetry 107.

The locking ring 110 preferably includes two concentric cylinders, an inner cylinder 250 and an outer cylinder 252 arranged along longitudinal axis 107 and connected by a rearward base wall 254.

Typically, two locking members 260 are formed on an outer surface of the outer cylinder 252, typically adjacent a forward end 262 thereof. The locking members 260 are preferably diametrically opposed to each other. It is specifically seen that the locking members 260 are preferably L-shaped, including a first portion 264 extending rearwardly from a location generally adjacent to the forward end 262 and a second portion 266 extending generally along the forward end 262. The second portion 266 has a rearwardly facing surface 268, adapted to engage a portion of the needle shield 114 and a forwardly facing surface 270, adapted to engage a portion of the rear housing 116.

It is additionally seen in Figs. 3A - 3J that typically two diametrically opposed stoppers 274 are formed generally adjacent a rearward end 276 of the outer cylinder 252. It is noted that each of the stoppers 274 is generally axially aligned with one of the locking members 260. Each of the stoppers 274 includes a generally rearwardly tapered surface 278.

A central bore 280 extends through the inner cylinder 250 and serves for passage of the second injection spring 124 therethrough.

It is seen in Figs. 3A - 3J that typically two generally diametrically opposed rotation-enabling elements 290 are formed on an outer surface of the inner cylinder 250 and protrude slightly radially outwardly therefrom. Each of the rotation-enabling elements 290 has a generally forwardly tapered surface 292, which is adapted to operatively engage a portion of the control unit 118 in certain operative orientations of the emergency automatic injection assembly 100 and a generally rearwardly tapered surface 294 on the opposite side thereof, which is adapted to operatively engage a portion of a plunger rod 122 in certain operative orientations of the emergency automatic injection assembly 100.

It is additionally seen in Figs. 3A - 3J that typically four pairs of diametrically opposed recesses 296, 298, 300 and 302 are formed on an inner surface of the outer cylinder 252, which are adapted to engage a portion of the rear housing element 116 in various operative orientations of the emergency automatic injection assembly 100.

An inner generally annular volume 304 is defined between the outer surface of the inner cylinder 250 and the inner surface of the outer cylinder 252.

Reference is now made to Figs. 4A, 4B, 4C, 4D, 4E, 4F, 4G, 4H & 4I, which are respectively two simplified perspective views, two simplified side plan views, a simplified top plan view, and four simplified sectional views taken along lines F - F in Fig. 4C, lines G - G in Fig. 4F, lines H - H in Fig. 4F and lines I - I in Fig. 4G respectively of the rear housing element 116 forming part of the emergency automatic injection assembly 100 of Figs. 1A & 1B.

The rear housing element 116 preferably is an integrally formed element, preferably injection molded of plastic and is arranged along longitudinal axis of symmetry 107.

The rear housing element 116 preferably includes a generally cylindrical rear portion 330 and a generally rounded rectangular front portion 332 extending forwardly from rear portion 330 and forming a rearwardly facing shoulder 334 therebetween.

A pair of generally diametrically opposed snap portions 340 are formed on the cylindrical rear portion 330 and are configured for operatively engaging one pair of recesses 296, 298, 300 or 302 of the locking ring 110 in various operative orientations of the emergency automatic injection assembly100.

The front portion 332 of the rear housing element 116 preferably includes two generally flat side walls 350 and two generally curved top and bottom walls 352. Each of the two side walls 350 preferably includes a guiding portion 354 bounded by two side ribs 356, formed adjacent the rearwardly facing shoulder 334, the guiding portion 354 serves to guide the displacement of the needle shield 114.

An opening 357 is formed on each of the side walls 350, the openings 357 are disposed forwardly of guiding portions 354 and define a rearwardly facing edge 358, which is adapted to engage a portion of the control unit element 118 in certain operative orientations of the emergency automatic injection device 100. The internal surface of the rear housing element 116 disposed forwardly of openings 357 defines a cylindrical circumferential surface 359.

Each of the two top and bottom walls 352 preferably includes a protrusion 360, formed adjacent the rearwardly facing shoulder 334, the protrusion 360 serves for engagement with the rear end element 104.

It is particularly seen in Figs. 4A - 4G that top and bottom walls 352 each terminate at a pair of radially spaced longitudinal arms 362 at the forward end thereof. A gap 364 is defined on side walls 350 between the two pairs of arms 362. The gap 364 on side walls 350 is bounded on the rearward end thereof by an edge 366, which engages a portion of the needle shield 114 in certain operative orientations of the emergency automatic injection assembly 100. Arms 362 preferably serve for centering of the syringe 130.

It is additionally seen in Figs. 4A - 4I that a protrusion 370 is formed at the rearward end of arms 362 for operative engagement with the front housing element 102.

Referring specifically to Fig. 4G, it is seen that an inwardly protruding frictional surface 380 is formed on an inner surface of each of the top and bottom arms 352, for operative engagement with dampening elements 120 in use. It is also seen in Figs. 4G and 4I that an inwardly extending flange 382 is formed on a rearward end of the cylindrical rear portion 330. Flange 382 defines a forwardly facing surface 384, which serves as a spring seat for the first injection spring 112.

Reference is now made to Figs. 5A, 5B, 5C, 5D, 5E, 5F, 5G, 5H & 5I, which are respectively two simplified perspective views, two simplified side plan views, a simplified top plan view, a simplified bottom plan view and three simplified sectional views taken along lines G - G in Fig. 5C, lines H - H and lines I - I in Fig. 5G of the plunger rod element 122 forming part of the emergency automatic injection assembly 100 of Figs. 1A & 1B.

The plunger rod element 122 preferably is an integrally formed element, preferably injection molded of plastic and is arranged along longitudinal axis of symmetry 107.

The plunger rod element 122 preferably includes a generally hollow cylindrical shaft 400 arranged along longitudinal axis 107 and defining an interior bore 402. The cylindrical shaft 400 has a protrusion 403, extending axially forwardly from a forward end of shaft 400 and defining a piston engaging wall 404 formed at the forward end thereof. The piston engaging wall 404 is generally disposed transversely to longitudinal axis 107.

It is seen in Figs. 5A - 5I that a circumferential recess 410 is formed generally adjacent to and rearwardly spaced from the protrusion 403. The recess 410 serves as a seat for the forward dampening element 128, which serves in use so as to dampen the plunger rod 122 displacement within syringe 130. A small air passage opening 412 is formed on an edge of recess 410. The function of this forward dampening element 128 in conjunction with the air passage opening 412 is described in detail in publication US Publication US20190275251A1, for example with reference to the improved plunger and damper assembly 3160.

A generally annular widened flange 420 is formed on the rearward end of the cylindrical shaft 400. The flange 420 has a rearwardly facing end surface 422, having several rearwardly extending protrusions 424 formed thereon, for operative engagement with a portion of the locking ring 110. The flange 420 also has a forwardly facing shoulder 426, which is operative for engagement with the control unit 118. The rearwardly extending protrusions 424, each define a forwardly tapered surface 428.

The cylindrical shaft 400 typically includes a pair of diametrically opposed generally longitudinal flat portions 430 formed on the circumference thereof. A longitudinal guiding rib 432 is formed on each of the flat portions 430 for guiding of the plunger rod 122 within the control unit 118.

There are typically two inwardly extending openings 440, each formed in between the two guiding ribs 432, and the two openings are preferably diametrically opposed to each other. The openings 440 are preferably disposed adjacent the widened flange 420. A longitudinal rib 442 extends longitudinally forwardly from each of openings 440, forming a rearwardly facing shoulder 444 between the opening 440 and the rib 442. It is noted that the openings 440 and rearwardly facing shoulders 444 associated therewith are configured for operative engagement with a portion of the control unit 118 in certain operative orientations of the emergency automatic injection assembly 100. The ribs 442 are configured for operative engagement with a portion of the control unit 118 in other operative orientations.

Reference is now made to Figs. 6A, 6B, 6C, 6D, 6E, 6F, 6G, 6H and 6I, which are respectively two simplified perspective views, two simplified side plan views, a simplified top plan view, a simplified bottom plan view and three simplified sectional views taken along lines G - G in Fig. 6C, lines H - H and lines I - I in Fig. 6G of the control unit element 118 forming part of the emergency automatic injection assembly 100 of Figs. 1A & 1B.

The control unit element 118 preferably is an integrally formed element, preferably injection molded of plastic and is arranged along longitudinal axis of symmetry 107, having typically two dampening elements 120 mounted thereon.

The control unit element 118 preferably includes a rear cylindrical portion 470, a generally rectangular base portion 472 disposed at a forward end thereof and defining a forwardly facing shoulder 473. Typically, two opposed longitudinal arms 474 extend forwardly from one side of the forwardly facing shoulder 473 of the base portion 472 to a forward edge 476. The base portion 472 also defines a rearwardly facing shoulder 478.

A syringe holding snap portion 480 is formed on each of arms 474 and is disposed generally adjacent each of the forward edges 476. The syringe holding snaps 480 preferably include a pair of inwardly protruding longitudinally spaced portions 482 adapted for operative engagement with the flange 134 of syringe 130 in certain operative orientations of the emergency automatic injection assembly 100. The syringe holding snaps 480 also preferably include an outwardly extending protrusion 484. It is noted that the syringe holding snap portion 480 is preferably selectively deflectable outwardly.

It is also seen in Figs. 6A - 6I that a hammer snap portion 500 is formed on each of arms 474 and is disposed generally adjacent the base portion 472. The hammer snap portions 500 preferably include an inwardly protruding portion 502 adapted for operative engagement with the plunger rod 122 in certain operative orientations of the emergency automatic injection assembly 100. It is noted that the hammer snap portion 500 is preferably selectively deflectable inwardly. An outwardly extending protrusion 504 is formed on an outer surface of the hammer snap portion 500 and defines a forwardly facing surface 506, which is operative to engage a portion of the needle shield 114 in certain operative orientations of the emergency automatic injection assembly 100.

An additional discard tooth snap portion 510 is formed on each of arms 474 and is slightly spaced forwardly from the hammer snap portion 500. The discard tooth snap portions 510 preferably include an inwardly protruding portion 512 adapted for operative engagement with the flange 134 of the syringe 130 in certain operative orientations of the emergency automatic injection assembly 100. It is noted that the discard tooth snap portion 510 is preferably selectively deflectable outwardly.

The control unit element 118 is similar to the driving assembly 30 as described in US publication US8376998B2 with respect to the aspects described hereinabove.

It is a particular feature of an embodiment of the present invention that an inwardly extending rotation-enabling protrusion 520 is formed on an inner surface of the rear cylindrical portion 470 of the control unit 118. The rotation-enabling protrusion 520 has a forwardly-facing tapered surface 530, which is configured for operative engagement with a portion of the locking ring 110 in certain operative orientations of the emergency automatic injection assembly 100.

Reference is now made to Figs. 7A, 7B, 7C, 7D, 7E, 7F, 7G, 7H and 7I, which are respectively two simplified perspective views, two simplified side plan views, a simplified top plan view, a simplified bottom plan view and three simplified sectional views taken along lines G - G in Fig. 7C, lines H - H and lines I - I in Fig. 7G of the syringe sleeve element 142 forming part of the emergency automatic injection assembly 100 of Figs. 1A & 1B.

The syringe sleeve element 142 preferably is an integrally formed element, preferably injection molded of plastic and is arranged along longitudinal axis of symmetry 107.

The syringe sleeve element 142 is preferably fixedly attached to the front housing element 106 and is configured for at least partially containing the syringe 130 and additionally for dampening, i.e. reducing the impact on the flange 134 of the syringe 130 during axial displacement of the syringe 130.

Syringe sleeve element 142 includes a container having a hollow cylindrical portion 550 for receiving at least a portion of the syringe 130 therein. A longitudinal window 552 is formed on each side of the cylindrical portion 550 and extends radially outwardly therefrom. The cylindrical portion 550 defines a rearwardly facing end edge 554.

Typically, two attachment portions 556 extend rearwardly from each side of the syringe sleeve element 142 and adapted for securing the syringe sleeve element 142 with respect to front housing element 102.

Formed on each of the attachment portions 556 is a double-sided dampening beam 560. Each of the double sided dampening beams 560 is attached to its respective attachment portion 556 and extends inwardly therefrom. The double-sided beams 560 are axially rearwardly spaced from rearwardly facing end edge 554 and are thus configured to be slightly forwardly deflected upon exertion of impact thereon. The double-sided beams 560 each defines a rearwardly facing surface 562 for operative engagement with the flange 134 of the syringe 130 in certain operative orientations of the emergency automatic injection assembly 100. The two double-sided beams 560 preferably form together a circumferential or nearly circumferential support for the flange 134 of syringe 130.

Reference is now made to Figs. 8A, 8B, 8C, 8D, 8E, 8F, 8G, 8H, 8I and 8J, which are respectively two simplified perspective views, two simplified side plan views, a simplified top plan view, a simplified bottom plan view and four simplified sectional views taken along lines G - G in Fig. 8C, lines H - H and lines I - I in Fig. 8G and lines J - J in Fig. 8H of the front housing element 102 forming part of the emergency automatic injection assembly 100 of Figs. 1A & 1B.

The front housing element 102 preferably is an integrally formed element, preferably injection molded of plastic and is arranged along longitudinal axis of symmetry 107.

The front housing element 102 preferably defines an outer surface 580, having a generally convex cross section, and an inner surface 582 preferably including several axially extending mutually radially spaced elongate ribs 584 adapted to guide and align the needle shield element 114.

Several gripping protrusions 586 are preferably provided on the outer surface 580 of the front housing element 102. It is a particular feature of an embodiment of the present invention that providing a different amount of protrusions 586 on the outer surface 580 of the front housing element 102 allows for facilitating tactile indication for the user for the type of medication contained within the emergency automatic injection assembly 100, even if injection has to be performed in darkness, for example.

An attachment portion 588 is formed generally at an intermediate location of the front housing element 102 and adapted for attachment thereof with the rear housing element 116. A second attachment portion 590 is formed on the front housing element 102 and generally spaced forwardly from attachment portion 588, configured for attachment of the front housing element 102 with the syringe sleeve element 142.

The front housing portion 102 includes a main elongate portion 596 and a generally narrower elliptical forward portion 598 extending forwardly therefrom and defining a forwardly facing shoulder 600 therebetween. A pair of diametrically opposed recesses 602 are generally formed on each side of the forward portion 598, generally adjacent shoulder 600 and are configured for removable attachment of the safety cap 154 to the front housing portion 102.

The forward portion 598 defines a forwardly facing end 606. Typically, two recesses 608 extend rearwardly from forwardly facing end 606 and are diametrically opposed to each other. Tapered surface 610 is formed on a forward edge of each of the recesses 608 and configured for operative engagement with the needle shield 114 in certain operative orientations of the emergency automatic injection assembly 100.

It is specifically seen in Figs. 8G & 8H that a generally annular flange 620 is formed within the inner volume of the front housing element 102 and extends transversely and generally inwardly from the inner surface 588 thereof. The flange 620 is generally slightly rearwardly spaced from shoulder 600 and defines a rearwardly facing shoulder 622, which is configured to operatively engage a portion of the needle shield 114 in certain operative orientations of the emergency automatic injection assembly 100.

A generally hollow cylindrical protrusion 626 extends axially longitudinally forwardly from flange 620 and defines a forwardly facing spring seat surface 628 adapted for supporting the needle shield spring 115. It is noted that the inner surface of cylindrical protrusion 626 is adapted for supporting and aligning the syringe 130.

It is noted that due to the fact that the cylindrical protrusion 626 has a generally circular cross-section, the cover remover assembly 150 is not required to be oriented in any particular manner, rather it can be mounted onto the front housing element 102 at any rotational orientation.

Reference is now made to Figs. 9A, 9B, 9C, 9D, 9E, 9F, 9G, 9H, 9I and 9K, which are respectively two simplified perspective views, two simplified side plan views, a simplified top plan view, and five simplified sectional views taken along lines H - H in Fig. 9C, lines F - F in Fig. 9D, lines G - G and lines J - J in Fig. 9H and lines I - I in Fig. 9J of the needle shield element 114 forming part of the emergency automatic injection assembly 100 of Figs. 1A & 1B.

The needle shield element 114 preferably is an integrally formed element, preferably injection molded of plastic and preferably has a generally cylindrical configuration including a generally tubular portion 670, having a forward-facing body engaging surface 672 including a generally annular ribbed protrusion 674 extending slightly forwardly therefrom. The internal rearwardly facing surface 675, located opposite from body engaging surface 672, serves as a spring-seat for spring 115.

Typically, a pair of diametrically opposed snap portions 676 is formed in recesses extending rearwardly from the forward-facing body engaging surface 672 of the tubular portion 670. Each of the snap portions 676 has an outwardly protruding finger 678 formed at a forward end thereof, the outwardly protruding finger 678 having a rearwardly-facing tapered surface 679, which finger 678 is selectively inwardly deflectable. It is noted that the snap portions 676 are configured for operative engagement with the safety cap 154.

Needle guard element 114 has a pair of side-to-side symmetric mounting arms 680 having rearwardmost ends 682, arranged symmetrically about a longitudinal axis 107. Arms 680 extend along and rearwardly of tubular portion 670 parallel to longitudinal axis 107.

Each of arms 680 defines an outer surface 690 and an inner surface 692. A window 694 is formed on each of arms 680 and adapted for operative engagement with a portion of the control unit 118 in certain operative orientations of the emergency automatic injection device 100. A generally longitudinal rib 696 is formed on two opposite sides of each of the windows, and the ribs 696 each define a forwardly facing surface 698, adapted to operatively engage the front housing element 102 in certain operative orientations of the emergency automatic injection device 100.

A generally trapezoidal stopping rib 700 is formed on the inner surface 692 of each of arms 680 and disposed generally adjacent the rearwardmost end 682.

An inwardly extending protrusion 702 is formed on the inner surface 692 of each of the arms 680 and is generally forwardly spaced from each of the stopping ribs 700. A generally rearwardly tapered surface 704 abuts the protrusion 702 on the rearward end thereof and continues toward the stopping rib 700. It is seen in Figs.9A - 9J that protrusions 702 are disposed generally adjacent to stopping ribs 700.

Disposed generally adjacent to and rearwardly of each of the windows 694 is a generally longitudinal raised protrusion 706 defining a rearwardly facing edge 708 and a guiding rib 710 extending generally rearwardly from the rearwardly facing edge 708. The guiding rib 710 defines a rearwardly facing edge 711.

Reference is now made to Figs. 10A, 10B, 10C, 10D, 10E, 10F, 10G, 10H, 10I and 10J, which are respectively two simplified perspective views, two simplified side plan views, a simplified top plan view, a simplified bottom plan view and four simplified sectional views taken along lines G - G in Fig. 10C, lines H - H and lines I - I in Fig. 10G, and lines J - J in Fig. 10H of the floating cylinder element 152 forming part of the emergency automatic injection assembly 100 of Figs. 1A & 1B.

The floating cylinder element 152 preferably is an integrally formed element, preferably injection molded of plastic and arranged along longitudinal axis 107. The floating cylinder element 152 preferably has a generally truncated conical configuration having a forwardmost end 730 and a rearwardmost generally circular edge 732.

Typically, two diametrically opposed outwardly protruding snap portions 740 are disposed generally adjacent the forwardmost end 730. Each of the snap portions 740 defines a rearwardly facing engagement surface 742, adapted to operatively engage the safety cap 154.

It is also seen in Figs. 10A - 10J that inwardly directed teeth 744 are formed at or in proximity of the rearwardmost end 732 for operative engagement with the cover 140.

The floating cylinder element 152 has a widened portion 750 adjacent the rearwardmost end 732, defining an outer surface 752, which is configured to engage the front housing element 102 and the syringe sleeve 142 for guiding of the floating cylinder element 152.

Reference is now made to Figs. 11A, 11B, 11C, 11D, 11E, 11F, 11G and 11H, which are respectively two simplified perspective views, two simplified side plan views, a simplified top plan view, a simplified bottom plan view and two simplified sectional views taken along lines G - G in Fig. 11C and lines H - H in Fig. 11D of the safety cap element 154 forming part of the emergency automatic injection assembly 100 of Figs. 1A & 1B.

The safety cap element 154 preferably is an integrally formed element, preferably injection molded of plastic and arranged along longitudinal axis 107.

The safety cap element 154 is a generally elongated element, which has a preferably oval cross-section and defines a forwardmost end 770, which is partially closed and preferably has two converging surfaces 772, forming the shape of an arrow.

The safety cap element 154 defines an outer surface 774, which has various gripping surfaces.

An internal hollow cylindrical protrusion 776 is formed within the interior volume of the safety cap element 154 and is arranged along longitudinal axis 107. A central bore 777 is formed through the cylindrical protrusion 776 and extends through the forwardmost end 770. An inwardly extending preferably annular rim 778 is formed on a rearward end of the cylindrical protrusion 776. The annular rim 778 has a forwardly facing edge 780 for operative engagement with the floating cylinder element 152.

Preferably two pins 790 are formed outwardly of the cylindrical protrusion 776 and extend rearwardly from the forwardmost end 770.

It is also seen in Figs. 11A - 11H that the safety cap element 154 defines a rearwardmost edge 792 and several inwardly extending protrusions 794 are formed adjacent the forwardmost end 770 for operative engagement with the front housing element 102.

It is noted that the cover remover assembly 150 is similar in most respects to the cover remover assembly that is described in detail in US Publication US8992477B2.

Reference is now made to Figs. 12A, 12B, 12C, 12D, 12E, 12F, 12G, 12H, 12I, 12J and 12K, which are simplified drawings of the emergency automatic injection assembly 100 of Figs. 1A - 11H in a "storage" operative orientation, including a simplified perspective view, two simplified side plan views, seven simplified sectional views taken along lines D - D in Fig. 12C, lines E - E in Fig. 12B, lines F - F and G - G in Fig. 12E, lines H - H in Fig. 12G and lines I - I in Fig. 12H and two partial cut-out views taken along lines J - J and K - K in Fig. 12A, shown without the forward portion of the emergency automatic injection assembly 100.

The emergency automatic injection assembly 100 comprises a rear end 104, in which is seated a locking element in the form of a locking ring 110, which at least partially surrounds the first injection spring 112, which upon actuation urges forward displacement of the control unit 118. The control unit 118 preferably includes a pair of elastomeric damping elements 120, and selectably engages plunger rod 122 and pre-filled syringe 130. Plunger rod 122 operatively engages pre-filled syringe 130 and is selectably operated by the control unit 118 to inject the liquid contents of pre-filled syringe 130 through hypodermic needle 136.

The forward portion of rear housing element 116 as well as second injection spring 124, control unit 118, plunger rod 122, syringe sleeve 142 and pre-filled syringe 130 are located within the front housing element 102. The needle shield 114 is at least partially slidably positioned within the front housing element 102 and slightly extends forwardly from the forwardmost end of the front housing element 102 and is biased forwardly by the needle shield spring 115. The cover remover assembly 150 is mounted onto the forward end of the needle shield 114 to protect the needle 136 and to enable removal of the cover 140 therefrom, as is described in detail hereinbelow.

As seen in Figs. 12A - 12J, in the storage operative orientation of the emergency automatic injection assembly 100, the rear end element 104 is coupled to the rear housing element 116 by snap fit engagement of snap portions 212 of the rear end element 104 with the protrusions 360 of the rear housing element 116. The front housing element 102 is joined to the rear housing element 116 by engagement of protrusions 370 of the front housing element 102 with attachment portions 588 of the rear housing element 116. Additionally, the syringe sleeve element 142 is fixed with respect to the front housing element 102 by engagement of attachment portions 556 of the syringe sleeve element 142 with the attachment portions 590 of the front housing element 102. It is noted that alternatively, the syringe sleeve element 142 may be formed as an integral portion of the front housing element 102.

It is a particular feature of an embodiment of the present invention that, as seen particularly in Fig. 12J, the control unit 118 is biased to be displaced forwardly along longitudinal axis 107 under the force of the first injection spring 112, however in this storage operative orientation, the first injection spring 112 is in a relatively compressed state and is held in this state by means of engagement between the needle shield 114 and the locking ring 110. Upon release of the locking ring 110, the first injection spring 112 is operative for acting on the control unit 118 and displace it forwardly along longitudinal axis 107, as described in more detail hereinbelow.

It is a further particular feature of an embodiment of the present invention that the control unit element 118 is restrained from forward displacement by means of engagement with the locking ring 110, such that rotation enabling protrusion 520 of the control unit element 118 engages rotation enabling element 290 of the locking ring 110, and particularly forwardly-facing tapered surface 530 of rotation enabling protrusion 520 is supported against forwardly tapered surface 292 of rotation enabling element 290, such that the control unit 118 is prevented from forward longitudinal axial displacement in this storage operative orientation. In turn, the locking ring 110 is prevented from axial displacement along longitudinal axis 107 due to the fact that it is being supported against the rear housing element 116, such that the forward end 262 of the locking ring 110 engages rearwardly facing shoulder 334 of the rear housing element 116, as shown specifically in the enlarged portion of Fig. 12E and in Fig. 12J.

It is seen that the needle shield 114 is in a first forward position in this storage operative orientation, wherein the outwardly protruding fingers 678 of the needle shield 114 are disposed slightly forwardly of the forwardmost end of the front housing element 102, such that rearwardly tapered surfaces 679 of outwardly protruding fingers 678 of needle shield 114 are supported against rearwardly tapered surfaces 610 of the front housing element 102, as particularly seen in Fig. 12E.

It is noted that the cover remover assembly 150 is removably mounted onto the forward portion 598 of the front housing element 102, such that an outer portion of the safety cap 154 at least partially surrounds the forward portion 598 of the front housing element 102 and the cylindrical protrusion 776 of the safety cap 154 at least partially receives a portion of the floating cylinder element 152 therein. It is seen that the floating cylinder element 152 is mounted onto the cover 140, which protects needle 136 of the syringe 130 and that the teeth 744 of the floating cylinder element 152 are snapped behind the rearward end of the cover 140 in order to remove the cover 140 from the needle 136 upon forward displacement of the floating cylinder element 152.

It is additionally seen that the floating cylinder element 152 is slidably mounted within the safety cap 154, such that the outwardly protruding snap portions 740 of floating cylinder element 152 are moveable along central bore 777 of the safety cap 154. It is seen that rearwardly facing engagement surface 742 of the floating cylinder element 152 is forwardly spaced in this example from forwardly facing edge 780 of the safety cap 154. The widened portion 750 of the floating cylinder element 152 is generally guided by the inner surface of the front housing element 102 and by the inner surface of the syringe sleeve element 142.

It is a further particular feature of an embodiment of the present invention that the needle shield 114 is prevented from rearward longitudinal displacement along axis 107. Particularly, snap portions 676 of the needle shield 114 are prevented from inward deflection with respect to longitudinal axis 107 due to the fact that snap portions 676 are supported inwardly by pins 790 of the safety cap 154, which urge engagement of rearwardly tapered surfaces 679 of snap portions 676 with rearwardly tapered surfaces 610 of front housing element 102 and thus prevents rearward displacement of the needle shield 114 relative to the front housing element 102.

It is seen particularly in Fig. 12D that the safety cap 154 is removably mounted onto the front housing element 102 such that inwardly extending protrusions 794 of the safety cap element 154 are seated within recesses 602 of the front housing element 102 and rearwardmost end 792 of the safety cap element 154 preferably abuts forwardly facing shoulder 600 of the front housing element 102, whereas forwardly facing shoulder 600 serves as a stopper for rearward mounting position of the safety cap element 154.

It is also particularly seen in Fig. 12D that the plunger rod 122 is generally enclosed within the control unit 118 and is configured to be restrained from forward displacement by the control unit 118, by means of engagement between the rearwardly facing shoulder 478 of the control unit 118 with the forwardly facing shoulder 426 of the plunger rod 122.

It is seen in Figs. 12D - 12J that in this storage operative orientation the control unit 118 and the syringe 130 are not moveable relative to each other due to the fact that the flange 134 of the syringe 130 is held in place by portions 482 of the syringe holding snap portions 480 of the control unit 118. It is noted that the syringe holding snap portions 480 are prevented from outward deflection by engagement of outwardly extending protrusions 484 with guiding ribs 710 of the needle shield 114. It is seen that portions 482 of the control unit 118 are rearwardly spaced from windows 694 of the needle shield 114 in this storage operative orientation. The syringe 130 is at least partially contained within and guided by the cylindrical portion 550 of the syringe sleeve element 142.

It is also seen particularly in Figs. 12D & 12I that the syringe 130 is partially contained within the syringe sleeve element 142, but the flange 134 of the syringe 130 is rearwardly spaced from double-sided dampening beams 560 of the syringe sleeve element 142.

The needle shield spring 115 is supported between spring seat surface 628 of front housing element 102 and internal surface 675 of the needle shield element 114 and is disposed in a partially compressed position in this storage operative orientation. The first injection spring 112 is supported between inwardly extending flange 382 of the rear housing element 116 and base portion 472 of the control unit 118 and is disposed in at least partially compressed position in this storage operative orientation. The second injection spring 124 is disposed within the inner volume of the plunger rod 122, guided by guiding shaft 126 of the rear end element 104 and is supported between annular protrusion 214 of the rear end element 104 and the forward end of the plunger rod 122 and is disposed in at least partially compressed position in this storage operative orientation.

It is noted that in accordance with this embodiment of the present invention there are two injection springs 112 and 124, whereas the second injection spring 124 is used for increasing the force exerted onto the plunger rod 122 during injection, which is advantageous for example in case of injection of highly viscous medications. It is noted however that a single injection spring can be used instead, such as described in detail in another embodiment of the present invention hereinbelow.

The second injection spring 124 is also beneficial for biasing the plunger rod 122 forwardly and prevent its rearward axial displacement along axis 107.

It is a particular feature of an embodiment of the present invention that upon release of the locking ring 110, the locking ring 110 is urged to rotate under the force of the first injection spring 112, which then acts on the control unit 118, which in turn displaces the plunger rod 122 and the syringe 130 forwardly together therewith along longitudinal axis 107, as is described in detail hereinbelow with respect to Figs. 15A - 15E.

It is particularly seen in Figs. 12E, 12F, 12G, 12J and 12K that in this storage operative orientation the locking ring 110 is prevented from rotation about longitudinal axis 107 by means of engagement of locking members 260 of the locking ring 110 with the stopping ribs 700, which are formed on mounting arms 680 of needle shield 114, particularly by overlapping of stopping ribs 700 of the needle shield 114 and first portions 264 of locking members 260 of the locking ring 110, which prevent the locking ring 110 from rotating.

It is further particularly seen that in this storage operative orientation the locking ring 110 is prevented from rotational displacement and thus prevented from acting on the control unit 118. It is specifically seen in Figs. 12H, 12I and 12J that rotation-enabling elements 290 of the locking ring 110 engage rotation-enabling protrusions 520 of the control unit 118, so that forwardly tapered surface 292 of the locking ring 110 are supported against forwardly-facing tapered surfaces 530 of the control unit 118.

It is particularly seen in Figs. 12F and 12K that the locking ring 110 prevents forward displacement of the needle shield 114 along longitudinal axis 107 in this storage operative orientation. Specifically, needle shield 114 is prevented from forward displacement along longitudinal axis 107 by means of engagement between the second portions 266 of the locking elements 260 of the locking ring 110 and the stopping ribs 700 of the needle shield 114.

It is particularly seen in the enlarged portion of Fig. 12E that the control unit 118 is disengaged from the plunger rod 122 in this storage operative orientation. Particularly, hammer snap portions 500 of the control unit 118 are disposed out of engagement with openings 440 formed in the plunger rod 122 and the outwardly extending protrusions 504 generally abut protrusions 705 of the needle shield element 114.

It is also seen particularly in Fig. 12J that rotation-enabling elements 290 of the locking ring 110 engage protrusions 424 of the plunger rod 122 in this storage operative orientation, such that rearward displacement of the plunger rod 122 in this storage operative orientation is prevented. Specifically, forwardly tapered surfaces 428 of rearwardly extending protrusions 424 are supported against rearwardly tapered surfaces 294 of rotation-enabling elements 290 in this storage operative orientation.

It is seen in Fig. 12D that in this storage operative orientation the dampening elements 120, which are mounted onto a portion of the control unit 118 are rearwardly spaced from frictional surfaces 380 formed on the inner surface of the rear housing element 116.

It is additionally seen in Figs. 12D, 12E, 12H & 12J that the forwardmost end of the plunger rod 122 is partially inserted into the syringe 130, such that the dampening element 128 is about to engage the inner surface of the syringe barrel 132, but the piston engaging wall 404 of the plunger rod 122 is slightly rearwardly spaced from the piston 138 of the syringe 130 to prevent inadvertent ejection of fluid out of the syringe 130.

It is specifically seen in Fig. 12G that snap portions 340 of the rear housing element 116 are engaged with internal recesses 298 of the locking ring 110, such that rotation of the locking ring 110 in a first rotational direction is prevented by means of engagement with the stopping ribs 700 of the needle shield 114 and rotation of the locking ring 110 is a second rotational direction is prevented by this engagement of snaps 340 with recesses 298.

It is particularly seen in Fig. 12E that forwardly facing surface 698 of the needle shield 114 is rearwardly spaced from rearwardly facing shoulder 622 of the front housing element 102.

Reference is now made to Figs. 13A, 13B, 13C, 13D and 13E, which are simplified drawings of the emergency automatic injection assembly 100 of Figs. 1A - 11H in a cover removal operative orientation, including a simplified perspective view, two simplified side plan views, and two simplified sectional views taken along lines D - D in Fig. 13B and lines E - E in Fig. 13C.

It is appreciated that all spatial relationships between the various components of the emergency automatic injection assembly 100 remain the same as described hereinabove with respect to the storage operative orientation illustrated in Figs. 12A - 12K, besides the following spatial relationships:
The user grips the cover remover assembly 150 and pulls it preferably longitudinally forwardly to detach it from the front housing element 102 and thereby remove the cover 140 to expose needle 136 of the syringe 130.

Following removal of the cover remover assembly 150, the forward end of the needle shield 114 is exposed and protrudes forwardly from forwardly facing end 606 of the front housing element 102 to a first longitudinal extent.

It is seen that the needle shield 114 is in the same first forward position in this cover removal operative orientation, wherein the outwardly protruding fingers 678 of the needle shield 114 are disposed slightly forwardly of the forwardmost end of the front housing element 102, such that rearwardly tapered surfaces 679 of outwardly protruding fingers 678 of needle shield 114 are supported against rearwardly tapered surfaces 610 of the front housing element 102.

It is noted that since cover remover assembly 150 is not mounted onto the forward portion 598 of the front housing element 102 anymore, the needle shield 114 is not prevented from rearward longitudinal displacement along axis 107 anymore. Particularly, snap portions 676 of the needle shield 114 are now not prevented from inward deflection with respect to longitudinal axis 107. Upon rearward displacement of the needle shield 114, rearwardly tapered surfaces 679 of snap portions 676 are allowed to slide over the rearwardly tapered surfaces 610 of front housing element 102 and allow rearward displacement of the needle shield 114 relative to the front housing element 102.

It is seen that once the cover remover assembly 150 is detached from the front housing element 102, the inwardly extending protrusions 794 of the safety cap element 154 disengage from recesses 602 of the front housing element 102.

It is noted that upon detachment of the cover remover assembly 150, the teeth 744 of the floating cylinder element 152 are pulling the cover 140 therewith in order to expose the needle 136 upon forward displacement of the floating cylinder element 152.

It is additionally seen that the floating cylinder element 152 is slidably mounted within the safety cap 154, such that the rearwardly facing engagement surface 742 of the floating cylinder element 152 now abuts the forwardly facing edge 780 of the safety cap 154.

It is a further particular feature of an embodiment of the present invention that in this cover removal operative orientation, the needle shield 114 is allowed to be displaced rearwardly along axis 107. Particularly, snap portions 676 of the needle shield 114 are allowed to deflect inwardly with respect to longitudinal axis 107.

Reference is now made to Figs. 14A, 14B, 14C, 14D, 14E, 14F, 14G, 14H and 14I, which are simplified drawings of the emergency automatic injection assembly 100 of Figs. 1A - 11H in a first activation stage operative orientation, including a simplified perspective view, two simplified side plan views, four simplified sectional views taken along lines D - D, E - E, F - F and lines G - G in Fig. 14B, and lines H - H and lines I - I in Fig. 14A and two partial cut-out views taken along lines H - H and I - I in Fig. 14A, shown without the forward portion of the emergency automatic injection assembly 100.

It is appreciated that all spatial relationships between the various components of the emergency automatic injection assembly 100 remain the same as described hereinabove with respect to the cover removal operative orientation illustrated in Figs. 13A - 13E, besides the following spatial relationships:
The user presses the emergency automatic injection assembly 100 against an injection site, thus displaces the needle shield 114 axially rearwardly along axis 107 with respect to the remainder of the emergency automatic injection assembly 100, thus compresses needle shield spring 115 and thereby initiates actuation of the emergency automatic injection assembly 100.

It is a particular feature of an embodiment of the present invention that the first actuation stage operative orientation is a momentary stage, in which the needle shield 114 is displaced rearwardly and thus the locking ring 110 is allowed to be rotated about axis 107 under the biasing force of the first injection spring 112. It is noted that in this first actuation stage operative orientation as illustrated in Figs., 14A - 14I, the locking ring 110 is not yet rotated, the illustrations are shown at a pre-rotation stage thereof, although nothing prevents the locking ring 110 from rotating about longitudinal axis 107 in this operative orientation.

The control unit element 118 is allowed to be displaced forwardly in this first actuation stage operative orientation under the urge of the first injection spring 112, but is not displaced yet as illustrated in Figs. 14C - 14I, so that rotation enabling protrusion 520 of the control unit element 118 still engages rotation enabling element 290 of the locking ring 110, and particularly forwardly-facing tapered surface 530 of rotation enabling protrusion 520 is still supported against forwardly tapered surface 292 of rotation enabling element 290, as particularly seen in Figs. 14G and 14H.

The rearwardly extending protrusions 424 of the plunger rod 122 are supported against rotation-enabling elements 290 of the locking ring 110 in this first actuation stage operative orientation. Specifically, the forwardly tapered surfaces 428 of the rearwardly extending protrusions 424 are supported against rearwardly tapered surfaces 294 of rotation-enabling elements 290.

It is seen that the needle shield 114 is rearwardly displaced in this first activation stage operative orientation and thus it is disposed in its rearward position, wherein forward facing body engagement surface 672 of the needle shield 114 is generally aligned with forwardly facing end 606 of the front housing element 102.

It is seen that upon rearward displacement of the needle shield 114, snap portions 676 of the needle shield 114 are deflected inwardly so that outwardly protruding fingers 678 of the needle shield 114 are now disposed rearwardly of rearwardly tapered surfaces 610 of the front housing element 102. Particularly, upon rearward displacement of the needle shield 114 rearwardly tapered surfaces 679 of outwardly protruding fingers 678 of needle shield 114 slide over the rearwardly tapered surfaces 610 of the front housing element 102.

The needle shield spring 115 is now disposed in a compressed position in this first actuation stage operative orientation. The first injection spring 112 is supported between inwardly extending flange 382 of the rear housing element 116 and base portion 472 of the control unit 118 and is disposed in at least partially compressed position in this first actuation stage operative orientation, just before release of spring 112.

It is a particular feature of an embodiment of the present invention that upon release of the locking ring 110, the locking ring 110 is urged to rotate about the longitudinal axis 107 under the force of the first injection spring 112, which acts on the control unit 118 and in turn on the locking ring 110. The locking ring 110 is in turn is operative for displacing the plunger rod 122 and the syringe 130 forwardly together therewith along longitudinal axis 107 until the control unit 118 engages the plunger rod 122, as is described in detail hereinbelow with respect to Figs. 15A - 15E.

It is a particular feature of an embodiment of the present invention that as seen particularly in Figs. 14E - 14G, in this first activation stage operative orientation the locking ring 110 is allowed to rotate about longitudinal axis 107 since the stopping ribs 700 are now rearwardly spaced from locking members 260 of the locking ring 110 and do not overlap anymore. Once the stopping ribs 700 of the needle shield 114 do not overlap with the locking members 260 of the locking ring 110, the locking ring 110 is allowed to rotate about longitudinal axis 107 under the urge of the first injection spring 112.

It is further particularly seen that in this first actuation stage operative orientation that the locking ring 110 is released, however as illustrated in Figs. 14A - 14I, the rotation-enabling elements 290 of the locking ring 110 still momentarily engage rotation-enabling protrusions 520 of the control unit 118, so that forwardly tapered surface 292 of the locking ring 110 are supported against forwardly-facing tapered surfaces 530 of the control unit 118.

It is noted that the needle shield 114 is prevented from forward displacement along longitudinal axis 107 by means of the force applied by the user against the needle shield spring 115, while pressing the emergency automatic injection assembly 100 against the skin.

It is particularly seen in Figs. 14E, 14F, 14G & 14I that the upon rearward displacement of the needle shield 114, the stopping ribs 700 are rearwardly displaced relative to locking elements 260 of the locking ring 110 and the stopping ribs 700 now engage stoppers 274 of the locking ring 110, specifically stopping ribs 700 engage rearwardly tapered surfaces 278 of the stoppers 274. This engagement serves as a safety measure for initiating the rotation of the locking ring 110 under the urge of first injection spring 112 upon rearward displacement of the needle shield 114. Specifically, if the locking ring 110 would not start rotating automatically under the urge of the first injection spring 112 upon rearward displacement of the needle shield 114, then engagement between the stopping ribs 700 and the tapered surfaces 278 of the stoppers 274 of the locking ring 110 would trigger rotation of the locking ring 110.

It is particularly seen in Figs. 14D - 14G that the control unit 118 starts engaging with the plunger rod 122 in this first actuation stage operative orientation. Particularly, hammer snap portions 500 of the control unit 118 start engaging openings 440 formed in the plunger rod 122 due to the fact the during rearward displacement of the needle shield 114, outwardly extending protrusions 504 of the hammer snap portions 500 slide over rearwardly tapered surfaces 704 of the needle shield element 114 and thus the hammer snap portions 500 are partially inwardly deflected, thereby causing inwardly protruding portions 502 of hammer snap portions 500 to start engaging with openings 440 of the plunger rod 122.

It is a particular feature of an embodiment of the present that the control unit 118 and the plunger rod 122 are displaced together during the actuation stages of the emergency automatic injection assembly 100, as illustrated in Figs. 14A - 15I. First, engagement between the locking ring 110 and the plunger rod 122 causes the plunger rod 122 to be displaced together with the control unit 118, specifically engagement between rearwardly tapered surfaces 294 of the rotation-enabling elements 290 of the locking ring 110 and forwardly tapered surfaces 428 of rearwardly extending protrusions 424 of the plunger rod 122 drives the plunger rod 122 forwardly upon forward displacement of the control unit 118 up to full engagement of the hammer snap portions 500 with openings 440 of the plunger rod 122. Subsequently, the control unit 118 and the plunger rod 122 are displaced forwardly together as a single unit up to a needle penetration operative orientation, as described in detailed hereinbelow with reference to Figs. 15A - 15I.

It is particularly seen in Fig. 14E that forwardly facing surface 698 of the needle shield 114 is more rearwardly spaced from rearwardly facing shoulder 622 of the front housing element 102 in comparison to storage operative orientation.

It is specifically seen in Fig. 14I that snap portions 340 of the rear housing element 116 are still engaged with internal recesses 298 of the locking ring 110 as the locking ring 110 is not rotated yet, such that rotation of the locking ring 110 in the second rotational direction is prevented by this engagement of snaps 340 with recesses 298.

Reference is now made to Figs. 15A, 15B, 15C, 15D, 15E, 15F, 15G, 15H and 15I, which are simplified drawings of the emergency automatic injection assembly 100 of Figs. 1A - 11H in a second activation stage operative orientation, including a simplified perspective view, two simplified side plan views, five simplified sectional views taken along lines D - D in Fig. 15B, lines E - E in Fig. 15C, lines F - F and lines G - G in Fig. 15D, lines H - H in Fig. 15G and a partial section view taken along lines I - I in Fig. 15A, shown without the forward portion of the emergency automatic injection assembly 100.

It is appreciated that all spatial relationships between the various components of the emergency automatic injection assembly 100 remain the same as described hereinabove with respect to the first actuation stage operative orientation illustrated in Figs. 14A - 14I, besides the following spatial relationships:
It is a particular feature of an embodiment of the present invention that following release of engagement between the needle shield 114 and the locking ring 110, which allowed the locking ring to rotate about axis 107, as described hereinabove, with reference to Figs. 14A - 14I, the locking ring 110 is rotated under the biasing force of the first injection spring 112 and due to the engagement of the locking ring 110 with the control unit 118 and thereby urge forward displacement of the plunger rod 122 along the longitudinal axis 107.

It is specifically noted that upon the release of the locking ring 110, due to rearward displacement of the needle shield 114, the force of the spring 112 is applied onto the control unit 118 and urges a forward displacement thereof along longitudinal axis 107. This forward displacement of the control unit 118 transfers the force to the locking ring 110 through the engagement of rotation-enabling protrusion 520 with rotation-enabling element 290, thereby rotating the locking ring 110 about longitudinal axis 107. This rotation of the locking ring 110 initially displaces the plunger rod 122 forwardly along longitudinal axis 107, due to engagement between the protrusions 424 of the plunger rod 122 with the rotation-enabling elements 290 of the locking ring 110, up to a point in which the control unit 118 engages the plunger rod 122 and is then displaced forwardly together therewith as a single unit up to a needle penetration operative orientation, as described in detail hereinbelow with reference to Figs. 16A - 16E.

It is particularly seen in Figs. 15D, 15H and 15I that the force of the first injection spring 112 is exerted onto the control unit 118 upon rearward displacement of the needle shield 114 causing the rotation enabling protrusion 520 of the control unit element 118 to slide over the rotation enabling element 290 of the locking ring 110, and particularly forwardly-facing tapered surface 530 of rotation enabling protrusion 520 slides over forwardly tapered surface 292 of rotation enabling element 290, thus causing the locking ring 110 to rotate about longitudinal axis 107.

It is a particular feature of an embodiment of the present invention that once the control unit element 118 transferred the force of the first injection spring 112 to the locking ring 110 and caused rotation of the locking ring 110 about longitudinal axis 107, the rotation enabling protrusion 520 of the control unit 118 does not engage the rotation-enabling element 290 of the locking ring anymore, as specifically seen in Figs. 15H and 15I and thus the control unit 118 is free to be forwardly displaced along longitudinal axis 107.

It is specifically seen in Figs. 15G - 15I that locking members 260 of the locking ring 110 following rotation are now disposed in a different angular position in comparison to their position as illustrated in Fig.14G before rotation of the locking ring 110.

Following rotation of the locking ring 110, the first injection spring 112 is supported between inwardly extending flange 382 of the rear housing element 116 and base portion 472 of the control unit 118 and is disposed in at least partially released position in this second actuation stage operative orientation. The second injection spring 124 is partially released in this operative orientation as well, since the control unit 118, the plunger rod 122 and the syringe 130 are forwardly axially displaced together under the urge of both the first injection spring 112 and the second injection spring 124.

It is a particular feature of an embodiment of the present invention that the second injection spring 124 constantly biases the plunger rod 122 to be displaced forwardly along axis 107.

It is noted that upon pressing the emergency automatic injection assembly 100 against the skin of the patient, causing rearward displacement of the needle shield 114 relative to the remainder of the emergency automatic injection assembly 100, if the locking ring 110 did not initiate rotation, then the engagement of stopping ribs 700 of the needle shield 114 with stoppers 274 of the locking ring 110 initiates rotation of the locking ring 110, thus serving as a safety measure for actuating the emergency automatic injection assembly 100 (not shown).

It is particularly seen in Figs. 15D & 15I that the control unit 118 is now fully engaged with the plunger rod 122 in this second actuation stage operative orientation. Particularly, hammer snap portions 500 of the control unit 118 are engaged within openings 440 formed in the plunger rod 122 due to the fact that during forward displacement of the control unit 118 along longitudinal axis 107, outwardly extending protrusions 504 of the hammer snap portions 500 slide further along inwardly extending protrusions 702 of the needle shield element 114 towards tapered edges 358 of the rear housing element 116 and thus the hammer snap portions 500 are further inwardly deflected, thereby causing inwardly protruding portions 502 of hammer snap portions 500 to further engage openings 440 of the plunger rod 122.

It is a particular feature of an embodiment of the present invention that during the actuation stage of the emergency automatic injection assembly 100, the control unit 118 is engaged with the plunger rod 122 and is forwardly displaceable together therewith.

This engagement of the control unit 118 with the plunger rod 122 provides for mutual displacement of the two parts, and since the flange 134 of the syringe 130 is held by portions 482 of the control unit 118, all three parts, namely the control unit 118, the plunger rod 122 and the syringe 130, are forwardly displaced together as a single unit to achieve needle penetration into the injection site, as described in detail hereinbelow with reference to Figs. 16A - 16E.

It is noted that in this second actuation stage operative orientation the flange 134 of the syringe 130 is preferably rearwardly spaced from double-sided dampening beams 560 of the syringe sleeve 142.

It is further noted, as seen particularly in Fig. 15E that the dampening elements 120 initiated their frictional slidable displacement along frictional surface 380 of the rear housing element 116 in order to dampen the motion of the plunger rod 122.

It is noted that the plunger rod 122 is slightly displaced forwardly relative to the locking ring 110 in this second actuation stage operative orientation, but the plunger rod 122 is not displaced relative to the syringe 130 yet.

It is specifically seen in Fig. 15G that snap portions 340 of the rear housing element 116 are now engaged with internal recesses 296 of the locking ring 110 as the locking ring 110 is now rotated, such that rotation of the locking ring 110 in the second rotational direction is prevented by this engagement of snaps 340 with recesses 296.

It is noted that the spatial relationships between the different components of the emergency automatic injection assembly 100 that exist in the following operative orientations are briefly described with reference to Fig. 16A - 22F and are generally similar to the operative orientations of the injector described in US publication US8376998B2.

Reference is now made to Figs. 16A, 16B, 16C, 16D and 16E, which are simplified drawings of the emergency automatic injection assembly 100 of Figs. 1A - 11H in a first needle insertion stage operative orientation, including a simplified perspective view, two simplified side plan views, and two simplified sectional views taken along lines D - D in Fig. 16B and lines E - E in Fig. 16C.

It is appreciated that all spatial relationships between the various components of the emergency automatic injection assembly 100 remain the same as described hereinabove with respect to the second actuation stage operative orientation illustrated in Figs. 15A - 15I, besides the following spatial relationships:
The locking ring 110 is preferably not operative anymore in any of the following operative orientations once the emergency automatic injection assembly 100 is actuated.

Upon release of the first and second injection springs 112 and 114, the control unit 118, along with plunger rod 122 and syringe 130 proceed their forward displacement along longitudinal axis 107 in order to perform the penetration of the needle 136 into the injection site.

It is a particular feature of an embodiment of the present invention that in this operative orientation, that the syringe 130 is displaced forwardly up to engagement of the flange 134 with the double-sided dampening beams 560 of the syringe sleeve 142, which are adapted to dampen the impact on the flange 134 upon forward displacement of the syringe 130 and penetration of the needle 136 in order to prevent damage to the syringe 130. It is appreciated that the dampening beams 560 are operative to dampen the impact on the flange 134 since they are axially spaced from rearwardly facing end edge 554 of the syringe sleeve 142 and thus are adapted to be slightly resiliently deflected forwardly upon engagement of the flange 134 therewith.

It is particularly seen in Fig. 16D that the portions 482 of the control unit 118, which retain the syringe 130 relative to the control unit 118, are now disposed in front of windows 694 of the needle shield 114. This is a momentary stage in the operation of the emergency automatic injection assembly 100, just prior to the outward deflection of syringe holding snap portions 480 of the control unit 118.

The first injection spring 112 is further released and acts on the control unit 118 to displace it forwardly and the second injection spring 124 also further released and acts on the plunger rod 122 to provide additional force.

It is particularly seen in Fig. 16D that the control unit 118 is still engaged with both the plunger rod 122 and the syringe 130 in this operative orientation and following the forward displacement thereof along axis 107, the needle 136 now protrudes forwardly from the body engaging surface 672 of the needle shield 114 into the injection site.

It is further seen in Fig. 16D that the hammer snap portions 500 of the control unit 118 are still engaged within openings 440 formed in the plunger rod 122 due to the fact that during forward displacement of the control unit 118, the outwardly extending protrusions 504 of the hammer snap portions 500 have slide over rearwardly facing tapered edges 358 and are further sliding along tapered surface 359 of the rear housing element 116 and are being supported thereby. The hammer snap portions 500 are thus further inwardly deflected, thereby causing inwardly protruding portions 502 of hammer snap portions 500 to further engage openings 440 of the plunger rod 122.

It is further noted, as seen particularly in Fig. 16E that the dampening elements 120 are now in frictional slidable engagement with frictional surface 380 of the rear housing element 116, thus compensating for the force of spring 112 and 124 and resulting in damping of the needle movement and absorbance of the shock applied by portions 482 on the flange 134.

It is noted that the plunger rod 122 is more forwardly spaced from the forwardmost end of the locking ring 110, but the plunger rod 122 is not displaced relative to the syringe 130 yet, thus the piston engaging wall 404 of the plunger rod 122 is still axially spaced from the piston 138 of syringe 130.

Reference is now made to Figs. 17A, 17B, 17C, 17D and 17E, which are simplified drawings of the emergency automatic injection assembly 100 of Figs. 1A - 11H in a second needle insertion stage operative orientation, including a simplified perspective view, two simplified side plan views, and two simplified sectional views taken along lines D - D in Fig. 17B and lines E - E in Fig. 17C.

It is appreciated that all spatial relationships between the various components of the emergency automatic injection assembly 100 remain the same as described hereinabove with respect to the first needle insertion stage operative orientation illustrated in Figs. 16A - 16E, besides the following spatial relationships:
It is particularly seen in Fig. 17D that the syringe holding snap portions 480 of the control unit, which retained the syringe 130 relative to the control unit 118, are now outwardly deflected into windows 694 of the needle shield 114, so that portions 482 do not hold the flange 134 of the syringe 130 anymore and the syringe 130 is disposed in its forwardmost position.

It is further seen in Fig. 17D that the control unit 118 is still engaged with the plunger rod 122, but not with the syringe 130 in this operative orientation and following the forward displacement thereof along axis 107, the plunger rod 122 is allowed to be displaced axially forwardly along axis 107 relative to syringe 130 and eject the medicament therefrom.

It is noted that in this illustration as seen in Figs. 17D & 17E, which represents a momentary stage in the operation of the emergency automatic injection assembly 100, the piston engaging wall 404 of the plunger rod 122 is still axially spaced from the piston 138 of syringe 130.

Reference is now made to Figs. 18A, 18B, 18C, 18D and 18E, which are simplified drawings of the emergency automatic injection assembly 100 of Figs. 1A - 11H in a third needle insertion stage operative orientation, including a simplified perspective view, two simplified side plan views, and two simplified sectional views taken along lines D - D in Fig. 18B and lines E - E in Fig. 18C.

It is appreciated that all spatial relationships between the various components of the emergency automatic injection assembly 100 remain the same as described hereinabove with respect to the second needle insertion stage operative orientation illustrated in Figs. 17A - 17E, besides the following spatial relationships:
It is particularly seen in Fig. 18D that the syringe holding snap portions 480 of the control unit, which retained the syringe 130 relative to the control unit 118, now passed the flange 134 and biased to deflect back inwardly and disposed forwardly of the flange 134.

It is further seen in Fig. 18D that discard tooth snap portions 510 of the control unit 118 are rearwardly spaced from flange 134 of syringe 130 in this operative orientation and following the forward displacement thereof along axis 107, the plunger rod 122 is allowed to be displaced axially forwardly along axis 107 relative to syringe 130.

It is noted that in this illustration as seen in Figs. 18D & 18E the piston engaging wall 404 of the plunger rod 122 now engages the piston 138 of syringe 130 and ready for ejection of medicament out of the syringe barrel 132 through needle 136.

The first injection spring 112 is further released and acts on the control unit 118 to displace it forwardly and the second injection spring 124 also further released and acts on the plunger rod 122 to provide additional force and displace the plunger rod 122 relative to the syringe 130.

Reference is now made to Figs. 19A, 19B, 19C, 19D and 19E, which are simplified drawings of the emergency automatic injection assembly 100 of Figs. 1A - 11H in an end of delivery operative orientation, including a simplified perspective view, two simplified side plan views, and two simplified sectional views taken along lines D- D in Fig. 19B and lines E - E in Fig. 19C.

It is appreciated that all spatial relationships between the various components of the emergency automatic injection assembly 100 remain the same as described hereinabove with respect to the third needle insertion stage operative orientation illustrated in Figs. 18A - 18E, besides the following spatial relationships:
It is seen in Figs. 19D & 19E that the control unit 118 and the plunger rod 122 are axially forwardly advanced along axis 107 while forcing the medicament out of syringe barrel 132 through needle 136 into the injection site. During drug delivery, the forward motion of the piston 138 is governed by friction between dampening elements 120 and tapered surface 359 of the rear housing element 116. The amount of friction may be selected by appropriately shaping the tapered surface 359 and the dampening elements 120. It is noted that if the tapered surface 359 is generally triangular, then it causes a reduction in friction as control unit 118 advances, which compensates for the reduction in the force applied by injection springs 112 and 124 as they extend. Alternatively, a rectangular tapered surface 359 preferably provides for a constant friction.

It is noted that in this illustration as seen in Figs. 19D & 19E the piston 138 has reached the forwardmost end of the barrel 132 of syringe 130, so that the entire amount of medicament is ejected therefrom in this operative orientation.

The second injection spring 124 now biases the plunger rod 122 forwardly to restrain rearward displacement of the plunger rod 122.

The hammer snap portions 500 of the control unit 118 slide off the tapered surfaces 359 of the rear housing element 116 at this end of delivery operative orientation and engage raised protrusions 706 of the needle shield 114, thereby providing an audible click as an indication to the user, due to increase in diameter between the portion of the rear housing element 116 formed by surfaces 359 and between the portion of the needle shield 114 formed by protrusions 706.

Discard tooth snap portions 510 of the control unit 118 are less rearwardly spaced from the flange 134 in this operative orientation.

Forwardly facing surface 698 of needle shield 114 is rearwardly spaced from rearwardly facing shoulder 622 of the front housing element 102 in this operative orientation since the needle shield 114 is still pressed against the injection site.

Reference is now made to Figs. 20A, 20B, 20C, 20D, 20E and 20F, which are simplified drawings of the emergency automatic injection assembly 100 of Figs. 1A - 11H in a removal from injection site operative orientation, including a simplified perspective view, two simplified side plan views, and three simplified sectional views taken along lines D - D and lines E- E in Fig. 20B and lines F - F in Fig. 20C.

It is appreciated that all spatial relationships between the various components of the emergency automatic injection assembly 100 remain the same as described hereinabove with respect to the end of delivery operative orientation illustrated in Figs. 19A - 19E, besides the following spatial relationships:
It is seen in Figs. 20A - 20F that the emergency automatic injection assembly 100 is in a process of removal from the injection site, whereas the needle shield 114 starts extending forwardly with respect to the remainder of the emergency automatic injection assembly and snap portions 676 are already disposed slightly forwardly of the forwardly facing end 606 of the front housing element 102.

It is noted that in this illustration, as seen in Figs. 20D - 20F, the needle shield spring 115 starts pushing the needle shield 114 forwardly, since the user releases it from the injection site, and the needle shield 114 is in the process of protecting the needle 136 after injection of the medicament.

It is seen that subsequent the initial forward displacement of the needle shield 114, the hammer snap portions 500 are released and deflected outwardly to their initial position and thus disengage the openings 440 of the plunger rod 122.

The control unit 118 is restrained from forward displacement by means of engagement of hammer snap portions 500 of the control unit 118 with rearwardly facing edge 708 of the needle shield 114 in this operative orientation, as seen particularly in Fig. 20E.

Discard tooth snap portions 510 of the control unit 118 are less rearwardly spaced from the flange 134 in this operative orientation.

Forwardly facing surface 698 of needle shield 114 is less rearwardly spaced from rearwardly facing shoulder 622 of the front housing element 102 in this operative orientation since the needle shield 114 is only partially pressed against the injection site.

Reference is now made to Figs. 21A, 21B, 21C, 21D, 21E and 21F, which are simplified drawings of the emergency automatic injection assembly 100 of Figs. 1A - 11H in a first discard stage operative orientation, including a simplified perspective view, two simplified side plan views, and three simplified sectional views taken along lines D - D in Fig. 21B, lines E - E in Fig. 21C and lines F - F in Fig. 21D.

It is appreciated that all spatial relationships between the various components of the emergency automatic injection assembly 100 remain the same as described hereinabove with respect to the removal from injection site operative orientation illustrated in Figs. 20A - 20F, besides the following spatial relationships:
At this stage, the emergency automatic injection assembly 100 is fully disengaged from the injection site and the needle shield 114 is fully extended to fully enclose the needle 136. When the needle shield 114 is fully extended it is locked with respect to the control unit 118 by means of engagement between rearwardly facing edge 708 of the needle shield 114 and hammer snap portions 500 of the control unit 118, thus rearward displacement of the needle shield 114 produces equivalent rearward displacement of the control unit 118.

The needle shield 114 is restrained from further forward axial displacement by means of engagement between forwardly facing surface 698 thereof with rearwardly facing shoulder 622 of the front housing element 102.

Discard tooth snap portions 510 of the control unit 118 are now positioned forwardly of flange 134 in this operative orientation, and thus lock the syringe with respect to the control unit 118.

It is noted that the needle shield 114 is locked with respect to the control unit 118 and the syringe 130 is locked with respect to the control unit 118, thus the needle shield 114 is locked with respect to the syringe 130, thereby rearward displacement of the needle shield 114 produces equivalent rearward displacement of the syringe 130, thus provides for constant protection of the needle 136.

It is also noted that the inwardly protruding portion 502 of hammer snap portions 500 is prevented from inward deflection by engagement of inwardly protruding portion 502 of the control unit 118 with the outer surface of the plunger rod 122, thus engagement of the control unit 118 with the plunger rod 122 is prevented.

Reference is now made to Figs. 22A, 22B, 22C, 22D, 22E and 22F, which are simplified drawings of the emergency automatic injection assembly 100 of Figs. 1A - 11H in a second discard stage operative orientation, including a simplified perspective view, two simplified side plan views, and three simplified sectional views taken along lines D - D and lines F - F in Fig. 22B and lines E - E in Fig. 22C.

It is appreciated that all spatial relationships between the various components of the emergency automatic injection assembly 100 remain the same as described hereinabove with respect to the first discard stage operative orientation illustrated in Figs. 21A - 21F, besides the following spatial relationships:
Should the needle shield 114 be pushed rearwardly with respect to the front housing element 102, the rearwardly facing edge 708 of the needle shield 114 pushes against the hammer snap portions 500 of the control unit 118, thus the control unit 118 is forced to move rearwardly together with the needle shield 114.

Due to engagement of discard tooth snap portions 510 and flange 134, the control unit 118 forces the needle 136 and syringe 130 to move rearwardly together with control unit 118, so that the needle 136 does not protrude from the needle shield 114. During this rearward movement, hammer snap portions 500 of the control unit 118 cannot bend inwards due to the fact that they are inwardly supported by the outer surface of the plunger rod 122.

It is seen specifically in Figs. 22D & 22F that the forwardly facing surface 698 of the needle shield 114 is rearwardly spaced from forwardly facing shoulder 622 of the front housing element 102 since the needle shield 114 is displaced rearwardly.

Reference is now made to Figs. 23A and 23B, which are respectively simplified exploded view and sectional exploded view of an emergency automatic injection assembly 900 constructed and operative in accordance with another embodiment of the present invention, the sectional view taken along lines B - B in Fig. 23A.

It is noted that emergency automatic injection assembly 900 is preferably similar to emergency automatic injection assembly 100, as described with reference to Figs. 1A - 22F. Same components of the two emergency automatic injection assemblies 100 and 900 are designated by the same reference numerals.

As seen in Figs. 23A and 23B, the emergency automatic injection assembly 900 comprises a front housing element 102 and a rear end element 104, which are preferably fixedly attached, such as by a snap-fit engagement. A label 105 is adapted to be mounted over the front housing element 102. It is noted that the front housing element 102 is formed with a window 106 and the label 105 has an opening 108, which is adapted to be aligned with the window 106 when the label is mounted onto the front housing element 102 to permit viewing of a portion of the contents of the emergency automatic injection assembly 900 therethrough. It is noted that the front housing element 102 and the rear end 104 are arranged along a mutual longitudinal axis 107.

Disposed within the enclosure formed by the rear end 104 and the front housing element 102 there is provided a locking element in the form of a locking ring 110, which is configured to be biased to rotate about longitudinal axis 107 under the force of an injection spring 112, but is operatively blocked from rotation in certain operative orientations of the emergency automatic injection assembly 900 by engagement with a rear portion of a needle shield 114. The needle shield 114 is arranged along longitudinal axis 107 and extends forwardly so as to protrude forwardly from the front housing element 102. The needle shield 114 is operative to be biased forwardly under the force of a needle shield spring 115.

A rear housing element 116 is also arranged along the longitudinal axis 107 and a rear portion thereof is at least partially received into the locking ring 110. The rear housing element 116 is preferably contained within the needle shield 114.

A control unit 118 is arranged along the longitudinal axis 107 and a rear portion thereof is at least partially received into the rear portion of the rear housing element 116. An injection spring 112 is disposed generally between the rear portion of the control unit 118 and the rear portion of the rear housing 116 and is adapted to act on the control unit 118 when released. It is noted that typically two resilient dampening elements 120 are mounted onto the rear portion of the control unit 118 for frictional engagement with an inner surface of the rear housing element 116.

It is noted that in the storage operative orientation, the a plunger rod 922 is generally enclosed within the control unit 118 and is configured to be restrained by the control unit 118 in its retracted rearward position, by means of engagement between the rearwardly facing shoulder 478 of the control unit 118 with the forwardly facing shoulder 426 of the plunger rod 922. The plunger rod 922 is arranged along longitudinal axis 107 and is generally similar to plunger rod 122 in most respects other than that described hereinbelow with reference to Figs. 24A - 24K. A forward dampening element 128 is preferably mounted onto a forward portion of the plunger rod 922.

A syringe 130 is configured to be held by the control unit 118 in certain operative orientations of the emergency automatic injection assembly. The pre-filled syringe 130 has a syringe barrel 132 having a flange 134 formed at its rearward end and a needle 136 fixedly attached to its forward end. A piston 138 is contained within the syringe barrel 132, which confines the medicament within the syringe barrel 132. A cover 140, suitable for single use, is adapted to seal and protect the needle 136. It is appreciated that syringe 130 can be any type of medicament container, such as pre-filled syringe or cartridge.

It is also noted that at least a portion of the syringe 130 is configured to reside within a syringe sleeve 142, which is preferably fixedly attached to the front housing element 102 or integrally made therewith.

A cover remover assembly 150 is adapted to be mounted over the forward portion of the front housing element 102 and of the needle shield 114 to protect the needle 136 in storage and permit removal of the cover 140 before injection.

It is seen in Figs. 23A & 23B that the cover remover assembly 150 comprises a floating cylinder 152 and a safety cap 154 at least partially disposed around it, both the floating cylinder 152 and the safety cap 154 being arranged along longitudinal axis 107. It is noted that the floating cylinder 152 is axially displaceable relative to the safety cap 154 along the longitudinal axis 107 to compensate for manufacturing tolerances of the various elements of the emergency automatic injection assembly.

Reference is now made to Figs. 24A, 24B, 24C, 24D, 24E, 24F, 24G, 24H, 24I & 24K, which are respectively two simplified perspective views, two simplified side plan views, a simplified top plan view, a simplified bottom plan view and five simplified sectional views taken along lines G - G in Fig. 24C, lines I - I in Fig. 24D, lines H - H and J - J in Fig. 24G and lines K - K in Fig. 24J of the plunger rod element 922 forming part of the emergency automatic injection assembly 900 of Figs. 23A & 23B.

The plunger rod element 922 is generally similar in most respects to plunger rod 122, which is described hereinabove with reference to Figs. 5A - 5I, and has an additional feature as described hereinbelow.

The plunger rod element 922 preferably is an integrally formed element, preferably injection molded of plastic and is arranged along longitudinal axis of symmetry 107.

The plunger rod element 922 preferably includes a generally hollow cylindrical shaft 400 arranged along longitudinal axis 107 and defining an interior bore 402. The cylindrical shaft 400 has a protrusion 403, extending axially forwardly from a forward end of shaft 400 and defining a piston engaging wall 404 formed at the forward end thereof. The piston engaging wall 404 is generally disposed transversely to longitudinal axis 107.

It is seen in Figs. 24A - 24K that a circumferential recess 410 is formed generally adjacent to and rearwardly spaced from the protrusion 403. The recess 410 serves as a seat for the forward dampening element 128, which serves in use so as to dampen the plunger rod 922 displacement within syringe 130. A small air passage opening 412 is formed on an edge of recess 410. The function of this forward dampening element 128 in conjunction with the air passage opening 412 is described in detail in publication US Publication US20190275251A1, for example with reference to the improved plunger and damper assembly 3160.

A generally annular widened flange 420 is formed on the rearward end of the cylindrical shaft 400. The flange 420 has a rearwardly facing end surface 422, having several rearwardly extending protrusions 424 formed thereon, for operative engagement with a portion of the locking ring 110. The flange 420 also has a forwardly facing shoulder 426, which is operative for engagement with the control unit 118. The rearwardly extending protrusions 424, each define a forwardly tapered surface 428.

The cylindrical shaft 400 typically includes a pair of diametrically opposed generally longitudinal flat portions 430 formed on the circumference thereof. A longitudinal guiding rib 432 is formed on each of the flat portions 430 for guiding of the plunger rod 922 within the control unit 118.

There are typically two inwardly extending openings 440, each formed in between the two guiding ribs 432, and the two openings are preferably diametrically opposed to each other. The openings 440 are preferably disposed adjacent the widened flange 420. A longitudinal rib 442 extends longitudinally forwardly from each of openings 440, defining a longitudinal surface 443 and forming a rearwardly facing shoulder 444 between the opening 440 and the rib 442. It is noted that the openings 440 and rearwardly facing shoulders 444 associated therewith are configured for operative engagement with a portion of the control unit 118 in certain operative orientations of the emergency automatic injection assembly 900. The ribs 442 are configured for operative engagement with a portion of the control unit 118 in other operative orientations.

Preferably two diametrically opposed snap portions 930 are formed on cylindrical shaft 400 of the plunger rod 922 and each defines a forwardly facing shoulder 932 for engagement with the needle shield 114 in certain operative orientations of the emergency automatic injection assembly 900, as described in detail hereinbelow.

It is noted that emergency automatic injection assembly 900 is functional preferably similarly to emergency automatic injection assembly 100 in all operative orientations described with reference to Figs. 12A - 20F and 22A - 22F, other than the fact that there is no second injection spring 124 in emergency automatic injection assembly 900 in comparison to emergency automatic injection assembly 100. Only the first injection spring 112 acts on the control unit 118, which in turn acts on the plunger rod element 922, as described in detail hereinabove, with reference to the emergency automatic injection assembly 100.

A different aspect of emergency automatic injection assembly 900 is reflected in the first stage of discard operative orientation, which is described with reference to Figs. 21A - 21F for emergency automatic injection assembly 100 and is now described below with reference to Figs. 25A - 25F for emergency automatic injection assembly 900, due to a slightly modified structure of the plunger rod 922, in comparison to plunger rod 122.

Reference is now made to Figs. 25A, 25B, 25C, 25D, 25E, 25F and 25G, which are simplified drawings of the emergency automatic injection assembly 900 of Figs. 23A and 23B in a first discard stage operative orientation, including a simplified perspective view, two simplified side plan views, and four simplified sectional views taken along lines D - D in Fig. 25B, lines E - E, lines F - F and lines G - G in Fig. 25C.

At this stage, the emergency automatic injection assembly 900 is fully disengaged from the injection site and the needle shield 114 is fully extended to fully enclose the needle 136. When the needle shield 114 is fully extended, it is locked with respect to the control unit 118 by means of engagement between rearwardly facing edge 708 of the needle shield 114 and hammer snap portions 500 of the control unit 118, thus rearward displacement of the needle shield 114 produces equivalent rearward displacement of the control unit 118.

The needle shield 114 is restrained from further forward axial displacement by means of engagement between forwardly facing surface 698 thereof with rearwardly facing shoulder 622 of the front housing element 102.

Discard tooth snap portions 510 of the control unit 118 are now positioned forwardly of flange 134 in this operative orientation, and thus lock the syringe with respect to the control unit 118.

It is noted that the needle shield 114 is locked with respect to the control unit 118 and the syringe 130 is locked with respect to the control unit 118, thus the needle shield 114 is locked with respect to the syringe 130, thereby rearward displacement of the needle shield 114 produces equivalent rearward displacement of the syringe 130, thus provides for constant protection of the needle 136.

It is also noted, as specifically seen in Fig. 25D, that relative displacement between the control unit 118 and the needle shield 114 is also prevented by engagement of the inwardly protruding portion 502 of hammer snap portions 500 of the control unit 118 with the outer surface of the plunger rod 922, thus preventing inward deflection of the hammer snap portions 500 and in turn prevents engagement of the control unit 118 with the plunger rod 922.

It is seen in Figs. 25D - 25G that the control unit 118 is disposed at its forwardmost position in this discard operative orientation.

It is a particular feature of an embodiment of the present invention that the plunger rod 922 is prevented from rearward displacement relative to the control unit 118 along longitudinal axis 107 in this discard operative orientation due to engagement of shoulders 932 of snap portions 930 of the plunger rod 922 with forwardly facing shoulder 473 of the control unit 118, thus assures supporting the hammer snap portions 500 of the control unit 118 in this discard operative orientation, locked between the needle shield element 114 and the plunger rod 922. Specifically, the hammer snap portions 500 of the control unit 118 are locked between longitudinal surface 443 of the plunger rod 922 and rearwardly facing edge 711 of the needle shield element 114.

This invention generally relates to an automatic injection device for parenteral administration of substances (e.g., a medication) to a living organism (human or animal). The administration may be delivered into the subcutaneous tissue.

The invention is further related to, but is not limited to a self-administration of patients with chronic diseases such as rheumatoid arthritis (RA), multiple sclerosis (MS), HIV, and growth hormone deficiency.

It is appreciated that in accordance with an embodiment of the present invention the medicament is enclosed in a pre-filled syringe, but it can alternatively be used with other drug enclosures such as vials or ampoules, where a vial adaptor or an ampoule adaptor is used to reconstitute, mix, or pump the drug into the syringe prior to injection. The pre-filled syringe can be either a conventional one chambered pre-filled syringe with a ready-to-inject liquid form drug, or it can be a multiple-chambered pre-filled syringe.

The emergency automatic injection device provides an automatic needle insertion through the skin, which therefore overcomes the main obstacle in self-administration, i.e., the needle phobia; the user does not see the needle through all the procedure, i.e., before, during and after injection.

It will be appreciated by persons skilled in the art that the present invention is not limited by what has been particularly shown and described hereinabove. Rather the scope of the present invention is defined by the claims.

## Claims

1. An automatic injection device (100) for use with a syringe (130) including at least one syringe piston (138) and a needle (136) coupled to a forward end thereof, comprising:
a housing element (102,104) arranged along a longitudinal axis (107) and having a forward end and a rearward end;
at least one resilient element (112) arranged to be located within said housing element (102,104);
a needle shield (114) selectably positionable with respect to said housing element (102,104);
and a control unit (118) adapted, when actuated, to be driven by said at least one resilient element (112) for initially displacing said syringe (130) relative to said housing element (102,104) from a non-penetration position to a penetration position and thereafter displacing said at least one syringe piston (138) in said syringe (130) to effect drug delivery,
and wherein said control unit (118) is configured to be actuated upon axial rearward displacement of said needle shield (114) with respect to said housing element (102,104),
and wherein the automatic injection device also comprises a locking element operative for selectable displacement relative to said housing element (102,104) and being operatively engageable with said needle shield (114),
the automatic injection device **characterized in that**
the locking element is a locking ring (110), and said locking ring (110) is configured to be biased to rotate about the longitudinal axis (107) under the urge of said at least one resilient element (112) but is blocked from rotation in a first operative orientation of the automatic injection device (100) by engagement with a rear portion of the needle shield (114); and
upon axial rearward displacement of said needle shield (114) with respect to said housing element (102,104), said locking ring (110) is permitted to rotate about said longitudinal axis (107) under the urge of said at least one resilient element (112).

2. The automatic injection device (100), according to claim 1, and wherein said locking ring (110) is selectably operatively engaged with said control unit (118) and wherein said control unit (118) is operative for causing rotation of said locking ring (110) under the urge of said at least one resilient element (112) upon axial rearward displacement of said needle shield (114) with respect to said housing element (102,104).

3. The automatic injection device (100), according to claim 1 or claim 2, also comprising a plunger rod (122), operative to selectably drive said at least one syringe piston (138) in axial motion relative to said housing element (102,104); said plunger rod (122) is operative to be displaced together with said control unit (118) from said actuation of said control unit (118) up to said penetration position of said syringe (130).

4. The automatic injection device (100), according to any one of claims 1 - 3, and wherein said at least one resilient element includes a single spring (112).

5. The automatic injection device (100), according to claim 3, and wherein said at least one resilient element includes a first spring and a second spring (124).

6. The automatic injection device (100), according to claim 5, and wherein said second spring (124) is at least partially disposed within said plunger rod (122) and is operative for biasing said plunger rod (122) to be displaced forwardly along said longitudinal axis (107).

7. The automatic injection device (100), according to any one of claims 1 - 6, also comprising a needle shield remover (150) configured to be removably attached to said housing element (102,104), operative for protecting said needle (136), and wherein said needle shield (114) is prevented from axial rearward displacement with respect to said housing element (102,104) when said needle shield remover (150) is attached to said housing element (102,104).

8. The automatic injection device (100), according to claim 7, and wherein said needle cover remover (150) includes at least one fixating element (790), operative to engage a corresponding fixating counter-element (676) formed on said needle shield (114) to prevent inadvertent rearward displacement of said needle shield (114) with respect to said housing element (102,104).

9. The automatic injection device (100), according to any one of claims 1 - 8, and wherein said locking ring (110) is permitted to be rotated in a single rotational direction.

10. The automatic injection device (100), according to any one of claims 1 - 9, and wherein said locking ring (110) is selectably positioned in one of a locked orientation and an unlocked orientation relative to said control unit (118); and wherein when said locking ring (110) is positioned in said unlocked orientation, said at least one resilient element is permitted to drive said control unit (118) axially forwardly relative to said housing element (102,104).

11. The automatic injection device (100), according to claim 10, and wherein said locking ring (110) has a rotation enabling element (290) and said control unit (118) has a counter rotation enabling element (520), which engages said rotation enabling element (290) when said locking ring (110) is disposed in said locked orientation.

12. The automatic injection device (100), according to claims 10 or 11, and wherein said needle shield (114) is prevented from axial forward longitudinal displacement relative to the housing (102,104) when said locking ring (110) is disposed in said locked orientation.

13. The automatic injection device (100), according to any one of claims 1 - 12 and also comprising a syringe sleeve (142), which is fixedly attached to or integrally made with said housing element (102,104) and includes a dampening element (560), adapted to dampen the impact on the syringe (130) upon forward displacement thereof and during penetration of said needle (136).

14. The automatic injection device (100), according to any one of claims 3 - 13 and wherein in a post-injection operative state, the plunger rod (122) is prevented from rearward axial displacement relative to said housing element (102,104).

15. The automatic injection device (100), according to claim 1, and wherein said locking ring (110) is prevented from displacement relative to said housing element (102,104) due to engagement with said needle shield (114) in a prior to injection operative state and wherein said control unit (118) is prevented from displacement relative to said housing element (102,104) due to engagement with said locking ring (110) in said prior to injection operative state.

## Patentansprüche

1. Automatisches Injektionsgerät (100) zur Verwendung mit einer Spritze (130), die mindestens einen Spritzenkolben (138) und eine Nadel (136), die an ein vorderes Ende davon gekoppelt ist, beinhaltet, umfassend:
ein Gehäuseelement (102, 104), das entlang einer Längsachse (107) eingerichtet ist und ein vorderes Ende und ein hinteres Ende aufweist;
mindestens ein elastisches Element (112), das dazu eingerichtet ist, sich innerhalb des Gehäuseelements (102, 104) zu befinden;
einen Nadelschutz (114), der in Bezug auf das Gehäuseelement (102, 104) wählbar positionierbar ist;
und eine Steuerungseinheit (118), die dazu angepasst ist, bei Betätigung, von dem mindestens einen elastischen Element (112) angetrieben zu werden, um die Spritze (130) zunächst relativ zu dem Gehäuseelement (102, 104) von einer Nichteindringungsposition in eine Eindringungsposition zu verschieben und danach den mindestens einen Spritzenkolben (138) in der Spritze (130) zu verschieben, um eine Medikamentenabgabe zu bewirken,
und wobei die Steuerungseinheit (118) dazu konfiguriert ist, bei einer axialen Rückwärtsverschiebung des Nadelschutzes (114) in Bezug auf das Gehäuseelement (102, 104) betätigt zu werden,
und wobei das automatische Injektionsgerät auch ein Verriegelungselement umfasst, das für eine wählbare Verschiebung relativ zu dem Gehäuseelement (102, 104) betriebsfähig ist und mit dem Nadelschutz (114) in Wirkeingriff gebracht werden kann,
wobei das automatische Injektionsgerät **dadurch gekennzeichnet ist, dass**
das Verriegelungselement ein Sicherungsring (110) ist, und der
Sicherungsring (110) dazu konfiguriert ist, vorgespannt zu werden, um sich um die Längsachse (107)
unter der Einwirkung des mindestens einen elastischen Elements (112) zu drehen, aber in einer ersten Betriebsausrichtung des automatischen Injektionsgeräts (100) durch Eingriff mit einem hinteren Abschnitt des Nadelschutzes (114) gegen Drehung blockiert ist; und
bei einer axialen Rückwärtsverschiebung des Nadelschutzes (114) in Bezug auf das Gehäuseelement (102, 104) es dem Sicherungsring (110) erlaubt ist, sich um die Längsachse (107) unter der Einwirkung des mindestens einen elastischen Elements (112) zu drehen.

2. Automatisches Injektionsgerät (100) nach Anspruch 1, und wobei der Sicherungsring (110) wählbar in Wirkeingriff mit der Steuerungseinheit (118) steht und wobei die Steuerungseinheit (118) dazu betriebsfähig ist, eine Drehung des Sicherungsrings (110) unter der Einwirkung des mindestens einen elastischen Elements (112) bei einer axialen Rückwärtsverschiebung des Nadelschutzes (114) in Bezug auf das Gehäuseelement (102, 104) zu bewirken.

3. Automatisches Injektionsgerät (100) nach Anspruch 1 oder Anspruch 2, auch umfassend einen Kolbenstab (122), der dazu betriebsfähig ist, den mindestens einen Spritzenkolben (138) wählbar in axialer Bewegung relativ zu dem Gehäuseelement (102, 104) anzutreiben; wobei der Kolbenstab (122) dazu betriebsfähig ist, zusammen mit der Steuerungseinheit (118) von der Betätigung der Steuerungseinheit (118) bis zu der Eindringungsposition der Spritze (130) verschoben zu werden.

4. Automatisches Injektionsgerät (100) nach einem der Ansprüche 1 bis 3, und wobei das mindestens eine elastische Element eine einzelne Feder (112) beinhaltet.

5. Automatisches Injektionsgerät (100) nach Anspruch 3, und wobei das mindestens eine elastische Element eine erste Feder und eine zweite Feder (124) beinhaltet.

6. Automatisches Injektionsgerät (100) nach Anspruch 5, und wobei die zweite Feder (124) zumindest teilweise innerhalb des Kolbenstabs (122) angeordnet ist und dazu betriebsfähig ist, den Kolbenstab (122) so vorzuspannen, dass er entlang der Längsachse (107) nach vorne verschoben wird.

7. Automatisches Injektionsgerät (100) nach einem der Ansprüche 1 bis 6, auch umfassend einen Nadelschutzentferner (150), der dazu konfiguriert ist, abnehmbar an dem Gehäuseelement (102, 104) befestigt zu werden, der dazu betriebsfähig ist, die Nadel (136) zu schützen, und wobei der Nadelschutz (114) an einer axialen Rückwärtsverschiebung in Bezug auf das Gehäuseelement (102, 104) gehindert wird, wenn der Nadelschutzentferner (150) an dem Gehäuseelement (102, 104) befestigt ist.

8. Automatisches Injektionsgerät (100) nach Anspruch 7, wobei der Nadelabdeckungsentferner (150) mindestens ein Befestigungselement (790) beinhaltet, das dazu betriebsfähig ist, mit einem entsprechenden Befestigungsgegenelement (676) in Eingriff zu treten, das an dem Nadelschutz (114) ausgebildet ist, um eine unbeabsichtigte Rückwärtsverschiebung des Nadelschutzes (114) in Bezug auf das Gehäuseelement (102, 104) zu verhindern.

9. Automatisches Injektionsgerät (100) nach einem der Ansprüche 1 bis 8, und wobei es dem Sicherungsring (110) erlaubt ist, in einer einzigen Drehrichtung gedreht zu werden.

10. Automatisches Injektionsgerät (100) nach einem der Ansprüche 1 bis 9, und wobei der Sicherungsring (110) wählbar in einer von einer verriegelten Ausrichtung und einer entriegelten Ausrichtung relativ zu der Steuerungseinheit (118) positioniert ist; und wobei, wenn der Sicherungsring (110) in der entriegelten Ausrichtung positioniert ist, es dem mindestens einen elastischen Element erlaubt ist, die Steuerungseinheit (118) axial nach vorne relativ zu dem Gehäuseelement (102, 104) anzutreiben.

11. Automatisches Injektionsgerät (100) nach Anspruch 10, und wobei der Sicherungsring (110) ein Drehfreigabeelement (290) aufweist und die Steuerungseinheit (118) ein Gegendrehfreigabeelement (520) aufweist, das mit dem Drehfreigabeelement (290) in Eingriff steht, wenn der Sicherungsring (110) in der verriegelten Ausrichtung angeordnet ist.

12. Automatisches Injektionsgerät (100) nach Anspruch 10 oder 11, und wobei der Nadelschutz (114) an einer axialen Vorwärtslängsverschiebung relativ zu dem Gehäuse (102, 104) gehindert wird, wenn der Sicherungsring (110) in der verriegelten Ausrichtung angeordnet ist.

13. Automatisches Injektionsgerät (100) nach einem der Ansprüche 1 bis 12 und auch umfassend eine Spritzenhülse (142), die fest an dem Gehäuseelement (102, 104) befestigt oder einstückig mit diesem hergestellt ist und ein Dämpfungselement (560) beinhaltet, das dazu angepasst ist, den Aufprall auf die Spritze (130) bei der Vorwärtsverschiebung davon und während des Eindringens der Nadel (136) zu dämpfen.

14. Automatisches Injektionsgerät (100) nach einem der Ansprüche 3 bis 13, und wobei in einem Betriebszustand nach der Injektion der Kolbenstab (122) an einer axialen Rückwärtsverschiebung relativ zu dem Gehäuseelement (102, 104) gehindert wird.

15. Automatisches Injektionsgerät (100) nach Anspruch 1, und wobei der Sicherungsring (110) an einer Verschiebung relativ zu dem Gehäuseelement (102, 104) aufgrund eines Eingriffs mit dem Nadelschutz (114) in einem Betriebszustand vor der Injektion gehindert wird und wobei die Steuerungseinheit (118) an einer Verschiebung relativ zu dem Gehäuseelement (102, 104) aufgrund eines Eingriffs mit dem Sicherungsring (110) in dem Betriebszustand vor der Injektion gehindert wird.

## Revendications

1. Dispositif d'injection automatique (100) destiné à être utilisé avec une seringue (130) comprenant au moins un piston de seringue (138) et une aiguille (136) couplée à une extrémité avant de celui-ci, comprenant :
un élément de boîtier (102, 104) agencé le long d'un axe longitudinal (107) et possédant une extrémité avant et une extrémité arrière ;
au moins un élément élastique (112) agencé de manière à être situé à l'intérieur dudit élément de boîtier (102, 104) ;
un protège-aiguille (114) pouvant être positionné de façon sélective par rapport audit élément de boîtier (102, 104) ;
et une unité de commande (118) adaptée, lorsqu'elle est actionnée, à être entraînée par ledit au moins un élément élastique (112) pour déplacer initialement ladite seringue (130) par rapport audit élément de boîtier (102, 104) d'une position de non-pénétration à une position de pénétration et ensuite déplacer ledit au moins un piston de seringue (138) dans ladite seringue (130) de manière à effectuer une administration de médicament,
et ladite unité de commande (118) étant configurée pour être actionnée lors du déplacement axial vers l'arrière dudit protège-aiguille (114) par rapport audit élément de boîtier (102, 104),
et ledit dispositif d'injection automatique comprenant également un élément de verrouillage servant à un déplacement sélectif par rapport audit élément de boîtier (102, 104) et pouvant être mis en prise de façon fonctionnelle avec ledit protège-aiguille (114),
ledit dispositif d'injection automatique étant **caractérisé en ce que**
l'élément de verrouillage est une bague de verrouillage (110), et ladite bague de verrouillage (110) est configurée pour être sollicitée en rotation autour de l'axe longitudinal (107) sous la poussée dudit au moins un élément élastique (112), mais est bloqué en rotation dans une première orientation fonctionnelle du dispositif d'injection automatique (100) par engagement avec une partie arrière du protège-aiguille (114) ; et
lors du déplacement axial vers l'arrière dudit protège-aiguille (114) par rapport audit élément de boîtier (102, 104), ladite bague de verrouillage (110) est autorisée à tourner autour dudit axe longitudinal (107) sous la poussée dudit au moins un élément élastique (112).

2. Dispositif d'injection automatique (100), selon la revendication 1, et ladite bague de verrouillage (110) étant en prise fonctionnellement de façon sélective avec ladite unité de commande (118) et ladite unité de commande (118) servant à provoquer la rotation de ladite bague de verrouillage (110) sous la poussée dudit au moins un élément élastique (112) lors du déplacement axial vers l'arrière dudit protège-aiguille (114) par rapport audit élément de boîtier (102, 104).

3. Dispositif d'injection automatique (100), selon la revendication 1 ou la revendication 2, comprenant également une tige de piston (122), servant à entraîner de façon sélective ledit au moins un piston de seringue (138) suivant un déplacement axial par rapport audit élément de boîtier (102, 104) ; ladite tige de piston (122) servant à être déplacée conjointement avec ladite unité de commande (118) depuis ledit actionnement de ladite unité de commande (118) jusqu'à ladite position de pénétration de ladite seringue (130).

4. Dispositif d'injection automatique (100), selon l'une quelconque des revendications 1 à 3, et ledit au moins un élément élastique comprenant un ressort unique (112).

5. Dispositif d'injection automatique (100) selon la revendication 3, ledit au moins un élément élastique comprenant un premier ressort et un second ressort (124).

6. Dispositif d'injection automatique (100), selon la revendication 5, et ledit second ressort (124) étant au moins partiellement disposé à l'intérieur de ladite tige de piston (122) et servant à solliciter ladite tige de piston (122) de manière à ce qu'elle soit déplacée vers l'avant le long dudit axe longitudinal (107).

7. Dispositif d'injection automatique (100), selon l'une quelconque des revendications 1 à 6, comprenant également un extracteur de protège-aiguille (150) conçu pour être fixé de manière amovible audit élément de boîtier (102, 104), servant à protéger ladite aiguille (136), et ledit protège-aiguille (114) étant empêché de se déplacer axialement vers l'arrière par rapport audit élément de boîtier (102, 104) lorsque ledit extracteur de protège-aiguille (150) est fixé audit élément de boîtier (102, 104).

8. Dispositif d'injection automatique (100), selon la revendication 7, et ledit extracteur de couvre-aiguille (150) comprenant au moins un élément de fixation (790), servant à mettre en prise un contre-élément de fixation correspondant (676) formé sur ledit protège-aiguille (114) de manière à empêcher le déplacement vers l'arrière par inadvertance dudit protège-aiguille (114) par rapport audit élément de boîtier (102, 104).

9. Dispositif d'injection automatique (100) selon l'une quelconque des revendications 1 à 8, ladite bague de verrouillage (110) étant autorisée à tourner dans un seul sens de rotation.

10. Dispositif d'injection automatique (100), selon l'une quelconque des revendications 1 à 9, et ladite bague de verrouillage (110) étant positionnée de façon sélective suivant une orientation parmi une orientation verrouillée et une orientation déverrouillée par rapport à ladite unité de commande (118) ; et ladite bague de verrouillage (110) étant positionnée suivant ladite orientation déverrouillée, ledit au moins un élément élastique étant autorisé à entraîner ladite unité de commande (118) axialement vers l'avant par rapport audit élément de boîtier (102, 104).

11. Dispositif d'injection automatique (100), selon la revendication 10, et ladite bague de verrouillage (110) comportant un élément permettant la rotation (290) et ladite unité de commande (118) comportant un élément permettant la contre-rotation (520), qui met en prise ledit élément permettant la rotation (290) lorsque ladite bague de verrouillage (110) est disposée suivant ladite orientation verrouillée.

12. Dispositif d'injection automatique (100) selon la revendication 10 ou 11, ledit protège-aiguille (114) étant empêché de se déplacer longitudinalement axialement vers l'avant par rapport au boîtier (102, 104) lorsque ladite bague de verrouillage (110) est disposée suivant ladite orientation verrouillée.

13. Dispositif d'injection automatique (100) selon l'une quelconque des revendications 1 à 12 et comprenant également un manchon de seringue (142), qui est fixé de manière fixe ou réalisé d'une seule pièce avec ledit élément de boîtier (102, 104) et comprend un élément d'amortissement (560), conçu pour amortir le choc sur la seringue (130) lors de son déplacement vers l'avant et au cours de la pénétration de ladite aiguille (136).

14. Dispositif d'injection automatique (100) selon l'une quelconque des revendications 3 à 13 et, dans un état de fonctionnement après injection, ladite tige de piston (122) étant empêchée de se déplacer axialement vers l'arrière par rapport audit élément de boîtier (102, 104).

15. Dispositif d'injection automatique (100), selon la revendication 1, et ladite bague de verrouillage (110) étant empêchée de se déplacer par rapport audit élément de boîtier (102, 104) en raison de la mise en prise avec ledit protège-aiguille (114) dans un état fonctionnel avant l'injection et ladite unité de commande (118) étant empêchée de se déplacer par rapport audit élément de boîtier (102, 104) en raison de le mise en prise avec ladite bague de verrouillage (110)dans ledit état fonctionnel avant injection.
